# EUROPEAN PATENT APPLICATION

(11) **EP 4 707 390 A1**
(43) Date of publication of application: **11.03.2026**
(21) Application number: 24797225.0
(22) Date of filing: 26.04.2024
(51) Int. Cl.: C12N 15/09, C07K 1/13, C07K 2/00, C40B 40/06, C40B 40/10

(54) **LINKER**

(30) Priority: 28.04.2023 JP 2023074709
(71) Applicant: PeptiDream Inc., Kawasaki-shi, Kanagawa 210-0821 (JP)
(72) Inventor: BASHIRUDDIN, Nasir Kato, Kawasaki-shi, Kanagawa 210-0821 (JP); OHUCHI, Masaki, Kawasaki-shi, Kanagawa 210-0821 (JP); TABUCHI, Yudai, Kawasaki-shi, Kanagawa 210-0821 (JP); NAKAJIMA, Rui, Kawasaki-shi, Kanagawa 210-0821 (JP); IJIRI, Hiroshi, Kawasaki-shi, Kanagawa 210-0821 (JP); YAMAGISHI, Kota, Kawasaki-shi, Kanagawa 210-0821 (JP); OGATA, Motoyuki, Kawasaki-shi, Kanagawa 210-0821 (JP)
(74) Representative: Mewburn Ellis LLP
(86) International application number: PCT/JP2024/016605
(87) International publication number: WO 2024/225483

(57) **Abstract**

The present invention relates to a linker and use thereof. The linker of the present invention is a linker including: an attachment part having a structure that can be attached to a desired genetic information material; and at least two or more puromycin-like substances, wherein each of the puromycin-like substances can be covalently bonded to a C-terminus of a desired peptide.

## Description

### TECHNICAL FIELD

The present invention relates to a linker including an attachment part having a structure that can be attached to a desired genetic information material and at least two or more puromycin-like substances, use of the linker, a genetic information material-linker conjugate containing the linker, a genetic information material-linker-peptide conjugate containing the linker, and others.

### BACKGROUND ART

### 1. Display method

Genotype-phenotype mapping techniques, which emerged as tools for evolutionary molecular engineering, are also called display methods, and include phage display, ribosome display, microbead droplet method, STABLE (non-covalent DNA display), mRNA display method ("In vitro virus", Nemoto N, et al., FEBS Lett. 414,405-408 (1997) (NPL 1), WO98/016636 (PTL 1); or "RNA-peptide fusions", Roberts, R. W. & Szostak, J. W., Proc. Natl. Acad. Sci. USA., 94, 12297-12302 (1997) (NPL 2), WO1998/31700 (PTL 12)), cDNA display method, photo-crosslinked cDNA display method (WO2016/159211 (PTL 6)), TRAP (transcription-translation coupled with association of puromycin linker) display method (T. Ishizuka et al., TRAP display: a high-speed selection method for the generation of functional polypeptides. Am. Chem. Soc. 2013, 135, 14, 5433-5440 (NPL 3)), cDNA TRAP display method (T. Kondo et al., cDNA TRAP display for rapid and stable in vitro selection of antibody-like proteins. Chem. Commun., 2021, 572416-572419 (NPL 4)).

In the display methods, when a functional peptide or protein molecule is selected from a library, its sequence can be easily read because the corresponding gene is conjugated to it, which is useful for selecting genetic information of a polypeptide with specific functions. The display method using a cell-free translation system (*in vitro* protein synthesis system) and reprogramming of the genetic code can be combined to synthesize a peptide (non-natural peptide) containing a non-natural amino acid residue(s).

### 2. Display method via puromycin

The above display method is a technology that unifies genotype and phenotype by conjugating mRNA as genotype and a peptide molecule as phenotype by using a cell-free translation system (*in vitro* protein synthesis system). A typical technique is a method in which a synthesized peptide molecule is conjugated to an mRNA encoding it via puromycin, an analog of the 3' end of tyrosyl-tRNA. In addition to puromycin, other substances such as puromycin derivatives have been reported to be able to conjugate a peptide to its mRNA in a similar manner (WO2011/049157 (PTL 2)).

In the display method mediated through such a puromycin-like substance, puromycin is conjugated to mRNA via an appropriate linker; when the resulting molecule is introduced into a cell-free translation system to synthesize a peptide from the mRNA, the puromycin-like substance is bonded to the C-terminus of the Elongating peptide chain as a substrate for a peptidyl transfer reaction on the ribosome; and the peptide molecule, which is the translation product, is thus conjugated to the mRNA via the puromycin-like substance. In the puromycin-like substance-mediated display method, mRNA and a puromycin-like substance are bonded covalently using RNA ligase or non-covalently by hybridization of nucleic acids. These include an mRNA display method (*in vitro* virus method), a cDNA display method, a photo-crosslinked cDNA display method (WO2016/159211 (PTL 6)), TRAP (transcription-translation coupled with association of puromycin linker) (transcription-translation coupled with association of puromycin linker) display method. The method makes it possible to conjugate each nucleic acid substance to a puromycin-like substance covalently or non-covalently.

In the above method, the puromycin-like substance acts as a substrate for a peptidyl transfer reaction on the ribosome. However, only one translation product conjugated to one nucleic acid is known for a conjugate in which a nucleic acid and its translation product is conjugated via a puromycin-like substance (e.g., WO1998/016636 (PTL 1), WO2011/049157 (PTL 2), WO2006/041194 (PTL 3), National Publication of International Patent Application No. 2011-528912 (PTL 4)). In the puromycin-like substance-mediated display method, only each monovalent translation product (peptide) conjugated to one nucleic acid has been used.

### CITATION LIST

### PATENT LITERATURE

PTL 1: WO1998/016636
PTL 2: WO20111049157
PTL 3: WO2006/041194
PTL 4: National Publication of International Patent Application No. 2011-528912
PTL 5: Japanese Patent Laid-Open No. 2008-125396
PTL 6: WO2016/159211
PTL 7: WO2012/026566
PTL 8: Japanese Patent Laid-Open No. 2008-125396
PTL 9: WO2007/066627
PTL 10: WO2019/077887
PTL 11: WO2023/234425
PTL 12: WO1998/31700

### NON PATENT LITERATURE

NPL 1: N Nemoto, et al., FEBS Lett., 1997, 414, 405-408
NPL 2: Roberts, R. W, et al., Proc. Natl. Acad. Sci. USA., 1997, 94, 12297-12302
NPL 3: T. Ishizuka et al., Am. Chem. Soc. 2013, 135, 14, 5433-5440
NPL 4: T. Kondo et al., Chem. Commun., 2021, 572416-572419
NPL 5: Liu et al., Proc. Natl. Acad. Sci. USA. 2012, 109(2), 413-418
NPL 6: Ko et al., J. Am. Chem. Soc. 2022, 144, 47, 21494-21501
NPL 7: I. P. Korndorefer and A. Skerra, Protein Sci., 2002, 11, 4, 883-893
NPL 8: K. A. McDonnell et al., J. Med. Chem., 2010, 53, 4, 1587-1596
NPL 9: M. N. Pascha et al., ACS Chem. Biol., 2022, 17, 9, 2425-2436
NPL 10: K. Muguruma et al., ACS Omega, 2019, 4, 11, 14390-14397
NPL 11: N. Terasaka et al., Nat. Chem. Biol., 2014, 10, 7, 555-557
NPL 12: H. Murakami et al., Nat. Methods, 2006, 3, 5, 357-359
NPL 13: T. Kawakami et al., Chem. Biol., 2008, 15, 1, 32-42
NPL 14: M. Saito et al., Nat. Commun., 2021, 12, 1, 2654
NPL 15: KATO T, et al. Nucleic Acids Res. 51, 8169-8180, 2023
NPL 16: Y. Goto et al., J. Am. Chem. Soc. 131, 14, 5040-5041, 2009
NPL 17: Zhenling Cui, et al., Front Bioeng Biotechnol. 8, 1031, 2020
NPL 18: Mizusawa et al., Bioorg. Med. Chem., 2009, 17, 6, 2381-2387
NPL 19: Hayashi et al., ACS Chem. Biol., 2012, 7, 3, 607-613
NPL 20: Hadidi et al., Angew Chem Int Ed Engl., 2023, 62, 23, e202216784
NPL 21: Starck et al., RNA., 2002, 8, 7, 890-903
NPL 22: Bao T Le et al., Mol Ther Nucleic Acids., 2019, 1, 14, 142-157
NPL 23: Irina Anosova et al., Nucleic Acids Res., 2016., Feb 18, 44, 3, 1007-1021
NPL 24: Michiko Kimoto et al., Front Mol Biosci, 2022, May 24, 9, 851646
NPL 25: Naho Akiyama et al., Nat Struct Mol Biol., 2024, Mar, 27
NPL 26: Valerie de Crecy-Lagard et al., Trends Microbiol., 2021, Jan, 29, 1, 41-53
NPL 27: M. H. Schreier et al., Journal of Molecular Biology, Vol. 116, No. 4, 727-753
NPL 28: H. Trachsel et al., Journal of Molecular Biology, Vol. 116, No. 4, 755-767
NPL 29: P. C. Jelenc et al., Proceedings of the Natural Academy Science of the United States of America Vol. 76, No. 7, 3174-3178

### SUMMARY OF INVENTION

### TECHNICAL PROBLEM

The purpose of the present invention is to provide, as an aspect, a linker including an attachment part having a structure that can be attached to a desired genetic information material and at least two or more puromycin-like substances, use of the linker, a genetic information material-linker conjugate containing the linker, a genetic information material-linker-peptide conjugate containing the linker, and others.

### SOLUTION TO PROBLEM

Surprisingly, the present inventors have found that by translating, on a ribosome, a target mRNA-linker conjugate, in which the target mRNA is attached to a linker containing multiple branches, each of which is coupled to a puromycin-like substance, a conjugate in which multiple translation products are conjugated to a single mRNA can be obtained; and by repeating this step, such a conjugate can be efficiently obtained, and have thus conceived the present invention. The linker of the present invention has multiple puromycin-like substances coupled. Accordingly, when an mRNA-linker conjugate of the present invention is introduced into a cell-free translation system to synthesize a peptide from the mRNA, a conjugate of the nucleic acid and the translation products can be obtained.

The present invention includes, without limitation, the following embodiments.

### [Embodiment 1]

A linker including:
an attachment part having a structure that can be attached to a desired genetic information material; and
at least two or more puromycin-like substances, wherein
each of the puromycin-like substances can be covalently bonded to a C-terminus of a desired peptide.

### [Embodiment 2]

The linker according to embodiment 1, wherein the attachment part having a structure that can be attached to a desired genetic information material includes a nucleic acid that can be attached to the desired genetic information material.

### [Embodiment 3]

The linker according to embodiment 1 or 2, for conjugating the genetic information material and a peptide encoded by the genetic information material.

### [Embodiment 4]

The linker according to any one of embodiments 1 to 3, wherein the genetic information material is a nucleic acid.

### [Embodiment 5]

The linker according to any one of embodiments 1 to 3, wherein the puromycin-like substance is puromycin.

### [Embodiment 6]

Use of a linker including:
an attachment part having a structure that can be attached to a desired genetic information material; and
at least two or more puromycin-like substances, wherein
each of the puromycin-like substances can be covalently bonded to a C-terminus of a desired peptide, wherein
the use is for conjugating the genetic information material and the peptide encoded by the genetic information material.

### [Embodiment 7]

A genetic information material-linker conjugate including:
(a) the linker according to any one of embodiments 1 to 3; and
(b) the genetic information material attached to the attachment part of the linker (a).

### [Embodiment 8]

A genetic information material-linker-peptide conjugate including:
(a) the linker according to any one of embodiments 1 to 3;
(b) the genetic information material attached to the attachment part of the linker (a); and
(c) a peptide encoded by the genetic information material and bonded to each of the at least two or more puromycin-like substances of the linker (a).

### [Embodiment 9]

A method for producing a genetic information material-linker-peptide conjugate, including:
step (1) of subjecting the genetic information material-linker conjugate according to embodiment 7 to a cell-free translation system to translate the genetic information material, wherein each of the puromycin-like substances in the linker and a translated peptide are bonded to each other to produce the genetic information material-linker-peptide conjugate.

### [Embodiment 10]

The production method according to embodiment 9, including, before step (1), step (0) of attaching the linker according to any one of embodiments 1 to 3 to the desired genetic information material to obtain the genetic information material-linker conjugate.

### [Embodiment 11]

The production method according to embodiment 9 or 10, including step (2) of repeating step (1) two or more times.

### [Embodiment 12]

A library including at least two of the genetic information material-linker-peptide conjugate according to embodiment 8.

### [Embodiment 13]

A method of screening for a peptide capable of binding to a desired target substance, including the step of bringing a library containing at least two of the genetic information material-linker-peptide conjugate according to embodiment 8 into contact with the target substance.

### [Embodiment 14]

A method of evaluating an ability of a peptide to bind to a desired target substance, including the step of bringing the genetic information material-linker-peptide conjugate according to embodiment 8 into contact with the target substance.

### [Embodiment 15]

A method for producing a genetic information material-linker-peptide conjugate, including:
step (1-i) of subjecting a genetic information material-linker conjugate, in which a linker containing at least one puromycin-like substance and a desired genetic information material are attached to each other, to a cell-free translation system to translate the genetic information material, wherein the puromycin-like substance in the linker and a translated peptide are bonded to each other to produce the genetic information material-linker-peptide conjugate; and
step (2-i) of repeating step (1-i) two or more times.

### [Embodiment 16]

A method for producing a genetic information material-linker-peptide conjugate, including:
step (1-ii) of subjecting a genetic information material-linker conjugate, in which a linker containing at least two or more puromycin-like substances and a desired genetic information material are attached to each other, to a cell-free translation system to translate the genetic information material, wherein each of the puromycin-like substances in the linker and a translated peptide are bonded to each other to produce the genetic information material-linker-peptide conjugate.

### [Embodiment 17]

A method of displaying, from a desired genetic information material, two or more peptides encoded by the genetic information material, wherein the peptides are each conjugated to the genetic information material via a functional group that can be covalently bonded to a C-terminus of each of the peptides.

### [Embodiment 18]

The method of displaying according to embodiment 17, including the step of subjecting the genetic information material-linker conjugate according to embodiment 7 to a cell-free translation system to translate the genetic information material into the peptides, wherein the puromycin-like substances and the translated peptides are bonded to each other.

### ADVANTAGEOUS EFFECTS OF INVENTION

The above-mentioned "conjugate in which multiple translation products are conjugated via puromycin-like substances to one genetic information material" can recognize a target substance through the multiple translation products if the translation products have target binding ability. This makes it possible to give the translation products a multivalent interaction (avidity effect) with the target. Thus, such conjugates of the invention may be used, for example, for a display method using a cell-free translation system (e.g., a puromycin-like substance-mediated display method) and affinity selection *(in vitro* selection method). This enables more efficient recovery of a peptide-genetic information material conjugate that binds to the target substance.

### BRIEF DESCRIPTION OF DRAWINGS

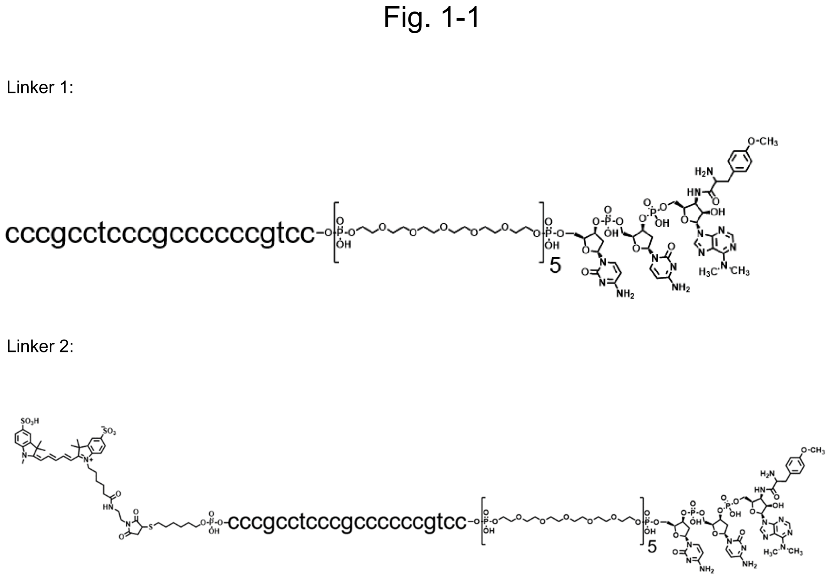

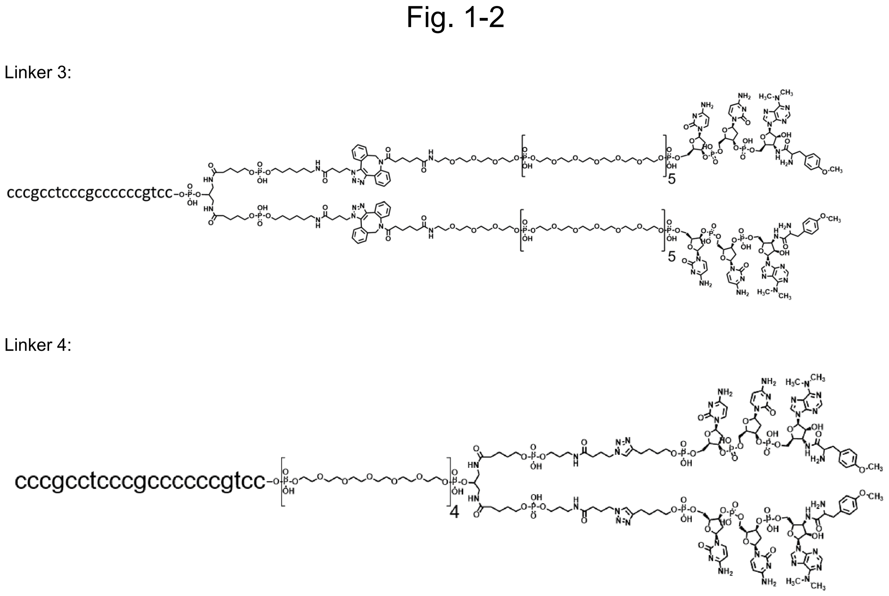

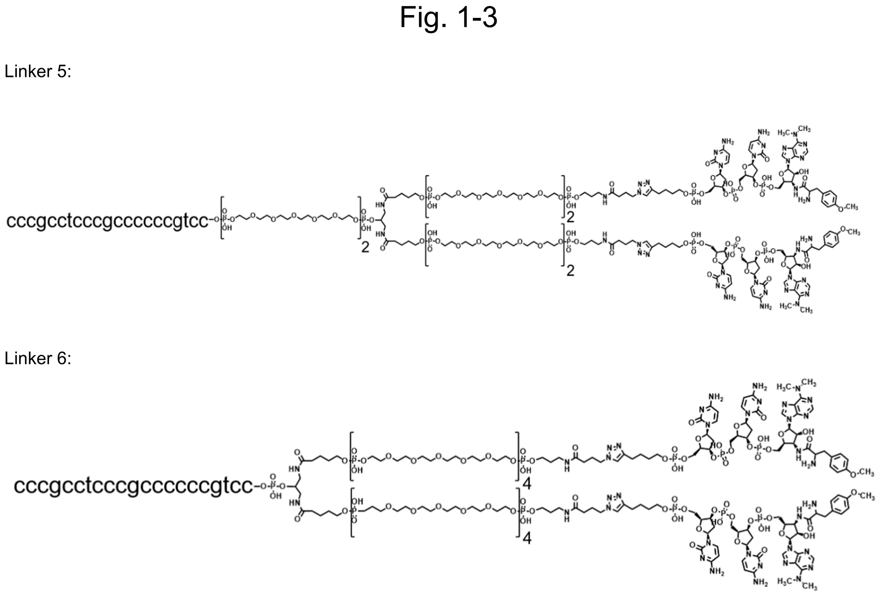

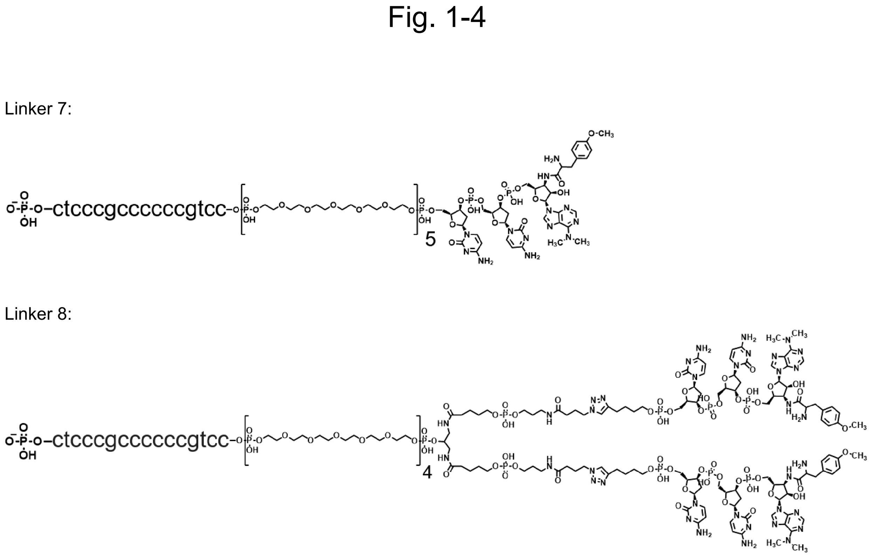

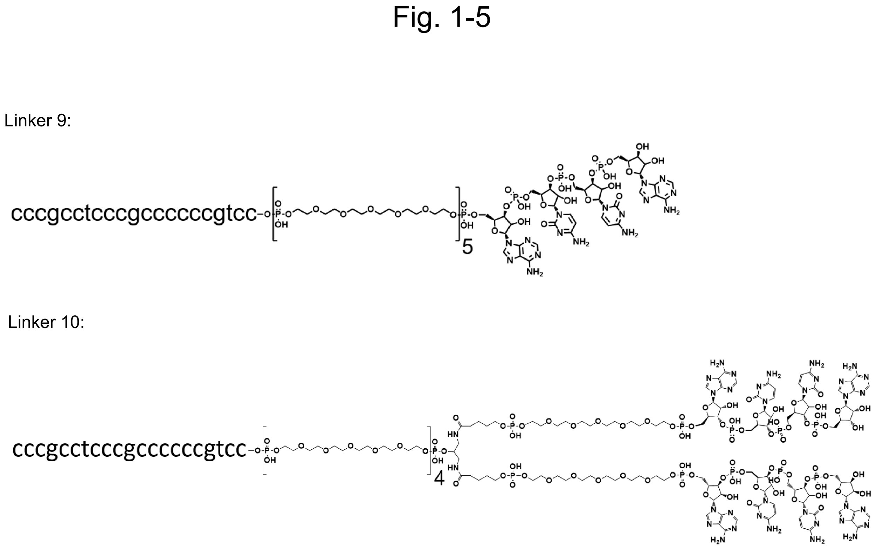

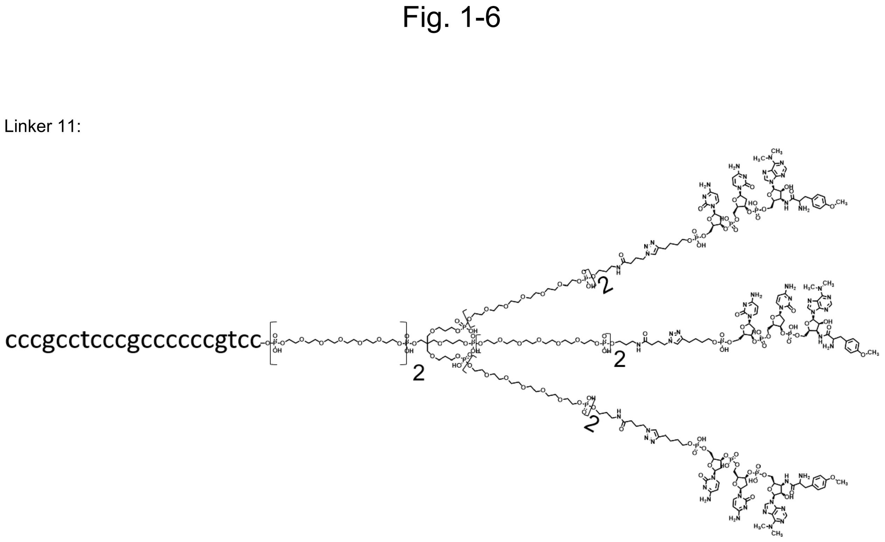

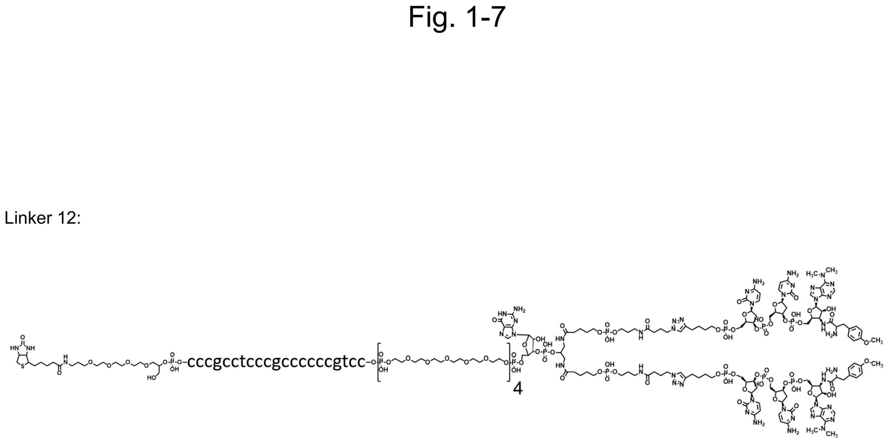

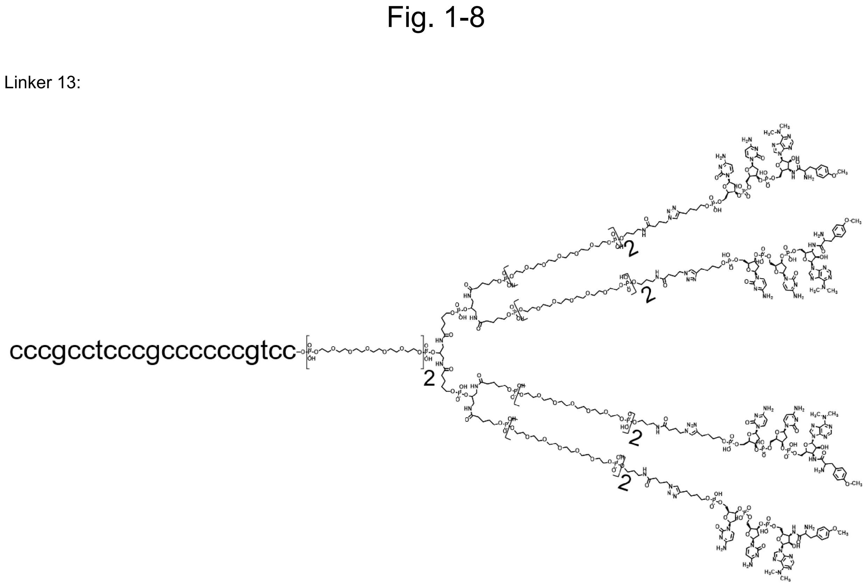

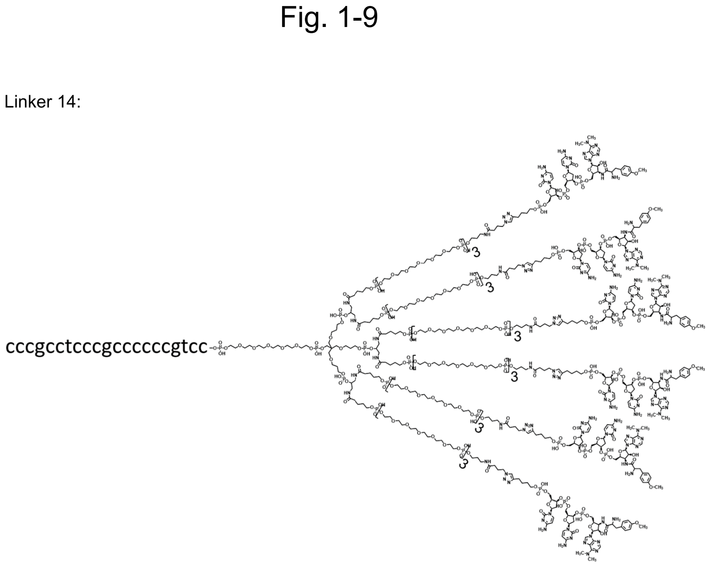

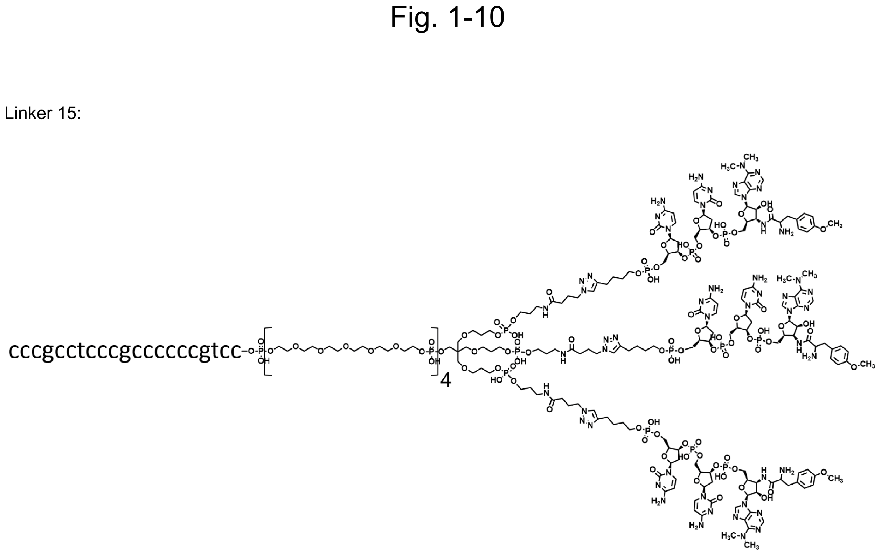

[Fig. 1-1] Fig. 1 shows the structure of each of linkers 1 to 15 used in Examples. The c, g, and t in the "cccgcctcccgccccccgtcc" (SEQ ID NO: 1) or "ctcccgccccccgtcc" (SEQ ID NO: 60) in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.
[Fig. 1-2] Fig. 1 shows the structure of each of linkers 1 to 15 used in Examples. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.
[Fig. 1-3] Fig. 1 shows the structure of each of linkers 1 to 15 used in Examples. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.
[Fig. 1-4] Fig. 1 shows the structure of each of linkers 1 to 15 used in Examples. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.
[Fig. 1-5] Fig. 1 shows the structure of each of linkers 1 to 15 used in Examples. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.
[Fig. 1-6] Fig. 1 shows the structure of each of linkers 1 to 15 used in Examples. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.
[Fig. 1-7] Fig. 1 shows the structure of each of linkers 1 to 15 used in Examples. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.
[Fig. 1-8] Fig. 1 shows the structure of each of linkers 1 to 15 used in Examples. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.
[Fig. 1-9] Fig. 1 shows the structure of each of linkers 1 to 15 used in Examples. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.
[Fig. 1-10] Fig. 1 shows the structure of each of linkers 1 to 15 used in Examples. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively.

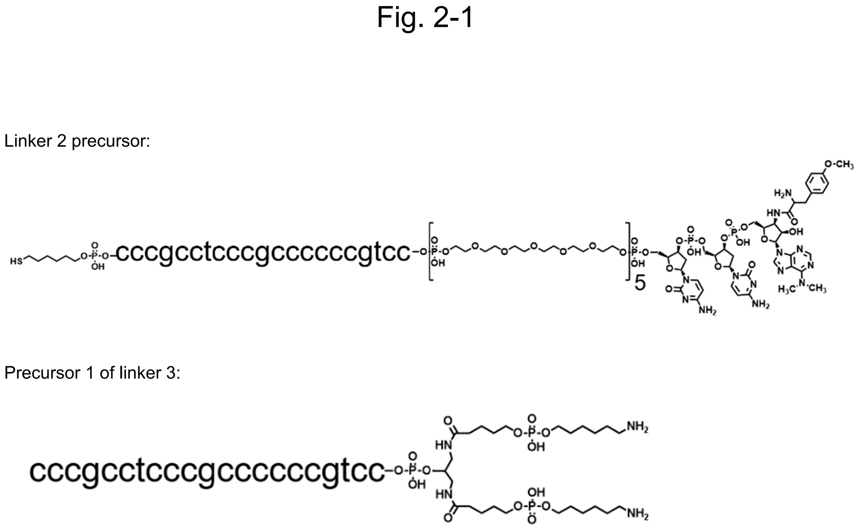

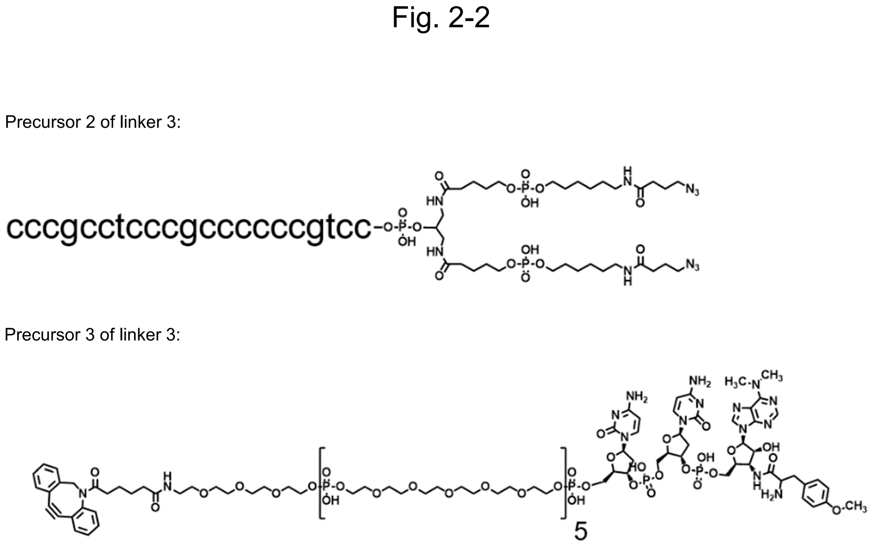

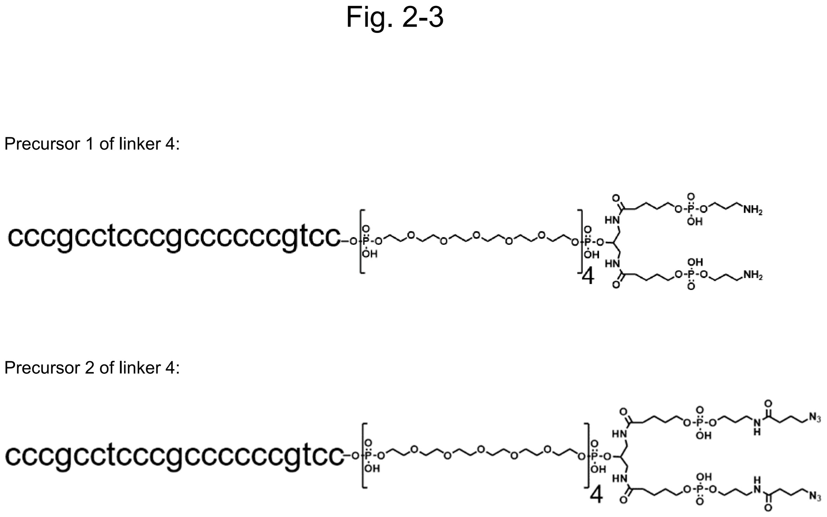

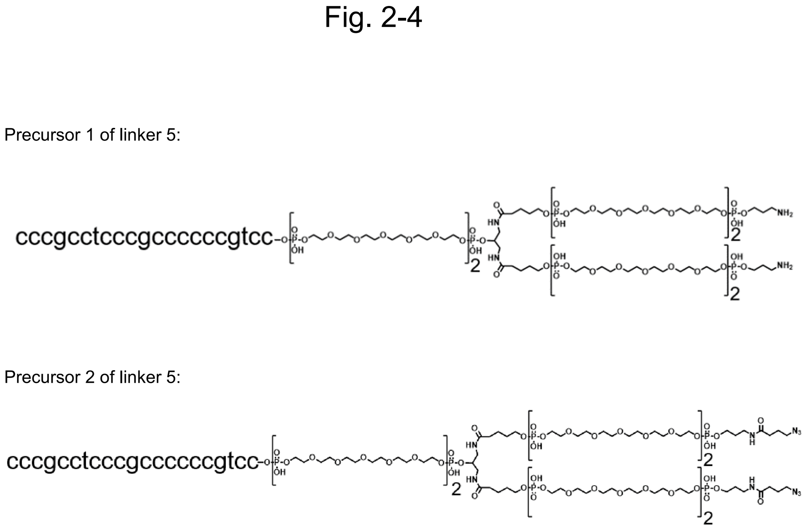

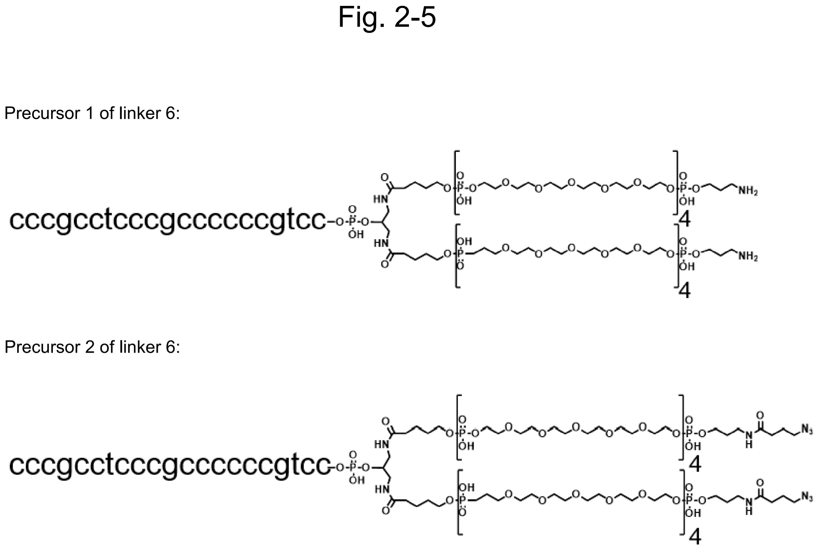

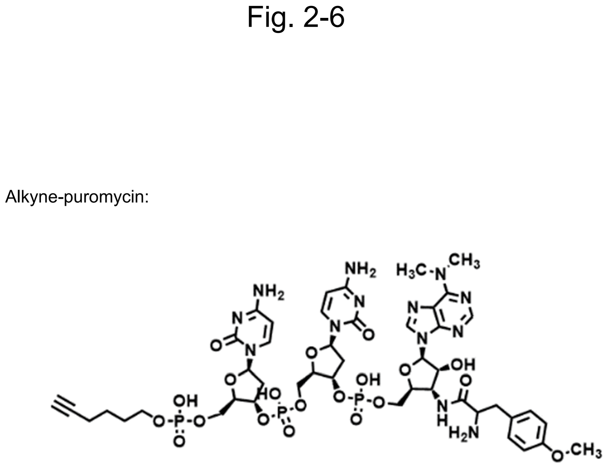

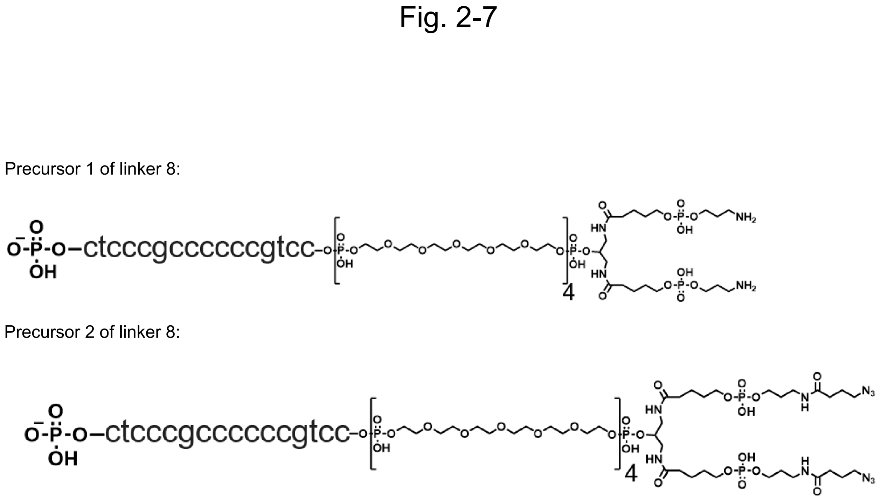

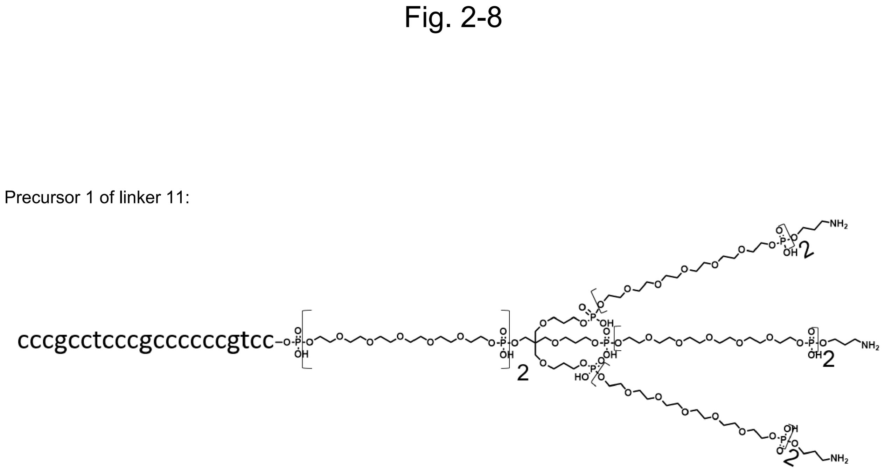

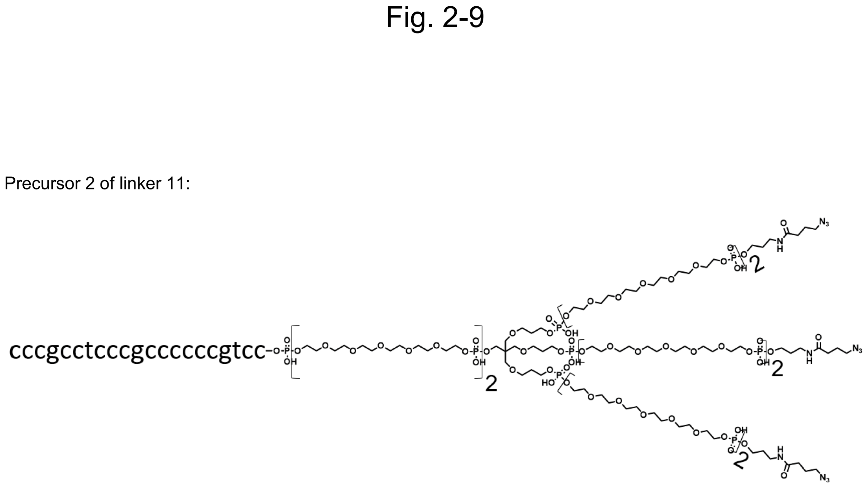

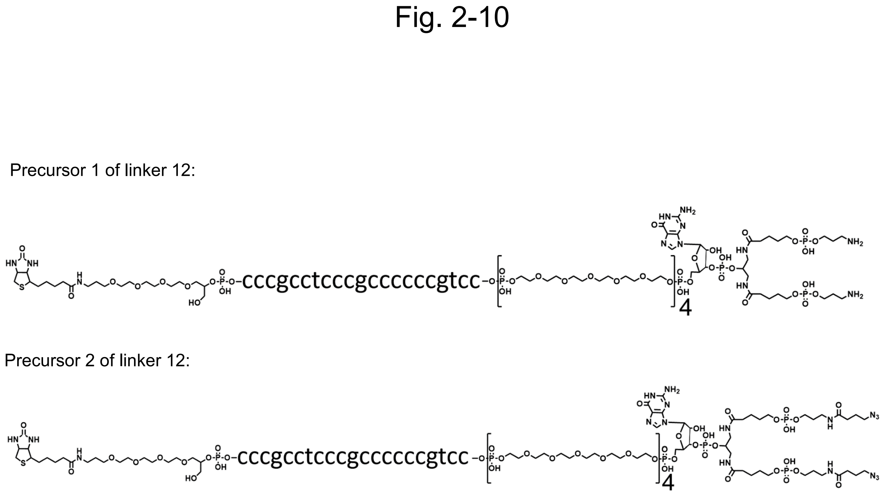

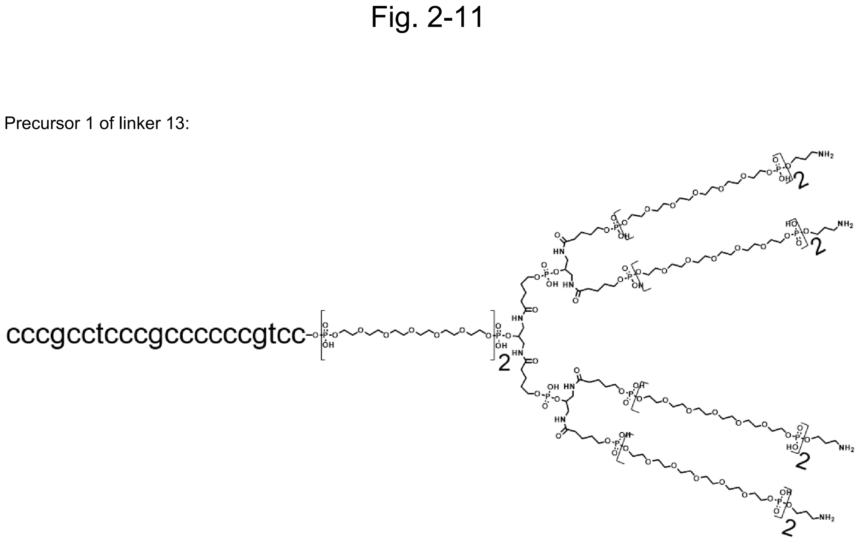

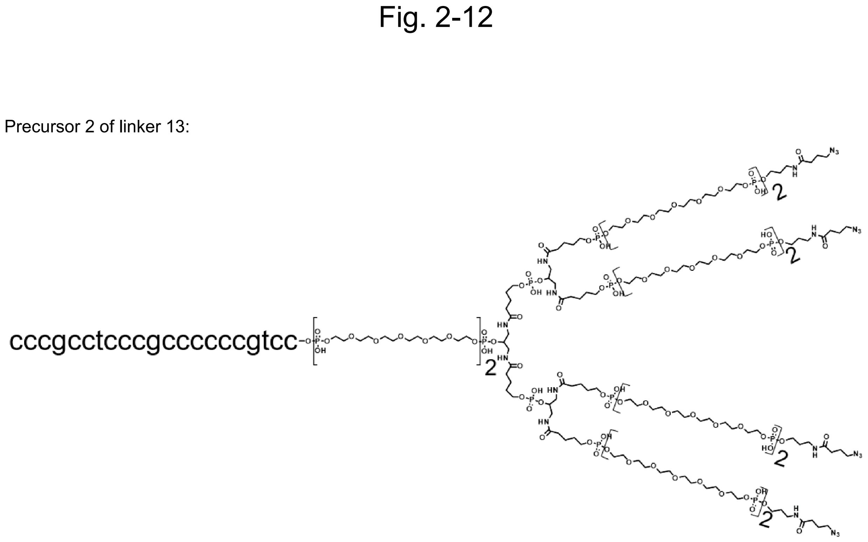

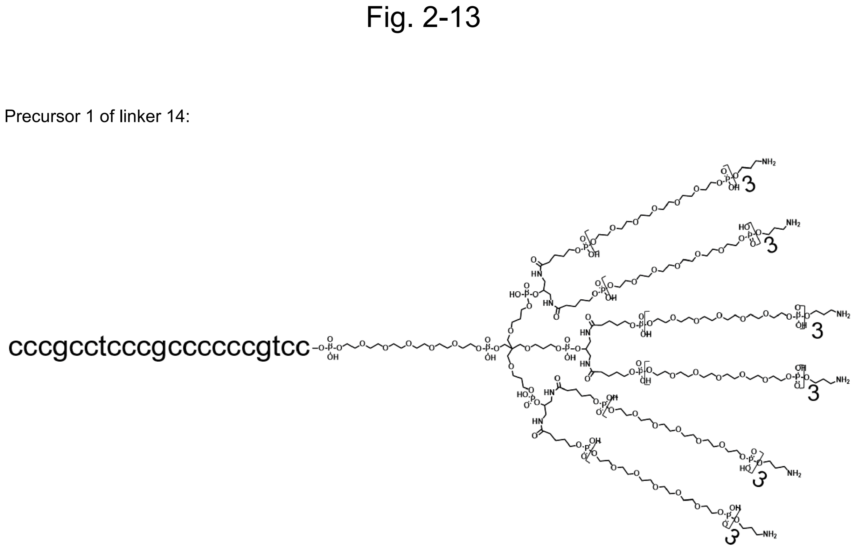

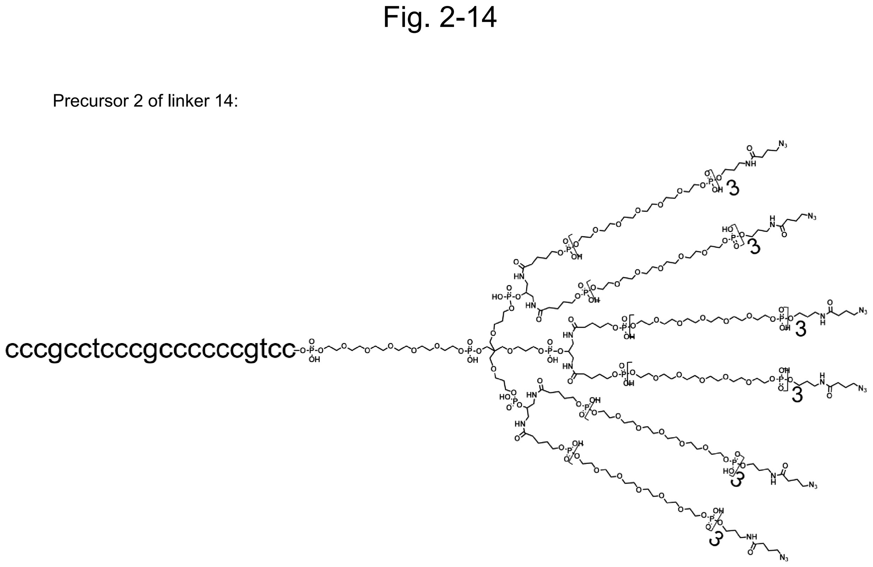

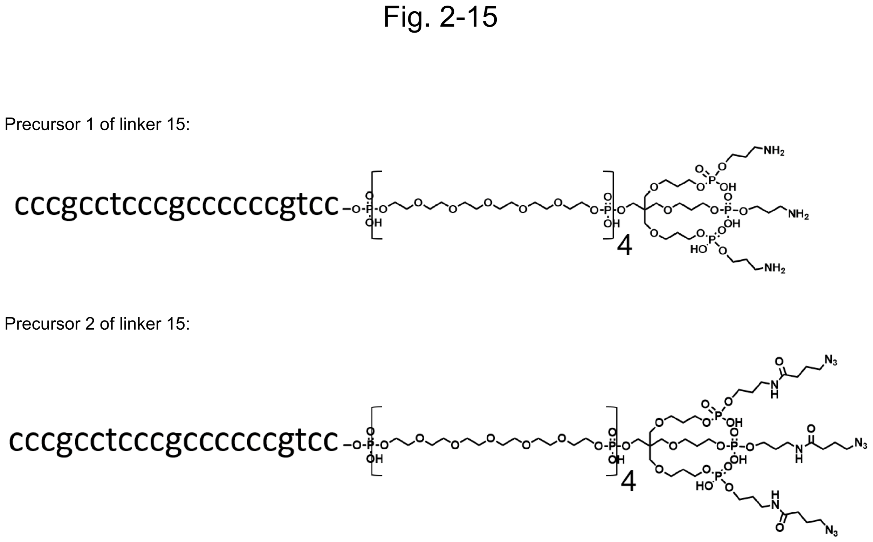

[Fig. 2-1] Fig. 2 shows the structure(s) of a precursor(s) used in the synthesis of each of the linkers 2 to 15. The c, g, and t in the "cccgcctcccgccccccgtcc" (SEQ ID NO: 1) or "ctcccgccccccgtcc" (SEQ ID NO: 60) in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-2] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-3] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-4] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-5] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-6] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-7] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-8] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-9] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-10] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-11] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-12] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-13] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-14] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.
[Fig. 2-15] Fig. 2 shows the structure(s) of a precursor(s) used in the synthesis of each of the linkers 2 to 15. The c, g, and t in the "cccgcctcccgccccccgtcc" or "ctcccgccccccgtcc" in the structure refer to nucleotides having, as a base, cytosine, guanine, and thymine, respectively, and the "cccgcctcccgccccccgtcc" or "ctcccgccccccgtcc" refers to a DNA in which these nucleotides are linked. In the DNA described above, a PEG or alkyl structure is bonded, via a phosphodiester bond, to a carbon atom at position 3' of a ribose at the 3' end. The structure in key brackets and the number in subscript indicate the repeating structure and the number of repetitions, respectively. Alkyne puromycin was used to synthesize the linkers 4 to 6, 8, and 11 to 15.

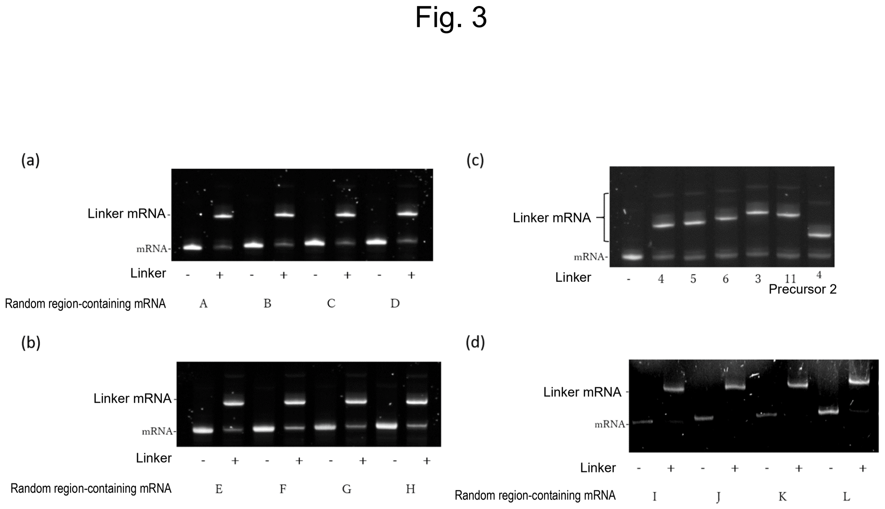

[Fig. 3] Fig. 3 shows the results of using gel electrophoresis and fluorescence imaging to evaluate the formation of an mRNA-linker conjugate. Figs. 3a and b show the results of analyzing whether a divalent linker (linker 4) can be attached to several mRNAs with the different total number of nucleotides, in which a part of the translation region is randomized to any of deoxynucleotides having, as a base, adenine, guanine, cytosine, or uracil. Fig. 3c shows the results of analyzing whether each linker can be attached to an mRNA, in which a part of the translation region is randomized with deoxynucleotides having, as a base, adenine, guanine, cytosine, or uracil, regardless of the branched chain structure or the number of puromycin molecules in the linker. Fig. 3d shows the results of analyzing whether a divalent linker (linker 8) that has a phosphate group at the 5' end and can thus covalently bond to the 3' end of mRNA by a ligation reaction can be attached to several mRNAs with the different number of nucleotides, in which a part of the translation region is randomized to any of deoxynucleotides having, as a base, adenine, guanine, cytosine, or uracil.

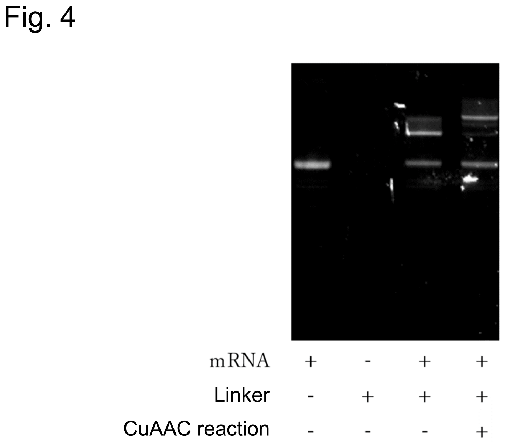

[Fig. 4] Fig. 4 shows the results of analyzing whether an mRNA-linker conjugate with two puromycin parts at the end can be produced by introducing, via the CuAAC (Azide-Alkyne-cycloaddition) reaction, alkyne puromycin into a conjugate in which an mRNA is attached to a linker (precursor 2 of linker 4) having an azido group at each end of two branched chain structures.

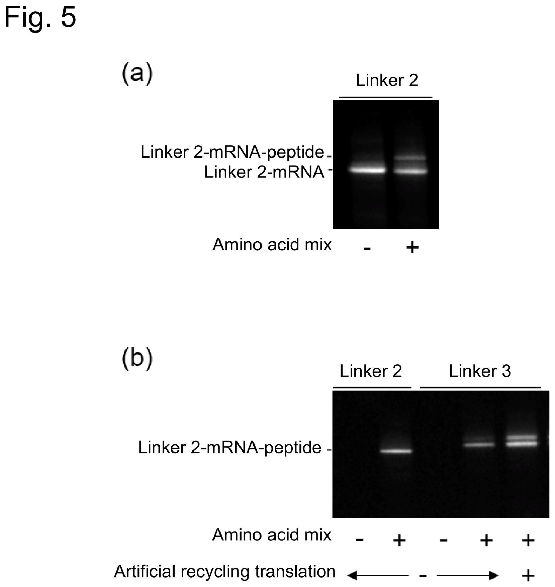

[Fig. 5] Fig. 5 shows the results of using gel electrophoresis and fluorescence imaging to evaluate a reaction of conjugating a peptide to an mRNA-linker conjugate. Figs. 5a and 5b show the images obtained when fluorescence derived from Cy5 and fluorescein, respectively, was detected. These are the results of analyzing whether artificial recycling translation using a divalent linker (linker 3) allows for efficient display of multiple peptides via puromycin molecules in the linker.

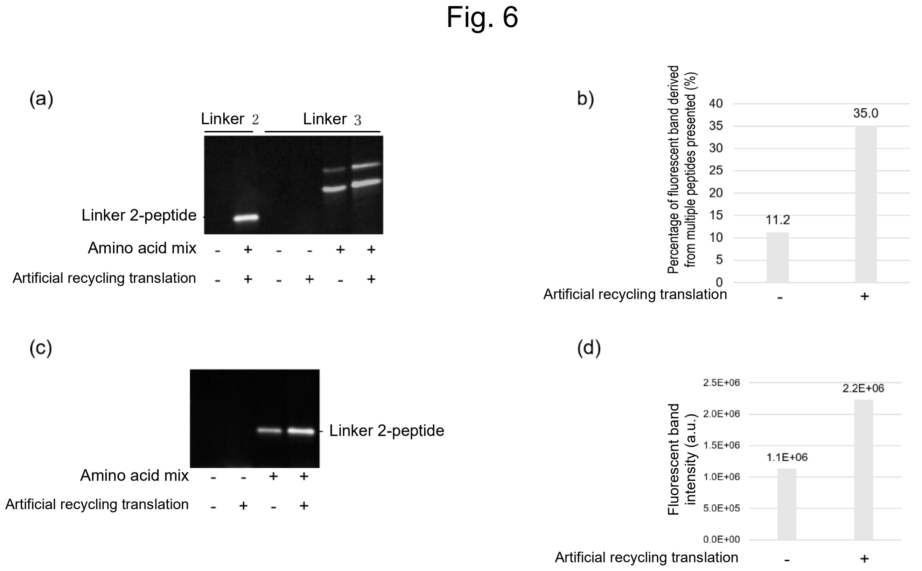

[Fig. 6] Fig. 6a shows the results of using gel electrophoresis and fluorescence imaging to analyze whether artificial recycling translation using a divalent linker (linker 3) allows for display of multiple peptides via puromycin molecules in the linker. Fig. 6b shows that artificial recycling translation increases the amount of linker having multiple peptides displayed. Figs. 6c and 6d show the results of analyzing whether artificial recycling translation increases the amount of peptide displayed by a monovalent linker (linker 2).

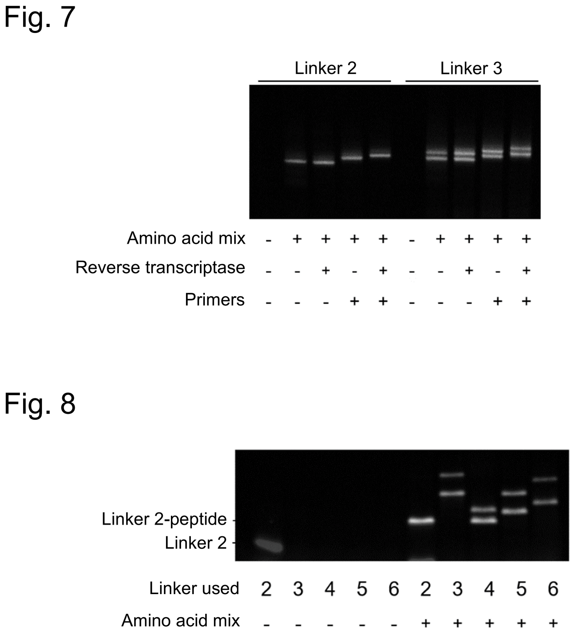

[Fig. 7] Fig. 7 shows the results of using gel electrophoresis and fluorescence imaging to analyze whether a reverse transcription reaction can be performed even for mRNA hybridized with a divalent linker (linker 3).
[Fig. 8] Fig. 8 shows the results of using gel electrophoresis and fluorescence imaging to analyze whether multiple peptides can be displayed even in divalent linkers with different linker lengths.

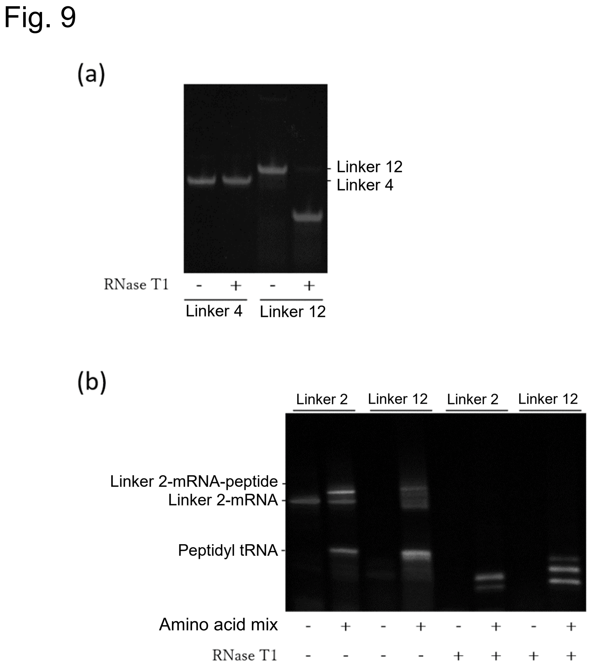

[Fig. 9] Fig. 9a shows the results of using gel electrophoresis and fluorescence imaging to analyze whether linker 12, in which the linker 4 is modified with biotin at the 5' end and a guanine-based deoxynucleotide just before the branching part in the linker, can be specifically cleaved by RNase T1. Fig. 9b shows the results of using gel electrophoresis and fluorescence imaging to evaluate a reaction of conjugating a peptide to an mRNA-linker 12 conjugate. This has demonstrated that artificial recycling translation using a divalent linker (linker 12) allows for efficient display of multiple peptides via puromycin molecules in the linker.

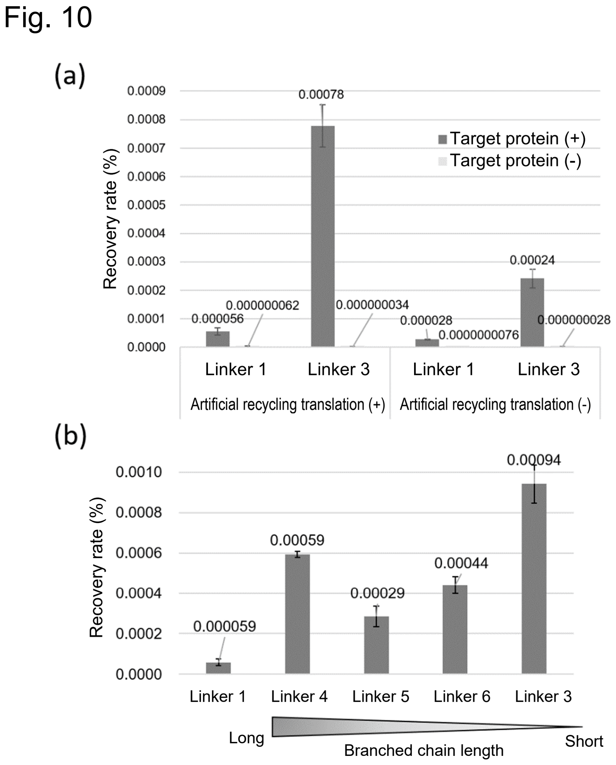

[Fig. 10] Fig. 10a shows that in the evaluation of the ability of Strep-tag II as a model peptide to bind to streptavidin as a model target protein, the use of a divalent linker (linker 3) improves the recovery rate of DNA due to binding to the target protein. It has also been demonstrated that artificial recycling translation improves the recovery rate of DNA due to binding to the target protein. The recovery rate (%) in the graphs in the figure indicates an average value, and error bars indicate standard deviations (n = 3). Fig. 10b shows that in the evaluation of the ability of Strep-tag II as a model peptide to bind to streptavidin as a model target protein, the use of a divalent linker (linkers 3 to 6) can increase the recovery rate of DNA due to binding to the target protein and the branched chain length affects the rate of increase. The recovery rate (%) in the graphs in the figure indicates an average value, and error bars indicate standard deviations (n = 3).

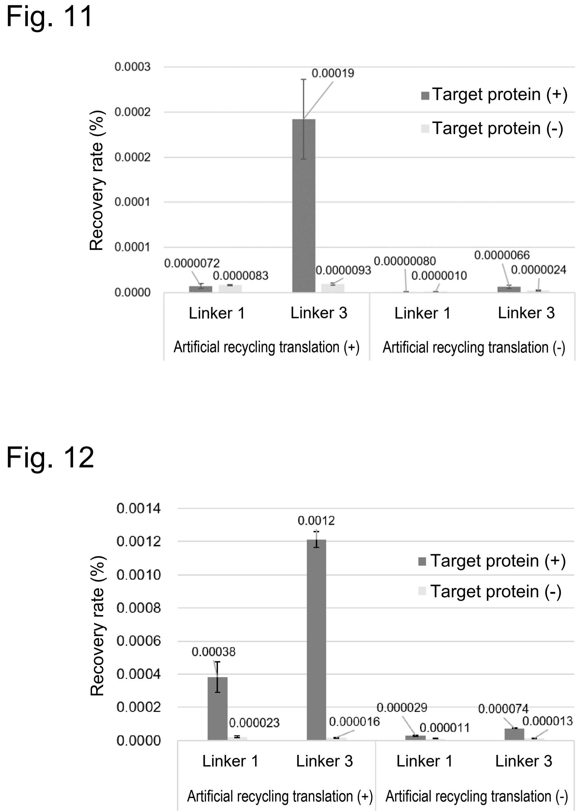

[Fig. 11] Fig. 11 shows that in the evaluation of the binding ability in the case of using a human IgG Fc protein-binding peptide, when artificial recycling translation using a divalent linker (linker 3) is performed, the recovery rate of DNA due to binding to the target protein can increase. The recovery rate (%) in the graphs in the figure indicates an average value, and error bars indicate standard deviations (n = 3).
[Fig. 12] Fig. 12 shows that in the evaluation of the recovery rate in the case of using a hemagglutinin (HA)-binding peptide, the use of a divalent linker (linker 3) improves the recovery rate of DNA due to binding to the target protein. It has also been demonstrated that artificial recycling translation improves the recovery rate of DNA due to binding to the target protein. The recovery rate (%) in the graphs in the figure indicates an average value, and error bars indicate standard deviations (n = 3).

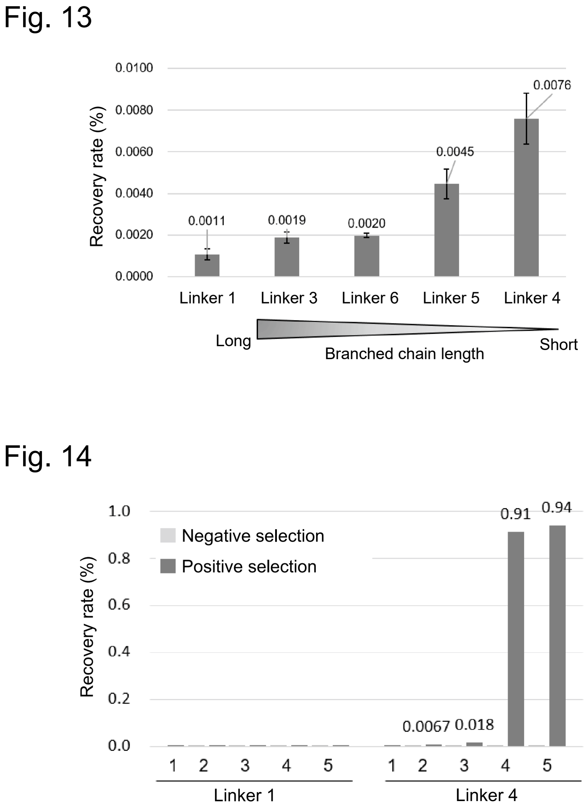

[Fig. 13] Fig. 13 shows that in the evaluation of the recovery rate in the case of using a neonatal Fc receptor (FcRn)-binding peptide, the use of a divalent linker (linkers 3 to 6) can increase the recovery rate of DNA due to binding to the target protein, and as the branched chain length becomes shorter, the rate of increase is higher. The recovery rate (%) in the graphs in the figure indicates an average value, and error bars indicate standard deviations (n = 3).
[Fig. 14] Fig. 14 shows that in the evaluation of the recovery rate of each round in the selection of Fc-binding peptides by using a random peptide library, when a divalent linker (linker 4) is used, the recovery rate of DNA due to binding to the target protein is significantly higher than the recovery rate of DNA due to nonspecific binding (obtained by negative selection) as the selection round increases.

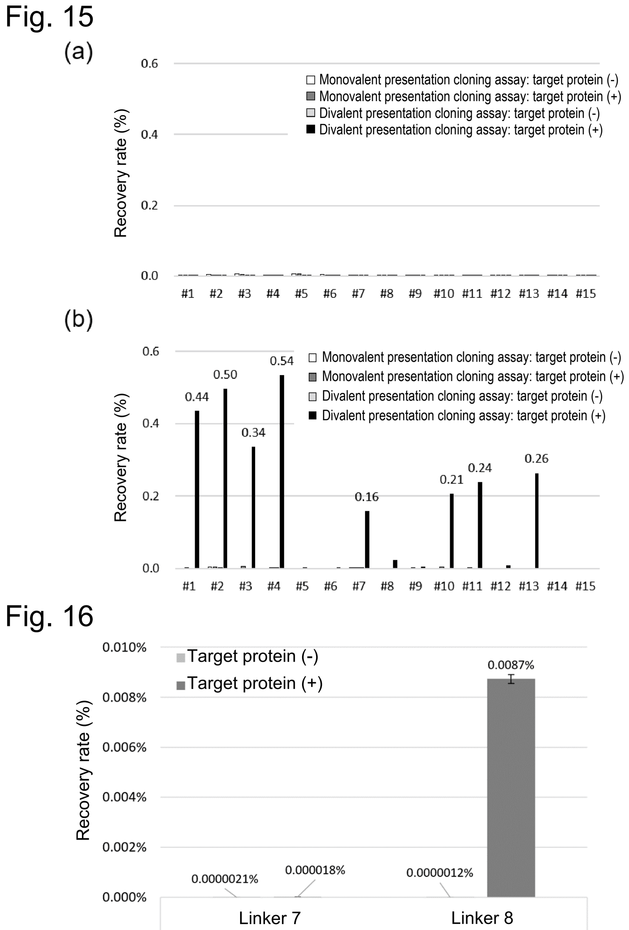

[Fig. 15] Fig. 15 shows that in the evaluation of the recovery rate in the case of using sequences obtained by gene sequence analysis during Fc-binding peptide selection, peptide sequences obtained from selections with a divalent linker (linker 4) have higher recovery rates of DNA due to binding to the target protein than recovery rates of DNA due to nonspecific binding.
[Fig. 16] Fig. 16 shows that in the evaluation of the ability of Strep-tag II as a model peptide to bind to streptavidin as a model target protein, even when a linker and an mRNA are covalently bonded, the use of a divalent linker (linker 8) improves the recovery rate of DNA due to binding to the target protein when compared to the case of using a monovalent linker (linker 7). The recovery rate (%) in the graphs in the figure indicates an average value, and error bars indicate standard deviations (n = 3).

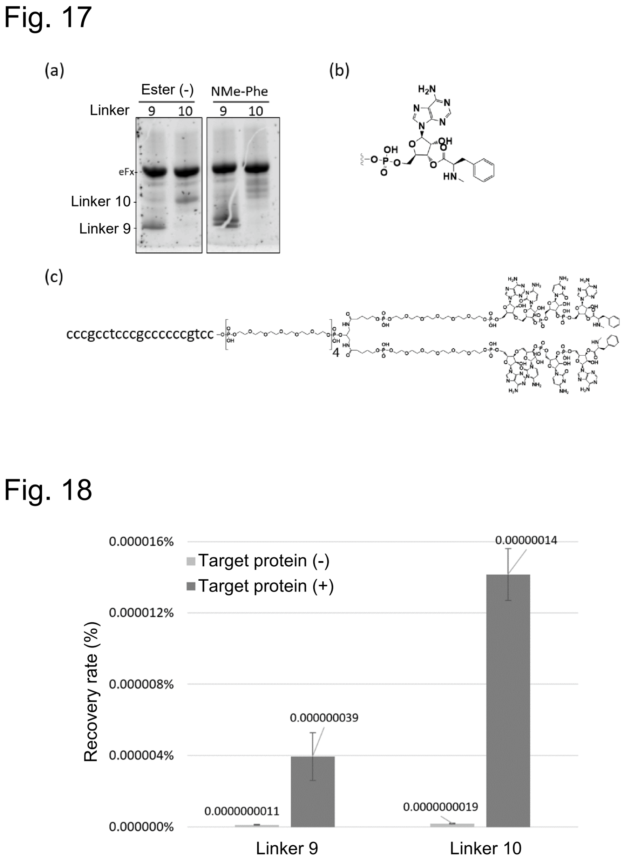

[Fig. 17] Fig. 17a shows the results of using gel electrophoresis and fluorescence imaging to analyze whether the 3' end of a monovalent linker (linker 9) or a divalent linker (linker 10) is amino acid-acylated. Fig. 17b shows the structure of a puromycin-like substance produced by making the 3' end of the linker amino-acid-acylated. Fig. 17c shows the structure of a divalent linker produced by making each 3' end of the linker amino-acid-acylated. The c, g, and t in the "cccgcctcccgccccccgtcc" in the structure refer to nucleotides having, as a base, cytosine, guanine, and thymine, respectively, and the "cccgcctcccgccccccgtcc" refers to a DNA in which these nucleotides are linked. In the DNA described above, a PEG structure is bonded, via a phosphodiester bond, to a carbon atom at position 3' of a ribose at the 3' end. The structure in key brackets and the number in subscript indicate the repeating structure and the number of repetitions, respectively.
[Fig. 18] Fig. 18 shows that in the evaluation of the ability of Strep-tag II as a model peptide to bind to streptavidin as a model target protein, even when an amino acid is covalently bonded, via an ester bond, to a ribose at the 3' end of a divalent linker (linker 10) and the characteristic as a puromycin-like substance is elicited, the use of a divalent linker (linker 10) improves the recovery rate of DNA due to binding to the target protein when compared to the case of using a monovalent linker (linker 9). The recovery rate (%) in the graphs in the figure indicates an average value, and error bars indicate standard deviations (n = 3).

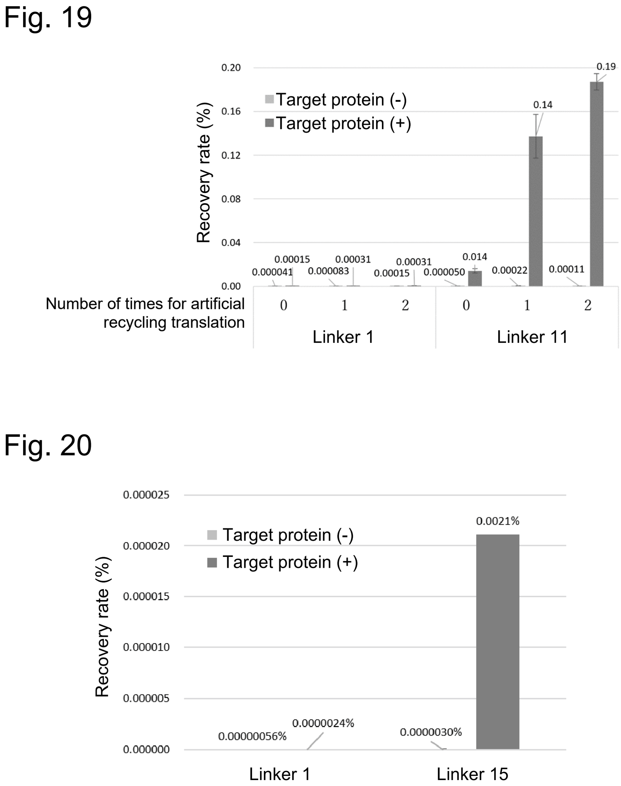

[Fig. 19] Fig. 19 shows that in the evaluation of the ability of Strep-tag II as a model peptide to bind to streptavidin as a model target protein, the use of a trivalent linker (linker 11) improves the recovery rate of DNA due to binding to the target protein when compared to the case of using a monovalent linker. It has also been demonstrated that artificial recycling translation improves, depending on the number of times for artificial recycling translation, the recovery rate of DNA due to binding to the target protein. The recovery rate (%) in the graphs in the figure indicates an average value, and error bars indicate standard deviations (n = 3).
[Fig. 20] Fig. 20 shows that in the evaluation of the ability of Strep-tag II as a model peptide to bind to streptavidin as a model target protein, the use of a trivalent linker (linker 15) improves the recovery rate of DNA due to binding to the target protein when compared to the case of using a monovalent linker. The recovery rate (%) in the graph in the figure indicates an average value (n = 2).

### DESCRIPTION OF EMBODIMENTS

The present invention includes, without limitation, the following embodiments. Unless otherwise indicated herein, technical and scientific terms used herein have the same meanings as those ordinarily understood by those skilled in the art. The substances, materials, and examples disclosed herein are merely illustrative and not intended to be limiting. Reference to "in one embodiment" herein means that it is not limited to the embodiment, i.e., it is not limited.

### 1. Linker

In one embodiment, the present invention relates to a linker. The linker of the present invention is a linker including:
an attachment part having a structure that can be attached to a desired genetic information material; and
at least two or more puromycin-like substances, wherein
each of the puromycin-like substances can be covalently bonded to a C-terminus of a desired peptide. The covalent bond is preferably an amide bond.

The "genetic information material" is a (information-containing) substance that is translated into a peptide on ribosomes.

In one embodiment, the "genetic information material" is a nucleic acid. The nucleic acid as a genetic information material may be in a natural or non-natural form. Natural nucleic acids include naturally occurring modified nucleic acids, while non-natural nucleic acids include nucleic acids, the structures of which have been modified or partially substituted with non-natural substances. The types of nucleic acids include RNA, RNA-DNA hybrids, and others. RNA may be preferably used as such nucleic acid, although the nucleic acid is not limited to RNA. The nucleotide sequence of the genetic information material may be known or unknown. The sequence may be based on a naturally occurring sequence or an artificially designed sequence. For example, the sequence may be randomly synthesized by organic synthesis, or it may encode a protein of unknown sequence by insertion of random mutations using the PCR method.

As described above, the "genetic information material" encodes a peptide, and the amino acid sequence of this peptide may be known or unknown. Also, the length of this peptide is not particularly limited. In one embodiment, the length of the peptide encoded in the "genetic information material" may consist of one or more amino acids, and preferably two or more. The upper limit is not particularly limited, and may be 1000 or less, 500 or less, 100 or less, 50 or less, 30 or less, or 20 or less.

Therefore, the length of the "genetic information material" may be 3 or more nucleotides in one embodiment and preferably 6 or more nucleotides. The upper limit is not particularly limited, and may be 3000 or less nucleotides, 1500 or less nucleotides, 300 or less nucleotides, 150 or less nucleotides, 90 or less nucleotides, or 60 or less nucleotides.

In addition, the kind of peptide encoded by the "genetic information material" is not particularly limited. The peptide may include a molecule to which tRNA can be attached and which can be condensed by a ribosome. Ribosomes are known to allow for translation using various molecules that differ in structure from common amino acids ("Translation initiation with exotic amino acids using EF-P-responsive artificial initiator tRNA", KATO T, et al., Nucleic Acids Res. 51, 8169-8180, 2023 (NPL 15) and, "Translation Initiation with Initiator tRNA Charged with Exotic Peptides", Goto Y, et al., J. Am. Chem. Soc.131, 14, 5040-5041, 2009 (NPL 16), "Cell-Free Approach for Non-canonical Amino Acids Incorporation Into Polypeptides", Zhenling Cui, et al., Front Bioeng Biotechnol. 8, 1031, 2020 (NPL 17)). Therefore, even if the structures of the molecules are different from those of amino acids, the molecules may be translated by a ribosome. This case is applicable to the present invention. In other words, in this specification, a peptide translated by a ribosome may be any molecule that can be attached to tRNA and can be translated by a ribosome.

The peptide may be a molecule containing natural amino acid residues, non-natural amino acid residues, or other structures that can be attached to tRNA and translated by a ribosome. Here, the non-natural amino acid may be any compound that can be attached to tRNA and is capable of being condensed by a ribosome. Non-limiting examples include β-amino acids, γ-amino acids, L-amino acids, D-amino acids (also called D-type amino acids), N-alkylamino acids such as N-methylamino acids and N-ethylamino acids, peptoids, α-substituted amino acids, α-α-disubstituted amino acids, cyclic α-amino acids, amino acid variants, amino acid derivatives, and other chemically modified amino acids. The peptide may include a hydroxy acid-containing molecule to which tRNA can be attached and which can be condensed by a ribosome. In addition, the peptide translated from the "genetic information material" by a ribosome is not limited to any particular form, but may take any form such as a single-chain peptide, a cyclic peptide (including a peptide that is partly cyclic), or a form with a specific secondary structure, after translation. In one embodiment, the translated peptide is cyclic.

In addition, in one embodiment, the peptide is a peptide that binds to a target substance. In one embodiment, the peptide is a fragment or the full length of a protein. In one embodiment, the peptide is an antagonist or agonist of a target substance. In one embodiment, the peptide is an antigen or an antibody against the antigen. The peptide is not limited and may be a peptide during elongation as a substrate for a peptidyl transfer reaction on a ribosome. As used herein, the term "peptide encoded by a genetic information material" includes those during elongation in translation as well as those that have been completely translated from the genetic information material.

The "structure that can be attached to a desired genetic information material" means a structure that can be attached directly or indirectly to a desired genetic information material.

The "structure that can be attached indirectly to a desired genetic information material" in one embodiment refers to a structure that can be attached via a suitable linker to a desired genetic information material. The suitable linker is a linker that can connect the desired genetic information material to the linker of the present invention. The suitable linker includes a substance that can be attached directly to the desired genetic information material and a substance that can be attached directly to the linker of the present invention. A nucleic acid that can bind directly to the desired genetic information material may be used as the substance that can bind directly to the desired genetic information material.

In addition, one embodiment of a substance that can be attached indirectly to the linker of the present invention is one of a set of functional groups that can be bonded to each other. By selecting a set of functional groups that can be bonded to each other, and by using one of the functional groups for the suitable linker described above and the other for the linker of the present invention, the suitable linker and the linker of the present invention can be bonded. In this case, the above "set of functional groups that can be bonded to each other" can be selected, if appropriate, based on the common technical knowledge of those skilled in the art. Non-limiting examples of a reaction for bonding using such a set of functional groups include: reactions through a click chemistry using, as a representative example, an azide-alkyne pair or a bioconjugation reaction; and nucleophilic substitution or nucleophilic addition reactions using a nucleophilic functional group-electrophilic functional group pair. Specific examples of these include pairs such as azide and non-cyclic strained alkyne, azide and cyclic strained alkyne, thiol and maleimide, and thiol and haloacetyl. The structure of the suitable linker is not particularly limited as long as the structure can achieve the above purpose. In view of the above purpose, an appropriate structure may be selected based on the common technical knowledge of those skilled in the art.

As described above, one embodiment of the "attachment part having a structure that can be attached indirectly to a desired genetic information material" of the linker of the present invention is an attachment part having a structure with the other functional group that can be attached to the "one of a set of functional groups that can be attached to each other" which the suitable linker has, when the desired genetic information material is attached via the suitable linker to the linker of the present invention.

Another embodiment of the "attachment part having a structure that can be attached to a desired genetic information material" is an attachment part containing a nucleic acid that can be attached to a desired genetic information material. When the "attachment part having a structure that can be attached to a desired genetic information material" contains a nucleic acid that can be attached to the desired genetic information material, the nucleic acid portion in the linker of the present invention can attach directly to the desired genetic information material.

The nucleic acid in the "nucleic acid that can be attached to a genetic information material" may be either natural or non-natural, and may be a mixture of natural and non-natural nucleic acids. DNA may be used as such nucleic acid, although the nucleic acid is not limited to DNA. In addition, as used herein, a peptide nucleic acid is included in the "non-natural nucleic acid" which can constitute the nucleic acid of the "nucleic acid that can be attached to a genetic information material". The peptide nucleic acid is a molecule having a structure similar to DNA or RNA with a peptide structure on the main chain, and is sometimes called PNA. The main chain of peptide nucleic acid has N-(2-aminoethyl)glycine units, instead of sugar (deoxyribose or ribose), amide-bonded. Then, purine and pyrimidine rings corresponding to nucleobases are attached to the main chain via methylene and carbonyl groups. Peptide nucleic acids, as well as natural-type nucleic acids, can be components of the above linker as long as they are that can be attached to the desired genetic information material.

Based on the purpose of the linker, one can select, as the nucleic acid, an appropriate structure "that can be attached to a desired genetic information material" based on common technical knowledge. Preferably, the structure can be covalently or non-covalently bonded. The nucleotide sequence of the nucleic acid is not particularly limited. The length of the nucleotide sequence of the above nucleic acid is also not particularly limited, and may be determined according to the length of the desired genetic information material, as long as the length is sufficient to allow it to attach to the desired genetic information material. In one embodiment, the length of the nucleotide sequence of the nucleic acid is a length allowing for specific hybridization with the desired genetic information material. In one embodiment, the length of the nucleotide sequence of the nucleic acid is at least 2, 3, 5, 10, 11, 12, 13, 14, 15, 16, 17, 18, 19, 20, or 22 nucleotides. From the viewpoint of maintaining the binding between the nucleic acid and the desired genetic information material, if the length of the nucleotide sequence of the nucleic acid to be hybridized is 9 nucleotides or less, it is preferable to covalently bond the nucleic acid to the desired genetic information material by the method described below, etc., in addition to hybridization. In addition, the length of the nucleotide sequence of the nucleic acid in one embodiment is less than 200 nucleotides or less, 150 nucleotides or less, 100 nucleotides or less, 80 nucleotides or less, 70 nucleotides or less, 60 nucleotides or less, 55 nucleotides or less, 50 nucleotides or less, 45 nucleotides or less, 40 nucleotides or less, 35 nucleotides or less, 30 nucleotides or less, or 25 nucleotides or less. In one embodiment, the length of the nucleotide sequence of the nucleic acid is within the range of any combination of the above-mentioned length or longer and the above-mentioned length or shorter. All or part of the nucleotide sequence of the nucleic acid is capable of attaching to the genetic information material.

The nucleic acid and the genetic information material can be attached through non-covalent and/or covalent bonds. A specific embodiment of attaching the nucleic acid to the genetic information material is not limited, but preferably it is an embodiment of non-covalent binding by single-stranded DNA capable of specifically hybridizing with a target RNA (preferably, a target mRNA). Additional embodiments include: an embodiment in which a photocrosslinkable non-natural nucleic acid-containing single-stranded DNA hybridized with a target RNA at a specific site is irradiated with UV light to photo-crosslink the target RNA and the single-stranded DNA; and an embodiment in which RNA ligase or DNA ligase is used to enzymatically covalently bond the ends of a target RNA and a single-stranded DNA.

In one embodiment, the "nucleic acid that can be attached to a genetic information material" is a single-stranded DNA capable of specifically hybridizing with a target RNA. Such DNA may contain a non-natural nucleic acid(s), without limitation.

The linker contains at least two or more puromycin-like substances. As used herein, the linker containing two puromycin-like substances is also referred to as "divalent linker" and the linker containing n puromycin-like substances as "n-valent linker".

As used herein, the "puromycin-like substance" means a substance that has a site that can be coupled to a substance constituting a linker and can be covalently bonded to the C-terminus of a peptide on a ribosome. Such a substance is not limited to puromycin and its derivatives described below, but may be, for example, the molecules described in PTL 1 in which puromycin and suppressor tRNA are attached to each other and recognized by ribosomes as a single unit. The "puromycin-like substance" is, in one embodiment, a substance that has the function of reacting with peptidyl-tRNA bound to the P-site of a ribosome to form a complex with an elongated peptide. If the substance is such a substance, the substance is not particularly limited. Here, the "peptidyl-tRNA" may be peptidyl-tRNA produced in the translation process of the peptide encoded by the genetic information material. In one embodiment, the puromycin-like substance can be covalently bonded, preferably amide-boned, to such a peptidyl-tRNA on a ribosome.

In addition, one embodiment of the "puromycin-like substance" has a structure in which a substance or a series of substances having a nucleoside or nucleic acid or a chemical structural skeleton similar thereto is chemically bonded to an amino acid or a substance with a chemical structural skeleton similar thereto, and is a substance having the function of reacting with peptidyl-tRNA bound to the P-site of a ribosome to form a complex with an elongated peptide.

In one embodiment, the puromycin-like substance is puromycin.

In addition, in one embodiment, the puromycin-like substance is a puromycin derivative. The puromycin derivative is not limited to those having the complete puromycin structure, but also include those lacking a part of the puromycin structure or having a part of the structure replaced by another structure. Non-limiting examples of the puromycin derivative include 3'-N-aminoacyl puromycin aminonucleoside (PANS-amino acid) and 3'-N aminoacyladenosine aminonucleoside (AANS-amino acid) obtained by linking via an amide bond formed by dehydration-condensation of the amino group of 3'-aminoadenosine and the carboxyl group of an amino acid. Examples of the PANS-amino acid include PANS-Gly, in which the amino acid moiety is glycine, PANS-Val for valine, PANS-Ala for alanine, or a PANS-amino acid mixture where the amino acid moiety corresponds to all the respective amino acids. In addition, examples of the AANS-amino acid include AANS-Gly, in which the amino acid moiety is glycine, AANS-Val for valine, AANS-Ala for alanine, or a AANS-amino acid mixture where the amino acid moiety corresponds to all the respective amino acids. In addition, it is also possible to use those using a nucleoside or those in which a nucleoside and an amino acid are ester-bonded (WO2011/049157 (PTL 2)). Further, non-limiting examples of the puromycin derivative include an alkyne analog of puromycin ("Imaging protein synthesis in cells and tissues with an alkyne analog of puromycin", Liu et al., Proc. Natl. Acad. Sci. USA., 2012, 109(2), 413-418 (NPL 5)), and Puroswitch, which can control translation with light ("Optical Control of Translation with a Puromycin Photoswitch", Ko et al., J. Am. Chem. Soc., 2022, 144, 47, 21494-21501 (NPL 6).

In one embodiment, the puromycin-like substance may also be a substance including puromycin or a derivative thereof and one or two deoxyribonucleotide or ribonucleotide residues. Non-limiting examples of such a substance include ribocytidyl puromycin (rCpPur), deoxydyl puromycin (dCpPur), and deoxyuridyl puromycin (dUpPur).

In addition, in one embodiment, the puromycin-like substance may be one in which the adenine-like structure attached to position 1 of the sugar backbone in puromycin is replaced by a substance having a different chemical structural backbone. Non-limiting examples include those in which the adenine-like structure attached to position 1 of the sugar backbone in puromycin is replaced by adenine, a thieno[3,4-d]pyrimidine backbone and azetidine structure, or a thieno[3,4-d]pyrimidine backbone and 3,3-difluoroazetidine structure ("Inherently Emissive Puromycin Analogues for Live Cell Labelling", Hadidi et al., Angew Chem Int Ed Engl., 2023, 62, 23, e202216784, NPL 20).

In addition, in one embodiment, the puromycin-like substance may be those in which an amino acid-like structure attached to position 3 of the sugar backbone in puromycin is replaced by a natural amino acid residue, a non-natural amino acid residue, or a hydroxy group-containing residue such as a hydroxy acid or hydroxy acid derivative, and the resulting residue and the C terminus of a peptide on a ribosome form an amide bond or an ester bond. Non-limiting examples include those in which the amino acid-like structure attached to position 3 of the sugar backbone in puromycin is replaced by β-alanine or (2r)-3-hydroxy-2-methylpropanoic acid ("Synthesis of puromycin derivatives with backbone-elongated substrates and associated translation inhibitory activities", Mizusawa et al., Bioorg. Med. Chem., 2009, 17, 6, 2381-2387, NPL 18)

In addition, in one embodiment, the puromycin-like substance may be those in which position 5 of the sugar backbone in puromycin is coupled to a variety of molecules such as one nucleic acid, a plurality of nucleic acids, a low-molecular-weight compound, or PEG. Non-limiting examples include those in which position 5 of the sugar backbone in puromycin is coupled to 1 to 30 nucleic acids, biotin, or fluorescein ("Puromycin oligonucleotides reveal steric restrictions for ribosome entry and multiple modes of translation inhibition", Starck et al., RNA, 2002, 8, 7, 890-903, NPL 21).

In addition, puromycin is an analog to the 3' end of tyrosyl-tRNA, and alternative nucleic acid structures as described below may be applicable. As a known finding, the sugar backbone or phosphate moiety of RNA or DNA can be replaced by various unnatural sugar backbones or amide-linked backbones (Bao T Le et al., Antisense Oligonucleotides Targeting Angiogenic Factors as Potential Cancer Therapeutics., Mol Ther Nucleic Acids., 2019, 1, 14, 142-157, NPL 22; and Irina Anosova et al., The structural diversity of artificial genetic polymers., Nucleic Acids Res., 2016, Feb 18, 44, 3, 1007-1021, NPL 23). Non-limiting examples of such a non-natural backbone include PNA, LNA, PS, 2'-F, 2'OMe, 2'-O-OMe, PMO, NP, UNA, 4'Thio, FANA, CeNA, HNA, tcDNA, ENA, ANA, hDNA, FRNA, GNA, TNA, XyNA, and dXyNA. One embodiment of the puromycin-like substance is a substance in which a sugar backbone portion or phosphate moiety of puromycin or puromycin analogue is replaced using a replaceable non-natural sugar backbone or phosphate backbone.

It is also known that RNA or DNA molecules with non-natural type bases have DNA or RNA hybridization function (Michiko Kimoto et al., Genetic Code Engineering by Natural and Unnatural Base Pair Systems for the Site-Specific Incorporation of Non-Standard Amino Acids Into Proteins., Front Mol Biosci, 2022, May 24, 9, 851646., NPL 24). Non-limiting examples of such a non-natural backbone include isoG, isoC, P, Z, s, y, Ds, Pa, Ds, Px, 5SICS, NaM, TPT3, NaM, CNMO, TAT1, NaM, and 5FM. One embodiment of the puromycin-like substance is a substance in which the base portion of puromycin or puromycin-like substance is replaced using a non-natural base backbone.

It is also widely known that naturally occurring modified RNA molecules retain or have some of the functions of natural RNAs (Naho Akiyama et al., Structural insights into the decoding capability of isoleucine tRNAs with lysidine and agmatidine., Nat Struct Mol Biol., 2024, Mar, 27(NPL 25); and Valerie de Crecy-Lagard., Functions of bacterial tRNA modifications: from ubiquity to diversity., Trends Microbiol., 2021, Jan, 29, 1, 41-53 (NPL 26)). One embodiment of the puromycin-like substance is a substance in which a sugar backbone, phosphate backbone, or base portion of puromycin or puromycin analogue is replaced using a naturally occurring modified RNA backbone.

The number of puromycin-like substances included in the linker is at least two or more, and the upper limit is not particularly limited. The number is not limited, and is preferably two to eight, two to seven, two to six, two to five, two to four, or two to three. In one embodiment, the number of puromycin-like substances included in the linker is two, three, four, five, or six.

The nucleic acid that can be attached to a desired genetic information material may be present either at the end or in the middle of the linker. Suppose that the above linker is interpreted as a copolymer made by linking two or more monomer compounds, the detailed structure of each monomer constituting the copolymer is omitted, and the structure of the linker is simply expressed by a line obtained by depicting the linker by connecting the linkage of each neighboring monomer with a line. As used herein, the end of a linker line represented by the above line is the end of the linker, and a part of the linker having an attachment part in the line at both ends is the middle part of the linker.

At least two or more puromycin-like substances may be present at either end or in the middle of the linker. In one embodiment, the linker is branched (branched linker), and a puromycin-like substance may be coupled to each of the branched chains. Note that as used herein, branching refers to the state in which a branched line is obtained when a linker is denoted by a line as described above. In addition, the length of each branched chain of the branched linker may be the same or different. In one embodiment, the length of each branched chain of the branched linker is the same.

The structure of the linker is not particularly limited as long as it can be bonded to the C-terminus of a peptide during elongation in a translation process. A person skilled in the art can select an appropriate structure based on the purpose of the above linker and on the basis of common technical knowledge.

The linker as a whole is, without limitation, based on a simple linear structure with moderate flexibility and few side chains, contains at least one branching part, and has a linear structure with few side chains, which structure may branch into at least three branches at the branching part (hereinafter, each chain-like structure portion joined at the branching part is referred to as a "branched chain"). Note that as used herein, three branches at the branching part means a state in which the respective ends of three chain-like structures are joined at the branching part. For example, linker 1 described in Examples has no branches, while linker 3 has one branching part and three branched chains. The branched chain may contain an additional branching part(s). In addition, without limitation, the linker may be hydrophilic as a whole. In addition, without limitation, the linker may be constructed by appropriately selecting and using an oligonucleotide such as single-stranded or double-stranded DNA or RNA, polyalkylene such as polyethylene, polyalkylene glycol such as polyethylene glycols (PEG), polystyrene, linear polysaccharides, a linear peptide, and other linear substances, or a combination thereof. When these linear substances are used in combination, they can be chemically linked with an appropriate linking group (e.g.,-NH-, -CO-, -O-, -NHCO-, - CONH-, -NHNH-, -O-PO₂H-O-, -(CH₂)ₙ- [n is, for example, 1 to 10 and preferably 1 to 3], - S-, -SO-).

In a non-limiting embodiment, the linker contains the structures (1) to (4) below.
(1) An attachment part attaching, via a phosphodiester bond, to a genetic information material and an attachment part attaching, via a phosphodiester bond, to puromycin;
(2) a branching part where three branched chains are joined;
(3) a linear part that includes PEG and is attached to the genetic information material or a linear part that includes PEG alone, alkylene alone, or a combination of PEG and alkylene;
   and
(4) a phosphodiester bond- and/or amide bond-mediated attachment part in the linear part and/or a CuAAC (AAC: Azide-Alkyne-cycloaddition) or SPAAC (strain-promoted Azide-Alkyne-cycloaddition) coupling reaction-mediated attachment part.

Alternatively, in a non-limiting embodiment, the linker contains the structures (1) to (4) below.
(1) An attachment part attaching, via a phosphodiester bond, to a genetic information material and an attachment part attaching, via a phosphodiester bond, to puromycin;
(2) a branching part where four branched chains are joined;
(3) a linear part that includes PEG and is attached to the genetic information material or a linear part that includes PEG alone, alkylene alone, or a combination of PEG and alkylene;
   and
(4) a phosphodiester bond- and/or amide bond-mediated attachment part in the linear part and/or a CuAAC (AAC: Azide-Alkyne-cycloaddition) or SPAAC (strain-promoted Azide-Alkyne-cycloaddition) coupling reaction-mediated attachment part.

The length of the linker is not particularly limited as long as the linker can be bonded to the C-terminus of a peptide during elongation in a translation process. It is known that the overall length of the linker does not significantly affect the efficiency of linker display ("cDNA TRAP display for rapid and stable in vitro selection of antibody-like proteins", T. Kondo et al., Chem. Commun., 2021, 572416-572419 (NPL 4)). A person skilled in the art can select, if appropriate, a suitable length based on the purpose of the present linker and on the basis of common technical knowledge.

The linker may be synthesized using a known procedure. For example, without limitation, a puromycin-like substance may be introduced into a linear substance, which is a branch and is attached to a nucleic acid, by using a CuAAC reaction (AAC: Azide-Alkyne-cycloaddition) or SPAAC reaction (strain-promoted Azide-Alkyne-cycloaddition).

The wording "puromycin-like substance can be covalently bonded to the C-terminus of a desired peptide" means that the puromycin-like substance is present in the linker (at the end or in the middle) in a state that can be covalently bonded to the C-terminus of a desired peptide. It is thought that the puromycin-like substance in such a state can be covalently bonded to the C-terminus of a peptide during elongation on a ribosome as a substrate for a peptidyl transfer reaction. In a preferable embodiment, the covalent bond is an amide bond. A person skilled in the art can design the linker accordingly to achieve such a state based on the conventional technology. One embodiment is in a state where a nucleoside (including a chemical structural skeleton similar to a nucleoside) included in the puromycin-like substance can be covalently bonded to an amino acid (including a substance having a chemical structure skeleton similar to an amino acid) positioned at the C-terminus of a desired peptide. In one embodiment, at least one, preferably two or more, more preferably all, of the puromycin-like substances are each coupled to the end of each branched chain included in the linker, which end is one of the ends and is not joined to the branching part. In addition, in one embodiment, the puromycin-like substance is coupled to a side chain of a branched chain in the middle of the linker.

Therefore, the type and size (length) of the peptide to which the puromycin-like substance can be bonded are not limited. One embodiment of the desired peptide is a linear peptide, and preferably a peptide whose C-terminus is linear. The peptide may contain not only a natural amino acid residue(s) but also a non-natural amino acid residue(s). Non-limiting examples of such a peptide include the fluorescent model peptide (amino acid sequence: SEQ ID NO: 19) described in the Examples, the streptavidin-binding peptide (amino acid sequence: SEQ ID NO: 21), the human neonatal Fc receptor (FcRn)-binding peptide (amino acid sequence: SEQ ID NO: 23), the hemagglutinin ( HA) binding peptide (amino acid sequence: SEQ ID NO: 25), and the human IgG Fc protein (Fc) binding peptide (amino acid sequence: SEQ ID NO: 27). The above peptide may be at least one or more peptides selected from among these.

In addition, without limitation, the peptide may be a peptide during elongation as a substrate for a peptidyl transfer reaction on a ribosome. As used herein, the term "peptide" includes those during elongation in translation as well as those that have been completely translated from a genetic information material.

In one embodiment, the linker is used to conjugate the genetic information material and a peptide encoded by the genetic information material.

The above linker may be modified, if appropriate, according to the purpose of its use. Known modification methods may be used without any particular limitation. For example, the linker may be modified with a binding substance such as biotin, a FLAG tag, a HA tag, or a His tag, or a coloring protein such as a fluorescent molecule, a fluorescent protein, a chemiluminescent protein, peroxidase, or alkaline phosphatase. By doing so, the genetic information material-linker-peptide conjugate described in the section 4 below can be used suitably for the section "8. Method of evaluating binding ability" below. In addition, the number of modifiers to the linker is not limited and may be single or multiple.

### 2. Use of linker

In one embodiment, the present invention relates to use of the above linker for conjugating a genetic information material and a peptide encoded by the genetic information material.

In one embodiment, the present invention relates to use of a linker including:
an attachment part having a structure that can be attached to a desired genetic information material; and
at least two or more puromycin-like substances, wherein
each of the puromycin-like substances can be covalently bonded to a C-terminus of a desired peptide, wherein
the use is for conjugating the genetic information material and the peptide encoded by the genetic information material.

The "linker" and its components, etc., are as described in "1. Linker". The "attachment part having a structure that can be attached to a desired genetic information material" in the linker is attached to the genetic information material. The above puromycin-like substance can be covalently bonded to the C-terminus of the desired peptide. Thus, the linker is used to conjugate the genetic information material and a peptide encoded by the genetic information material. Since at least two or more of the puromycin-like substances are present in the linker, it is possible to conjugate the two or more peptides to the genetic information material.

The present invention relates to a linker used to conjugate a genetic information material and a peptide encoded by the genetic information material.

The present invention relates to a kit or composition (e.g., a laboratory composition) containing the linker. The kit or composition is used to conjugate, for example, a genetic information material and a peptide encoded by the genetic information material. Alternatively, the above kit or composition is used in the following "7. Screening method", "8. Method of evaluating binding ability", "10. Method of displaying peptides", etc.

### 3. Genetic information material-linker conjugate

In one embodiment, the present invention relates to a genetic information material-linker conjugate. The genetic information material-linker conjugate includes:
(a) the above linker; and
(b) a genetic information material attached to an attachment part of the linker (a).

The "linker" and its components, "genetic information material", etc., are as described in "1. Linker" or "2. Use of linker".

The conjugate is an embodiment in which the genetic information material is attached to an "attachment part having a structure that can be attached to a desired genetic information material" in the linker.

As explained in "1. Linker", one embodiment of the linker is a linker including an attachment part having a structure that can indirectly attach to a desired genetic information material. In this embodiment, the conjugate may further include the above-described suitable linker, and may be a conjugate: a genetic information material-the suitable linker-the linker of the present invention. The suitable linker is a linker that can connect the desired genetic information material to the linker of the present invention. The suitable linker includes a substance that can be attached directly to the desired genetic information material and a substance that can be attached directly to the linker of the present invention.

In addition, the conjugate should include (a) and (b) above as its components. In the process of producing the conjugate, it is not necessary to use the linker mentioned above, but a "linker precursor" may be used. The "linker precursor" refers to an intermediate for making the linker, e.g., an intermediate before the linker is coupled to a puromycin-like substance. One type of such a linker precursor has two or more reactive groups for coupling a puromycin-like substance(s) and has the characteristic of becoming the linker by coupling a puromycin-like substance to each of the groups. Such a linker precursor is also an embodiment of the present invention.

For example, as described in Example 3-2, a genetic information material-linker conjugate is obtained by attaching a desired genetic information material to a linker precursor and then coupling to puromycin-like substances. This genetic information material-linker conjugate includes (a) and (b) above as its components, and is thus an embodiment of the "genetic information material-linker conjugate".

In addition, in one embodiment, the present invention encompasses a conjugate of a desired genetic information material and puromycin-like substances, including:
a desired genetic information material; and
at least two or more puromycin-like substances, wherein
the desired genetic information material and the at least two or more puromycin-like substances are conjugated to each other, and
each of the at least two or more puromycin-like substances can be covalently bonded to the C-terminus of a desired peptide.

The linker contains at least two or more puromycin-like substances, wherein each of the puromycin-like substances can be covalently bonded to the C-terminus of a desired peptide. As used herein, the "conjugate of a genetic information material and puromycin-like substances" may include the above-mentioned "genetic information material-linker conjugate" unless particularly technically inconvenient.

The genetic information material-linker conjugate can be used to conjugate the genetic information material and a peptide encoded by the genetic information material via the linker constituting the conjugate. In one embodiment, the present invention relates to use of the above conjugate for conjugating a genetic information material and a peptide encoded by the genetic information material.

The above conjugate can also be suitably used in the preparation of "6. Library", "7. Screening method", "8. Method of evaluating binding ability", "10. Method of displaying peptides", etc. The present invention, in one embodiment, relates to use of a conjugate of the linker and the genetic information material in preparation of a library, a screening method, or a method of evaluating binding ability.

In one embodiment, the present invention relates to a conjugate of the linker and the genetic information material, used for conjugating a genetic information material and a peptide encoded by the genetic information material. The present invention, in one embodiment, also relates to a conjugate of the linker and the genetic information material, used in preparation of a library, a screening method, or a method of evaluating binding ability.

The present invention relates to a kit or composition (e.g., a laboratory composition) containing a conjugate of the linker and the genetic information material. The kit or composition is used to conjugate, for example, a genetic information material and a peptide encoded by the genetic information material. Alternatively, the above kit or composition is used in the following preparation of "6. Library", "7. Screening method", "8. Method of evaluating binding ability", "10. Method of displaying peptides", etc.

### 4. Genetic information material-linker-peptide conjugate

In one embodiment, the present invention relates to a genetic information material-linker-peptide conjugate. The genetic information material-linker-peptide conjugate includes
(a) the linker;
(b) the genetic information material attached to the attachment part of the linker (a); and
(c) a peptide encoded by the genetic information material and bonded to at least one puromycin-like substance of the linker (a).

The "linker" and its components, "genetic information material", "peptide", etc., are as described in "1. Linker", "2. Use of linker", or "3. Genetic information material-linker conjugate".

The peptide (c) is bonded to at least one puromycin-like substance, preferably two or more puromycin-like substances, of the linker. Preferably the peptide (c) is bonded to all or some (two or more) of the puromycin-like substances of the linker. In one embodiment, the peptide (c) is bonded to all of the puromycin-like substances of the linker. That is, the upper limit of the number of the peptide (c) is the number of puromycin-like substances in the linker. In one embodiment, the peptide (c) is bonded to all of the puromycin-like substances of the linker. The number of peptides bonded is not limited, and is two or more and preferably two to eight, two to seven, two to six, two to five, two to four, or two to three. In one embodiment, two peptides are bonded.

The above conjugate can also be suitably used in the preparation of "6. Library", "7. Screening method", "8. Method of evaluating binding ability", "10. Method of displaying peptides", etc. The present invention, in one embodiment, relates to use of the genetic information material-linker-peptide conjugate for preparation of a library, a screening method, or a method of evaluating binding ability. The present invention, in one embodiment, relates to the genetic information material-linker-peptide conjugate used in preparation of a library, a screening method, or a method of evaluating binding ability.

In addition, in one embodiment, the present invention encompasses
a genetic information material-puromycin-like substance-peptide conjugate including:
(d) a conjugate of a desired genetic information material and puromycin-like substances;
(e) a peptide encoded by the desired genetic information material and bonded to each of the puromycin-like substances in the conjugate (d).

The "conjugate of a desired genetic information material and puromycin-like substances", the "peptide", etc., are as described in 3. Genetic information material-linker conjugate or as described above.

The present invention relates to a kit or composition (e.g., a laboratory composition) containing the genetic information material-linker-peptide conjugate. The present invention relates to a kit or composition (e.g., a laboratory composition) containing the genetic information material-puromycin-like substance-peptide conjugate. The above kit or composition is used in, for instance, the following preparation of "6. Library", "7. Screening method", "8. Method of evaluating binding ability", "10. Method of displaying peptides", etc.

### 5. Method (1) for producing genetic information material-linker-peptide conjugate

In one embodiment, the present invention relates to a method for producing a genetic information material-linker-peptide conjugate. The production method includes:
step (1) of subjecting the genetic information material-linker conjugate according to the present invention to a cell-free translation system to translate the genetic information material, wherein each of the puromycin-like substances in the linker and a translated peptide are bonded to each other to produce the genetic information material-linker-peptide conjugate.

The production method is not limited, but includes, before step (1), step (0) of attaching the linker to a desired genetic information material to obtain the genetic information material-linker conjugate.

The "linker" and its components, "genetic information material", "peptide", etc., are as described in "1. Linker", "2. Use of linker", "3. Genetic information material-linker conjugate", or "4. Genetic information material-linker-peptide conjugate".

In step (0), the technique of attaching the linker to the genetic information material is not particularly limited and can be performed by any known procedure depending on the attachment mode. The "hybridization" can be performed by subjecting the linker and genetic information material to conditions (e.g., a temperature, a salt concentration) suitable for nucleic acid hybridization. In addition, single-stranded DNA hybridized with target mRNA at specific sites is irradiated with UV light and bonded by photo-crosslinking in one embodiment; and RNA ligase or DNA ligase is used to enzymatically covalently bond the ends of a target RNA and a single-stranded DNA in one embodiment. These embodiments can also be implemented using known materials and under known conditions.

In step (1), the genetic information material is translated on a ribosome based on the genetic information material-linker conjugate obtained in step (0). Each of the puromycin-like substances in the linker and a translated peptide are bonded to each other to produce the genetic information material-linker-peptide conjugate.

The method of translating a genetic information material is not particularly limited and can be performed using known methods for translation. The peptide may be a peptide that has completed the translation process and is bonded to the puromycin-like substance described above, or it may be a peptide during elongation a ribosome as a substrate for a peptidyl transfer reaction.

The production method (especially, the translation process in (1)) above uses, without any particular limitation, a cell-free translation system. The cell-free translation system is, without any particular limitation, a combination of ribosomes extracted from cells, a group of protein factors involved in translation, tRNAs, amino acids, energy sources such as ATP, and their regeneration systems, as long as it is capable of translating mRNA into protein. The cell-free translation system can include, without any particular limitation, initiation factors, elongation factors, release factors, aminoacyl-tRNA synthetases, etc. These factors can be obtained by purification from extracts of various cells. Cells for factor purification can be, for example, prokaryotic or eukaryotic cells. Examples of the prokaryotic cells include *E. coli* cells, highly thermophilic bacteria cells, or *Bacillus subtilis* cells. Examples of known eukaryotic cells as the materials include yeast cells, wheat germs, rabbit reticulocytes, plant cells, insect cells, or animal cells. In addition to naturally occurring tRNAs and aminoacyl-tRNA synthetases (ARS), artificial tRNAs and artificial aminoacyl-tRNA synthetases that recognize nonnatural amino acids can be used. In addition, chemically synthesized tRNAs and tRNAs linked by RNA ligase can also be used.

Also, the cell-free translation system is, without any particular limitation, a reconstituted cell-free translation system. Examples of the reconstituted cell-free translation system include a system in which *E. coli* extracts as main components are subdivided and each factor is reconstituted to remove components unrelated to translation. The reconstituted cell-free translation system requires purified ribosomes, translation initiation factors, translation elongation factors, mRNA, aminoacyl-tRNAs, and substrates such as ATP and GTP (M. H. Schreier, B. Erni and T. Staehelin (1977), "Initiation of mammalian protein synthesis. I. Purification and characterization of seven initiation factors.", Journal of Molecular Biology, Vol.116, No.4, 727-753 NPL 27); H. Trachsel, B. Emi, M. H. Schreier and T. Staehelin (1977) "Initiation of mammalian protein synthesis. II. The assembly of the initiation complex with purified initiation factors.", Journal of Molecular Biology, Vol.116, No.4, 755-767 NPL28)). Of these, aminoacyl-tRNAs can be substituted by adding tRNA and aminoacyl-tRNA synthetase and its substrates in the same reaction solution. In addition, proteins and enzymes such as translation termination factors, ribosome regeneration factors, creatine kinase, myokinase, nucleotide diphosphate kinase, and pyrophosphatase, and their substrates can be added to increase the efficiency and fidelity of the translation reaction, as is performed in common cell-free translation systems (P. C. Jelenc and C. G. Kurland (1979) "Nucleoside triphosphate regeneration decreases the frequency of translation errors", Proceedings of the Natural Academy Science of the United States of America Vol.76, No.7, 3174-3178 (NPL 29)).

In the reconstituted cell-free translation system, the contamination of inhibitors such as nucleases and proteases can be more easily prevented than the conventional cell-free translation system using cell extracts.

In addition, the FIT system (WO2012/026566 (PTL 7)) can be used, without any particular limitation, for the synthesis of peptides containing special amino acids.

As shown in Fig. 5(b) of Example 5 and Figs. 6(a) and 6(b) of Example 6, in the case of a single translation reaction, the amount of translation is less than that of artificial recycling translation. However, a genetic information material-linker-peptide conjugate, in which a translated peptide is bonded to each of the puromycin-like substances in the linker, has been obtained. Although not bound by any theory, one possible reason for this is that after the translation reaction, the ribosome spontaneously dissociates from the peptide and initiates translation of the second or subsequent peptide, and the translated and synthesized peptides are each bonded to a puromycin-like substance in the linker. Alternatively, it is assumed that multiple ribosomes attach to the genetic information material to be translated to form polysomes, and the peptide translated by each ribosome is bonded to each of the puromycin-like substances in the linker.

The genetic information material-linker-peptide conjugate should be dissociated from the ribosome after it is produced, i.e., after the peptide synthesis is completed (including the case in which the peptide synthesis is in progress). The conjugate and ribosome are dissociated, i.e., the peptide is dissociated from the ribosome by known methods. In order for the ribosome to perform its function, it is necessary to maintain an appropriate three-dimensional structure, which requires a Mg ion. Therefore, the addition of a substance that can bind to a Mg ion, such as EDTA, deprives the ribosome of Mg ions and causes the ribosome to be modified and to lose its function. For example, without limitation, modification of the ribosome by adding EDTA, thus efficiently yields the conjugate in a state dissociated from the ribosome. Instead of EDTA, a substance that can modify/dissociate ribosomes may be added.

In one embodiment, the production method includes repeating step (1) two or more times (step (2)) (continuously). The above production method involving repeating step (1) two or more times is sometimes referred to herein as the "artificial recycling translation (method)". The number of times of repeating step (1) is not limited. In one embodiment, the number of repetitions is 2 to 8 times, 2 to 7 times, 2 to 6 times, 2 to 5 times, 2 to 4 times, or 2 to 3 times. In one embodiment, step (1) is repeated twice. Alternatively, in one embodiment, step (1) is repeated three times.

Note that when an n-valent linker is used, artificial recycling translation may be performed two times or more. However, from the viewpoint of efficiency of bonding the peptide to the puromycin-like substance, a large number of repetitions is preferable, and the number is more preferably n or more. In order to repeat the translation of genetic information material two times or more, it is desirable to dissociate the ribosome from the peptide once the translation is completed or in the process of translation. In other words, it is desirable to perform the ribosome dissociation process after the translation process of the genetic information material. The method of ribosome dissociation is as described above. In some cases, a ribosome spontaneously dissociates from the peptide and initiates translation of the second or subsequent peptide without artificial ribosome dissociation.

Ribosome dissociation may be performed by adding a substance capable of binding to a Mg ion as described above. In this case, it is preferable to add additional unmodified ribosomes after setting the ribosomes to non-modified conditions for the next translation process.

For example, by adding Mg(OAc)₂ (e.g., at a concentration of about 9-20 mM), Mg ions can be added and the ribosomes can be made translatable.

In addition, the genetic information material-linker-peptide conjugate may be immobilized on a solid layer such as magnetic beads, and the liquid-phase portion under the ribosome modifying conditions is removed; and the ribosome can be made translatable by adding a solution in ribosome non-modifying conditions.

In one embodiment, the genetic material-linker-peptide conjugate may be produced and then treated to improve the stability of the mRNA portion of the mRNA-linker-peptide conjugate. As the stabilizing treatment, for example, a reverse transcription reaction may be performed using the conjugate as a template to form an RNA-DNA hybrid strand. Without limitation, a reverse transcription reaction solution is added to the reaction solution containing the genetic information material-linker-peptide conjugate obtained by the translation reaction.

Without limitation, the genetic information material-linker-peptide conjugate has a higher recovery rate by recovery using a peptide-binding substance than in the case of using the linker containing only one puromycin-like substance. In one embodiment, by using the genetic information material-linker-peptide conjugate, the recovery rate by recovery using a peptide-binding substance is 1.5-fold, 2-fold, 3-fold, 5-fold, 10-fold, 20-fold, 100-fold, or 600-fold higher than that using a linker that contains only one puromycin-like substance. In this way, the peptide-binding substance (target substance) can be obtained.

A peptide-binding substance (target substance) is not particularly limited to any kind, and is any substance that can bind to the peptide. Non-limiting examples of the target substance include proteins, nucleic acids, sugar chains, low molecular weight compounds, cells, etc. In one embodiment, the target substance is a protein.

In one embodiment, the present invention relates to
a genetic information material-linker-peptide conjugate produced by a method including:
step (1) of attaching the linker to a desired genetic information material to obtain a genetic information material-linker conjugate;
step (2) of subjecting the genetic information material-linker conjugate obtained in step (1) to a cell-free translation system to translate the genetic information material, wherein each of the puromycin-like substances in the linker and a translated peptide are bonded to each other to produce the genetic information material-linker-peptide conjugate.

The above "genetic information material-linker-peptide conjugate" can also be suitably used in the preparation of "6. Library", "7. Screening method", "8. Method of evaluating binding ability", "10. Method of displaying peptides", etc.

### 6. Library

In one embodiment, the present invention relates to a library including at least two genetic information material-linker-peptide conjugates according to the present invention.

The "linker" and its components, "genetic information material", "peptide", etc., are as described in "1. Linker", "2. Use of linker", "3. Genetic information material-linker conjugate", or "4. Genetic information material-linker-peptide conjugate".

The library is characterized by containing at least two genetic information material-linker-peptide conjugates, and in other points, the library can be prepared by known methods. For example, as shown in Examples 10, 11, 12, 13, 15, and 16, the above linker can be used for known display methods and their libraries using puromycin-like substances, since it showed high recovery rates in all the different display methods.

Without limitation, the library is a library prepared by a combination of display methods in which the linker is covalently or non-covalently bonded to the genetic information material in a cell-free translation system. Also, without limitation, the library is a library for mRNA display, TRAP display, or RAPID display methods.

Also, without limitation, the library is a random peptide library. For example, by using randomized nucleic acids as the genetic information materials, it is possible to obtain a library including genetic information material-linker-peptide conjugates containing a variety of peptides encoded by the nucleic acids.

The genetic information material-linker-peptide conjugate contains at least two or more desired peptides, each of the peptides is bonded to one of the puromycin-like substances in the linker, and the two or more peptides can be present in close proximity to each other such that they can act upon each other (depending on the length of the linker, etc.). Therefore, it is possible to screen for a peptide-binding substance having a weaker ability to bind to the above peptide. Moreover, by using the library of the present invention, it is possible to highly efficiently recover a genetic information material-peptide conjugate that binds to the target substance.

### 7. Screening method

In one embodiment, the present invention relates to a method of screening for a peptide that binds to a desired target substance. The method includes the step of bringing the target substance into contact with a library containing at least two genetic information material-linker-peptide conjugates.

The "linker" and its components, "genetic information material", "peptide", "library", etc., are as described in "1. Linker", "2. Use of linker", "3. Genetic information material-linker conjugate", "4. Genetic information material-linker-peptide conjugate", or "6. Library".

Non-limiting examples of the target substance include proteins, nucleic acids, sugar chains, low molecular weight compounds, and cells. In one embodiment, the target substance is a protein.

The screening method is characterized by containing at least two genetic information material-linker-peptide conjugates, i.e., by using the library, and in other points, known screening methods can be used. In the screening method, it is possible to screen for a peptide-binding substance having a weaker ability to bind to the above peptide. In addition, the screening method of the present invention makes it possible to highly efficiently recover a genetic information material-peptide conjugate that binds to the target substance.

### 8. Method of evaluating binding ability

In one embodiment, the present invention relates to a method of evaluating an ability of a peptide to bind to a desired target substance. The method includes the step of bringing the target substance into contact with genetic information material-linker-peptide conjugates of the present invention.

The "linker" and its components, "genetic information material", "peptide", etc., are as described in "1. Linker", "2. Use of linker", "3. Genetic information material-linker conjugate", or "4. Genetic information material-linker-peptide conjugate".

The evaluation method is characterized in that the genetic information material-linker-peptide conjugate of the present invention is brought into contact with the target substance, and is not limited in other respects. In one embodiment, the evaluation method includes the steps of bringing the target substance into contact with a genetic information material-linker-peptide conjugate, and measuring an ability of the peptide to bind to the target substance in the conjugate. The method of evaluating a binding ability is not particularly limited, and any known method of evaluating a binding ability can be used. The target substance may be present in a solution, immobilized on a carrier such as beads, chips, or plates, or expressed in cells or viruses. In addition, the genetic information material-linker-peptide conjugate of the present invention may be immobilized on beads or sensor chips to evaluate the interaction with the target substance. The ability to bind to the target substance can be evaluated by known methods. Non-limiting examples of such a method of evaluating a binding ability include: detection methods based on mass differences, such as surface plasmon resonance and biolayer interferometry; and detection methods such as ELISA, qPCR quantification, flow cytometry, dye staining, chemical coloring detection, chemiluminescence detection, and fluorescence detection.

The genetic information material-linker-peptide conjugate of the present invention may be modified with a known affinity tag or a modification substance, for example, a binding substance such as a FLAG tag, a HA tag, or a His tag, or a coloring protein such as a fluorescent molecule, a fluorescent protein, a chemiluminescent protein, peroxidase, or alkaline phosphatase. It is also possible to label with biotin, as described in the Examples. The genetic information material-linker-peptide conjugate as so modified can be used, if appropriate, for immobilization, purification, and evaluation of binding ability.

In addition, the modified genetic information material-linker-peptide conjugate may also be reacted with a target substance immobilized on beads, chips, or plates, or a target substance expressed in cells or viruses, and their binding performance may be identified by using known labeling detection methods.

The ability to bind to the modified genetic material-linker-peptide conjugate can be evaluated by known methods. Non-limiting examples include: detection methods based on mass differences, such as surface plasmon resonance and biolayer interferometry; and detection methods such as ELISA, qPCR quantification, flow cytometry, dye staining, chemical coloring detection, chemiluminescence detection, and fluorescence detection. These methods may be used to evaluate the binding ability.

As described in the Examples, in the evaluation method, the genetic information material-linker-peptide conjugate of the present invention is used. This improves the ability to bind to a target substance. Thus, even when the peptide-binding substance has a weaker binding ability, the binding ability can be evaluated more precisely.

### 9. Method (2) for producing genetic information material-linker-peptide conjugate

In one embodiment, the present invention includes:
step (1-i) of subjecting a genetic information material-linker conjugate, in which a linker containing at least one puromycin-like substance and a desired genetic information material are attached to each other, to a cell-free translation system to translate the genetic information material, wherein each of the puromycin-like substances in the linker and a translated peptide are bonded to each other to produce the genetic information material-linker-peptide conjugate;
   and
step (2-i) of repeating step (1-i) two or more times.

In one embodiment, the present invention relates to a method for producing a genetic information material-linker-peptide conjugate, the method including, before step (1-i), step (0-i) of attaching a linker to a desired genetic information material, the linker including: an attachment part having a structure that can be attached to the genetic information material; and at least one puromycin-like substance, wherein the puromycin-like substance can be covalently bonded to a C-terminus of a desired peptide to obtain a genetic information material-linker conjugate.

In one embodiment, the present invention relates to
a method for producing a genetic information material-linker-peptide conjugate, including:
step (1-ii) of subjecting a genetic information material-linker conjugate, in which a linker containing at least two or more puromycin-like substances and a desired genetic information material are attached to each other, to a cell-free translation system to translate the genetic information material, wherein each of the puromycin-like substances in the linker and a translated peptide are bonded to each other to produce the genetic information material-linker-peptide conjugate.

In one embodiment, the present invention includes, before step (1-ii), step (0-ii) of attaching a linker to a desired genetic information material, the linker including: an attachment part having a structure that can be attached to the genetic information material; and at least two or more puromycin-like substances, wherein each of the puromycin-like substances can be covalently bonded to a C-terminus of a desired peptide to obtain a genetic information material-linker conjugate. Here, the linker components, "genetic information material", "peptide", etc., are as described in "1. Linker", "2. Use of linker", "3. Genetic information material-linker conjugate", or "4. Genetic information material-linker-peptide conjugate". In addition, steps (1) and (2) are as described above.

The production method described in "5. Method (1) for producing genetic information material-linker-peptide conjugate" includes a step of artificial recycling translation. The present inventors have found that the method including a step of artificial recycling translation is not limited to the linker "containing two or more puromycin-like substances" but is also effective when a linker "containing one puromycin-like substance" (also herein referred to as "monovalent linker") is used. This is shown, for example, in Fig. 10 of Example 10, Fig. 11 of Example 11, and Fig. 12 of Example 12. In all of these Examples, in the case of using the monovalent linker, when the case of single translation step is compared with the case of translation twice, the recovery rate of the latter has been significantly increased. Although not bound by any theory, this may be because when a linker with a single puromycin-like substance is subjected to the translation process, a large number of linkers to which the peptide is not bonded remain in the reaction solution after one translation step, but by repeating the translation step two or more times, the number of peptide-bonded linkers in the reaction solution increases.

The genetic information material-linker-peptide conjugate obtained by the above production method can be suitably used in the method of screening for a peptide that binds to a desired target substance and in the method of evaluating an ability of a peptide to bind to a desired target substance. There are no particular limitations on the screening method or the method of evaluating a binding ability as described above, and each of the known methods can be used.

The matters described in "5. Method (1) for producing a genetic information material-linker-peptide conjugate" are also applicable to "9. Method (2) for producing a genetic information material-linker-peptide conjugate" as long as there is no technical inconsistency.

### 10. Method of displaying peptides

In one embodiment, the present invention relates to
a method of displaying, from a desired genetic information material, two or more peptides encoded by the genetic material, wherein
each peptide is conjugated to the genetic information material via a functional group that can be covalently bonded to the C-terminus of the peptide.

The "genetic information material", "peptide", etc., are as described in "1. Linker", "2. Use of linker", "3. Genetic information material-linker conjugate", or "4. Genetic information material-linker-peptide conjugate".

In one embodiment, the display method includes
the step of subjecting the genetic information material-linker conjugate to a cell-free translation system to translate the genetic information material into the peptides, wherein each of the puromycin-like substances and a translated peptide are bonded to each other.

The "step of attaching the linker to the genetic information material," "cell-free translation system," "step of translating into the peptides," etc., are as described in "5. Method (1) for producing genetic information material-linker-peptide conjugate".

The display method of the present invention can be used to present multiple peptides encoded by a desired genetic information material. This improves avidity and enables more efficient recovery of a genetic information material-peptide conjugate that binds to a target substance.

### EXAMPLES

Hereinafter, the present invention will be described in detail based on Examples. The present invention, however, is not limited to these Examples. A person skilled in the art may easily make modifications and changes to the present invention based on the description herein, and they are included in the technical scope of the present invention.

### [Example 1: Synthesis of various linkers]

Each linker was designed to contain "cccgcctcccgccccccgtcc" (SEQ ID NO: 1) or "ctcccgccccccgtcc" (SEQ ID NO: 60) as an mRNA hybridization region. In addition, linkers 1, 2, 7, and 9 were designed to contain one puromycin that can form an amide bond with the C-terminus of a desired peptide, linkers 3 to 6, 8, 10, and 12 to contain two, linkers 11 and 15 to contain three, linker 13 to contain four, and linker 14 to contain six (Figs. 1-1, 1-2, 1-3, 1-4, 1-5, 1-6, 1-7, 1-8, 1-9, and 1-10). The synthesis scheme of each linker was designed as follows.

Linkers 1, 7, 9, 10: see the corresponding NPLs below; linker 1 (NPL 3); linker 7 ("In Vitro Selection of Anti-Akt2 Thioether-Macrocyclic Peptides Leading to Isoform-Selective Inhibitors" Hayashi et al., ACS Chem. Biol., 2012, 7, 3, 607-613, NPL 19); linkers 9 and 10 (PTL 2), synthesized by a common phosphoramidite method and purified by high-performance liquid chromatography (HPLC).

Linker 2: linker 2 precursor with a thiol at the 3' end (Fig. 2-1) was synthesized by a common phosphoramidite method, followed by Cy5 modification by Michael addition of the above thiol to maleimide and purification by HPLC.

Linker 3: linker 3 precursor 1 (Fig. 2-1) with two primary amines at the 5' ends was synthesized by a common phosphoramidite method; precursor 2 of linker 3 (Fig. 2-2) was synthesized by a reaction with 4-azido-butane-1-oic acid NHS ester having amine-reactive N-hydroxysuccinimide (NHS) ester at one end, followed by HPLC purification. Precursor 3 of linker 3 (Fig. 2-2) was then synthesized by a common phosphoramidite method, and linker 3 was synthesized by coupling to precursor 2 of linker 3 via the SPAAC reaction, followed by purification by HPLC.

Linkers 4 to 6, 8, 11 to 15: precursors 1 (Figs. 2-3, 2-8, 2-10, 2-11, 2-13, and 2-15) of linkers 4 to 6, 8, 11 to 15 with multiple primary amines at the 5' end (Figs. 2-3, 2-4, 2-8, 2-10, 2-11, 2-13, and 2-15) were synthesized by a common phosphoramidite method, and reacted with 4-azido-butane-1-oic acid NHS ester having an amine-reactive NHS ester at one end to synthesize precursors 2 (Figs. 2-3, 2-4, 2-5, 2-7, 2-9, 2-10, 2-12, 2-14, and 2-15) of linkers 4 to 6, 8, and 11 to 15, followed by HPLC purification. Alkyne puromycin (Fig. 2-6) was then synthesized by a common phosphoramidite method, and was coupled, via CuAAC reaction, to precursors 2 of linkers 4 to 6, 8, and 11 to 15 to synthesize linkers 4 to 6, 8, and 11 to 15, followed by purification by HPLC.

Based on the synthetic schemes designed above, the synthesis of linkers 1 and 3 was entrusted to BEX (Japan), and that of linkers 2, and 4 to 15 to Gene Design (Japan). Linker 12 was synthesized using precursor 2 of linker 12, which was synthesized by Gene Design, Inc. (Japan), and alkyne puromycin. Linkers 4 to 12, 15, and precursor 2 of linker 12 were analyzed by Gene Design using HPLC (BioAccord^{™} SYSTEM, Waters) (column used: XBridge C18 Column 130 Å 2.5 µm 4.6 mm × 75 mm; column temperature: 60°C; solvent A: 100 mM hexafluoroisopropanol (HFIP) and 8 mM triethylamine; solvent B: methanol; gradient of solvent B: 5-40% (5-30% for linkers 4, 9, 10, and precursor 2 of linker 12; 5-50% for linker 8; 5-60% for linkers 11 and 13 to 15), 20 min, flow rate: 1 mL/min). The purity of each linker as determined from the above analysis was as follows.

Linker 4: 96.3%
Linker 5: 95.6%
Linker 6: 91.6%
Linker 7: 97.6%
Linker 8: 95.54%
Linker 9: 98.75%
Linker 10: 98.18%
Linker 11: 90.3%
Precursor 2 of linker 12: 97.68%
Linker 13: 92.65%.
Linker 14: 89.38%
Linker 15: 96.17%

Meanwhile, the following is the results of mass spectrometry using an electrospray ionization time-of-flight mass spectrometer (ESI-TOF-MS, BioAccord^{™} SYSTEM, Waters) conducted by Gene Design, Inc.

Linker 4: ESI-TOF-MS, [M-H]⁻ calculated 10808.12; measured 10807.00 (after deconvolution).

Linker 5: ESI-TOF-MS, [M-H]⁻ calculated 11496.72; measured 11496.00 (after deconvolution).

Linker 6: ESI-TOF-MS, [M-H]⁻ calculated 12185.32; measured 12184.00 (after deconvolution).

Linker 7: ESI-TOF-MS, [M-H]⁻ calculated 7587.33; measured 7588.0 (after deconvolution).

Linker 8: ESI-TOF-MS, [M-H]⁻ calculated 9398.84; measured 9400.30 (after deconvolution).

Linker 9: ESI-TOF-MS, [M-H]⁻ calculated 9150.22; measured 9150.90 (after deconvolution).

Linker 10: ESI-TOF-MS, [M-H]⁻ calculated 1115.62; measured 11115.00 (after deconvolution).

Linker 11: ESI-TOF-MS, [M-H]⁻ calculated 13659.18; measured 13662.00 (after deconvolution).

Linker 12 precursor: ESI-TOF-MS, [M-H]⁻ calculated 9303.66; measured 9302.00 (after deconvolution).

Linker 13: ESI-TOF-MS, [M-H]⁻ calculated 16493.59; measured 16493.00 (after deconvolution).

Linker 14: ESI-TOF-MS, [M-H]⁻ calculated 22880.98; measured 22880.60 (after deconvolution).

Linker 15: ESI-TOF-MS, [M-H]⁻ calculated 12281.98; measured 12284.80 (after deconvolution).

First, 3-hydroxypicolinic acid (final concentration: 50% (v/v), saturated solution, 50% acetonitrile, 0.1% trifluoroacetic acid, 10 mg/mL diammonium hydrogen citrate) was added to each of synthesized linkers 1 to 12 (final concentration: 20 µM), and the mixture was then crystallized (at RT) on MTP 384 TARGET PLATE POLISHED STEEL BC (Bruker). The crystallized samples were analyzed by mass spectrometry (linear mode, positive ion mode) using a matrix-assisted laser desorption/ionization time-of-flight mass spectrometer (MALDI-TOF-MS, autoflex (registered trademark) maX, Bruker). The results of mass spectrometry were as follows.

Linker 1: MALDI-TOF-MS (m/z), [M+H]⁺ calculated 8995.34; measured 8994.27.

Linker 2: MALDI-TOF-MS (m/z), [M+H]⁺ calculated 9956.42; measured 9956.17.

Linker 3: MALDI-TOF-MS (m/z), [M+H]⁺ calculated 13782.77; measured 13782.73.

Linker 4: MALDI-TOF-MS (m/z), [M+H]⁺ calculated 10810.13; measured 10808.55.

Linker 5: MALDI-TOF-MS (m/z), [M+H]⁺ calculated 11498.73; measured 11496.18.

Linker 6: MALDI-TOF-MS (m/z), [M+H]⁺ calculated 12187.33; measured 12185.45.

Linker 7: MALDI-TOF-MS (m/z), [M+H]⁺ calculated 7589.35; measured 7589.90.

Linker 8: MALDI-TOF-MS (m/z), [M+H]⁺ calculated 9400.86; measured 9400.68.

Linker 9: MALDI-TOF-MS (m/z), [M+H]⁺ calculated 9152.24; measured 9152.58.

Linker 10: MALDI-TOF-MS (m/z), [M+H]⁺ calculated 11117.64; measured 11117.02.

Linker 11: MALDI-TOF-MS (m/z), [M+H]⁺ calculated 13661.20; measured 13661.66.

Linker 12: MALDI-TOF-MS (m/z), [M+H]⁺ calculated 11725.66; measured 11724.23.

These results have demonstrated that the substances obtained above are each a linker of interest.

### [Example 2: Synthesis of DNA used in Examples]

A template DNA was synthesized to prepare an mRNA to be attached to each linker.

The template DNA was prepared by annealing synthetic single-stranded DNA and primers by polymerase chain reaction (PCR). Tables 1-1 and 1-2 show the nucleotide sequence of each synthetic single-stranded DNA used in PCR to prepare each template DNA. In addition, Tables 1-3 shows the sequences of primers used in the Examples described herein. Table 2 shows combinations of each synthetic single-stranded DNA and primers used in the synthesis of each template DNA in the Examples.

**[Table 1-1]**

| Sequence list. Lowercase letters each refer to a DNA. n refers to one of nucleotides having adenine, cytosine, guanine, or thymine as a base. | | |
|---|---|---|
| Name | Sequence | SEQ ID NO: |
| Synthetic single-stranded DNA for a model sequence | taagaaggagatatacatatgcgttgtgttaattttagtgatggttctactcctgctcttcatatttattggggaggtggtggaagtagc | 2 |
| Synthetic single-stranded DNA for Strep-tag II | taagaaggagatatacatatgagtcatcctcaatttgaaaaaggaggtggtggaagtagt | 3 |
| Synthetic single-stranded DNA for FcRn binder | taagaaggagatatacatatgcgcttctgcaccggccacttcggcggcctctacccctgcaacggccccggaggtggtggaagtagt | 4 |
| Synthetic single-stranded DNA for HA binder | taagaaggagatatacatatgtggcacaagaacaagtacgtcctcacctactcctgcctcttcgccgccggcggaggtggtggaagtagc | 5 |
| Synthetic single-stranded DNA for Fc binder | taagaaggagatatacatatgcccgactgcgcctaccacaagggcgagctcgtctggtgcaccggaggtggtggaagtagc | 6 |
| Synthetic single-stranded DNA for mRNA A containing a random region | taagaaggagatatacatatgnntnntnntnnttggggaggtggtggaagtagc | 32 |
| Synthetic single-stranded DNA for mRNA B containing a random region | taagaaggagatatacatatgnntnntnntnntnnttggggaggtggtggaagtagc | 33 |
| Synthetic single-stranded DNA for mRNA C containing a random region | taagaaggagatatacatatgnntnntnntnntnntnnttggggaggtggtggaagtagc | 34 |
| Synthetic single-stranded DNA for mRNA D containing a random region | Taagaaggagatatacatatgnntnntnntnntnntnntnnttggggaggtggtggaagtagc | 35 |

**[Table 1-2]**

| | | |
|---|---|---|
| Synthetic single-stranded DNA for mRNA E containing a random region | taagaaggagatatacatatgnntnntnntnntnntnntnntnnttggggaggtggtggaagtagc | 36 |
| Synthetic single-stranded DNA for mRNA F containing a random region | taagaaggagatatacatatgnntnntnntnntnntnntnntnntnnttggggaggtggtggaagtagc | 37 |
| Synthetic single-stranded DNA for mRNA G containing a random region | taagaaggagatatacatatgnntnntnntnntnntnntnntnntnntnnttggggaggtggtggaagtagc | 38 |
| Synthetic single-stranded DNA for mRNA H containing a random region | taagaaggagatatacatatgnntnntnntnntnntnntnntnntnntnntnnttggggaggtggtggaagtagc | 39 |
| Synthetic single-stranded DNA for mRNA I containing a random region | taagaaggagatatacatatgnntnntnntnnttggggaggtggtggaagtagc | 40 |
| Synthetic single-stranded DNA for mRNA J containing a random region | taagaaggagatatacatatgnntnntnntnntnnttggggaggtggtggaagtagc | 41 |
| Synthetic single-stranded DNA for mRNA K containing a random region | taagaaggagatatacatatgnntnntnntnntnntnnttggggaggtggtggaagtagc | 42 |
| Synthetic single-stranded DNA for mRNA L containing a random region | taagaaggagatatacatatgnntnntnntnntnntnntnnttggggaggtggtggaagtagc | 43 |

**[Table 1-2]**

| Sequence list. Lowercase letters each refer to a DNA. | | |
|---|---|---|
| Name | Sequence | SEQ ID NO: |
| PD-T7g10M.F52 | ctagtaatacgactcactatagggttaactttaagaaggagatatacatatg | 7 |
| TGG-ssG4S2.R44 | cccgcctcccgccccccgtcctagctacttccaccacctcccca | 8 |
| TGG-ssG4S2.R23RT | tagctacttccaccacctcccca | 9 |
| RV_Strep-tag II | cccgcctcccgccccccgtcctaactacttccaccacctcctttttc | 10 |
| RV_FcRn binder | cccgcctcccgccccccgtcctaactacttccaccacctccggg | 11 |
| RV_HA binder | cccgcctcccgccccccgtcctagctacttccaccacctccgcc | 12 |
| RV_Fc binder | cccgcctcccgccccccgtcctagctacttccaccacctccggt | 13 |
| RT_Strep-tag II | taactacttccaccacctccttt | 14 |
| RT_FcRn binder | taactacttccaccacctccggg | 15 |
| RT_HA binder | tagctacttccaccacctccgcc | 16 |
| RT_Fc binder | tagctacttccaccacctccggt | 17 |
| RV_R39_mRNA display | tttccgccccccgtcctagctacttccaccacctcccca | 44 |
| RV_mRNA display_Strep-tag II | tttccgccccccgtcctaactacttccaccacctcctttttc | 45 |
| F70-ID12-07-4 | cacgacgctcttccgatctgtgctgcgcgtttcactatagggttaactttaagaaggagatatacatatg | 46 |
| F70-ID12-07-5 | cacgacgctcttccgatctgcagctgaggaatcactatagggttaactttaagaaggagatatacatatg | 47 |
| R38-3UTR-SBS-R | agacgtgtgctcttccgatcttcccgccccccgtccta | 48 |
| F63-P5-SBS-F | aatgatacggcgaccaccgagatctacacacactctttccctacacgacgctcttccgatct | 49 |
| R58-SBSR-P7 | caagcagaagacggcatacgagatgtgactggagttcagacgtgtgctcttccgatct | 50 |

**[Table 2]**

| Nucleic acid combinations used for synthesis of a template DNA by PCR | | | |
|---|---|---|---|
| Name of template DNA to be produced | Synthetic single-stranded DNA | Forward primer | Reverse primer |
| Template DNA for a model sequence | Synthetic single-stranded DNA for a model sequence | PD-T7g10M.F52 | TGG-ssG4S2. R44 |
| Template DNA for Strep-tag II | Synthetic single-stranded DNA for Strep-tag II | PD-T7g10M.F52 | RV_Strep-tag II |
| Template DNA for FcRn binder | Synthetic single-stranded DNA for FcRn binder | PD-T7g10M.F52 | RV_FcRn binder |
| Template DNA for HA binder | Synthetic single-stranded DNA for HA binder | PD-T7g10M.F52 | RV_HA binder |
| Template DNA for Fc binder | Synthetic single-stranded DNA for Fc binder | PD-T7g10M.F52 | RV_Fc binder |
| Template DNA for mRNA A to H containing a random region | Synthetic single-stranded DNA for mRNA A to H containing a random region | PD-T7g10M.F52 | TGG-ssG4S2. R44 |
| Template DNA for mRNA I to L containing a random region | Synthetic single-stranded DNA for mRNA I to L containing a random region | PD-T7g10M.F52 | RV_R39_mRNA display |
| Template DNA for mRNA display format Strep-tag II | Synthetic single-stranded DNA for Strep-tag II | PD-T7g10M.F52 | RV_mRNA display_Strep-tag II |

Table 3 shows each template DNA obtained by PCR described above and the amino acid sequence of the peptide encoded by the translation region of the above template DNA in the translation reaction in the following Examples. The above template DNA includes T7 promoter sequence, ribosome binding sequence, initiation codon, peptide sequence, spacer peptide sequence, amber codon, and linker hybridization sequence.

**[Table 3-1]**

| Template DNA sequence produced by PCR and amino acid sequence of a peptide encoded by the translation region of the template DNA in each Example. | | |
|---|---|---|
| Name | Sequence | SEQ ID NO: |
| Template DNA for a model sequence | ctagtaatacgactcactatagggttaactttaagaaggagatatacatatgcgttgtgttaattttagtgatggttctact cctgctcttcatatttattggggaggtggtggaagtagctaggacggggggcgggaggcggg | 18 |
| Fluorescent model peptide containing a fluorescein structure encoded by the translation region of SEQ ID NO: 18 | Fph-R-MeA-VNFSDGSTPALHIYWGGGGSS | 19 |
| Template DNA for Strep-tag II | ctagtaatacgactcactatagggttaactttaagaaggagatatacatatgagtcatcctcaatttgaaaaaggaggtg gtggaagtagttaggacggggggcgggaggcggg | 20 |
| Streptavidin-binding peptide encoded by the translation region of SEQ ID NO: 20 | WSHPQFEKGGGGSS | 21 |
| Template DNA for FcRn binder | ctagtaatacgactcactatagggttaactttaagaaggagatatacatatgcgcttctgcaccggccacttcggcggc ctctacccctgcaacggccccggaggtggtggaagtagttaggacggggggcgggaggcggg | 22 |
| FcRn binding peptide encoded by the translation region of SEQ ID NO: 22 | MRFCTGHFGGLYPCNGPGGGGSS | 23 |

**[Table 3-2]**

| Template DNA sequence produced by PCR and amino acid sequence of a peptide encoded by the translation region of the template DNA in each Example. n means one of a, t, g, or c. | | |
|---|---|---|
| Name | Sequence | SEQ ID NO: |
| Template DNA for HA binder | ctagtaatacgactcactatagggttaactttaagaaggagatatacatatgtggcacaagaacaagtacgtcc tcacctactcctgcctcttcgccgccggcggaggtggtggaagtagctaggacggggggcgggaggcggg | 24 |
| HA-binding peptide encoded by the translation region of SEQ ID NO: 24 | CIAcY-WHKNKYVLTYSCLFAAGGGGGSS | 25 |
| Template DNA for Fc binder | ctagtaatacgactcactatagggttaactttaagaaggagatatacatatgcccgactgcgcctaccacaagg gcgagctcgtctggtgcaccggaggtggtggaagtagctaggacggggggcgggaggcggg | 26 |
| Fc-binding peptide encoded by the translation region of SEQ ID NO: 26 | MPDCAYHKGELVWCTGGGGSS | 27 |
| Template DNA for mRNA display format Strep-tag II | ctagtaatacgactcactatagggttaactttaagaaggagatatacatatgagtcatcctcaatttga aaaaggaggtggtggaagtagttaggacggggggcggaaa | 51 |
| Streptavidin-binding peptide encoded by the translation region of SEQ ID NO: 51 | WSHPQFEKGGGGSS | 52 |

In the Table 3-1 and Table 3-2, lower case letters denote DNA, upper case letters denote commonly used amino acid abbreviations, and Fph denotes N-(3',6'-dihydroxy-3-oxo-3-H-spiro[isobenzofuran-1,9'-xanthene]-5-carboxyl)-L-phenylalanine, MeA denotes N-methyl-L-alanine, and ClAcY denotes N-2-chloroacetyl-L-tyrosine. The underline refers to the translation region in each template DNA.

Note that the template DNA (SEQ ID NO: 18) produced by using the "Synthetic single-stranded DNA for a model sequence" in the table includes an oligonucleotide encoding a fluorescent model peptide (SEQ ID NO: 19) containing a fluorescein structure; the template DNA (SEQ ID NO: 20) produced by using the "Synthetic single-stranded DNA for Strep-tag II" includes an oligonucleotide encoding a streptavidin-binding peptide (SEQ ID NO: 21, "Improved affinity of engineered streptavidin for the Strep-tag II peptide is due to a fixed open conformation of the lid-like loop at the binding site", I. P. Korndorefer and A. Skerra, Protein Sci., 2002, 11, 4, 883-893 (NPL 7)); the template DNA (SEQ ID NO: 22) produced by using the "Synthetic single-stranded DNA for FcRn binder" includes an oligonucleotide encoding a human neonatal Fc receptor (FcRn)-binding peptide (SEQ ID NO: 23, "Synthesis and Structure-Activity Relationships of Dimeric Peptide Antagonists of the Human Immunoglobulin G-Human Neonatal Fc Receptor (IgG-FcRn) Interaction", K. A. McDonnell et al., J. Med. Chem., 2010, 53, 4, 1587-1596 (NPL 8)); the template DNA (SEQ ID NO: 24) produced by using the "Synthetic single-stranded DNA for HA binder" includes an oligonucleotide encoding the hemagglutinin (HA)-binding peptide (SEQ ID NO: 25, "Inhibition of H1 and H5 Influenza A Virus Entry by Diverse Macrocyclic Peptides Targeting the Hemagglutinin Stem Region", M. N. Pascha et al., ACS Chem. Biol., 2022, 17, 9, 2425-2436 (NPL 9)); and the template DNA (SEQ ID NO: 26) produced by using the "Synthetic single-stranded DNA for Fc binder" includes an oligonucleotide encoding the human IgG Fc protein (Fc) binding peptide (SEQ ID NO: 27, "Kinetics-Based Structural Requirements of Human Immunoglobulin G Binding Peptides", K. Muguruma et al., ACS Omega, 2019, 4, 11, 14390-14397 (NPL 10)).

According to Table 2, 100 µL of a PCR reaction solution (final concentration: 1× Phusion HF buffer, 200 µM dNTPs, 3% dimethyl sulfoxide (DMSO), 2 units Phusion DNA polymerase) containing each synthetic single-stranded DNA (final concentration: 2 nM) other than the synthetic single-stranded DNA for mRNA A to L containing a random region and the corresponding primers (final concentration: 500 nM) was prepared, and was reacted using a thermal cycler (T100 Thermal Cycler, Bio-Rad) at 98°C for 60 seconds and then 25 cycles (98°C for 10 seconds, 61°C for 30 seconds, and 72°C for 30 seconds). The reaction products were purified using AMPure XP (Beckman Coulter).

In addition, according to Table 2, an extension PCR reaction solution (final concentration: 1 µM corresponding reverse primer, 1× buffer for KOD-Plus-ver. 2 (Toyobo), 200 µM dNTPs, 1.5 mM MgSO₄, 0.02 U/µL KOD plus (Toyobo)) containing each synthetic single-stranded DNA (final concentration: 1 µM, each of SEQ ID NOs: 32 to 43) for mRNA A to L containing a random region was prepared, and was reacted using a thermal cycler (T100 Thermal Cycler, Bio-Rad) at 94°C for 2 minutes, 5 cycles (60°C for 40 seconds and 68°C for 60 seconds) and then 68°C for 60 seconds. Subsequently, the post-reaction solution prepared above was diluted 40-fold with a PCR reaction solution (final concentration: 10 mM Tris-HCl (pH 8.5), 50 mM KCl, 0.1% (v/v) Triton X-100, 2 mM MgCl₂, 250 µM dNTPs, 250 nM corresponding forward primer, 250 nM corresponding reverse primer, Taq DNA Polymerase (NEB) 0.02 U/µL), and was reacted using a thermal cycler (T100 Thermal Cycler, Bio-Rad) at 95°C for 40 seconds, 4 cycles {95°C for 40 seconds, 50°C for 40 seconds, and 72°C for 60 seconds}, and 72°C for 60 seconds. After the reaction, the product was purified by phenol/chloroform extraction and ethanol precipitation, and the obtained pellet was redissolved in ultrapure water.

### [Example 3: Preparation of mRNA-linker conjugate]

### Example 3-1 To prepare mRNA and produce mRNA-linker conjugate

mRNA was synthesized from each template DNA prepared in Example 2 (excluding each template DNA of mRNA A to L containing a random region) by transcription reaction with T7RNA polymerase, and the obtained reaction product was purified using RNAClean XP (Beckman Coulter). After purification, the concentration of mRNA was determined from UV absorption at 260 nm and the mRNA was diluted to 20 µM.

Each template DNA of mRNA A to L containing a random region was diluted 5-fold with Transcription mix (final concentration: 40 mM Tris-HCl (pH 8.0), 1 mM Spermidine, 0.01% (v/v) Triton X-100, 10 mM DTT, 20 mM MgCl₂, 25 mM KOH, 3.75 mM NTPs, 0.24 µM T7 RNA polymerase), and reacted at 37°C for 6 hours. After the reaction, a DNase reaction solution (final concentration: 40 mM Tris-HCl (pH 8.0), 10 mM MgSO₄, 1 mM CaCl₂, 0.025 U/µL RQ1 RNase-Free DNase (Promega)) was added, and the mixture was reacted at 37°C for 1 hour. Finally, the above post-reaction solution was purified by phenol-chloroform extraction, and the concentration of mRNA was determined from UV absorption at 260 nm and the mRNA was diluted to 20 µM.

Each mRNA prepared in this way (excluding mRNAs prepared from template DNAs for mRNA I to L containing a random region) (final concentration: 5 µM) and each linker prepared in Example 1 (final concentration: 5.5 µM) were heated (at 95°C for 3 minutes) in 4-(2-hydroxyethyl)-1-piperazineethanesulfonic acid (HEPES)-KOH (final concentration: 62.5 mM, pH 7.6) and KOAc (final concentration: 375 mM, pH 7.6). The resulting mixture was allowed to stand at room temperature for hybridization. In this way, a 5 µM mRNA-linker conjugate was prepared.
Among the mRNA-linker conjugate thus prepared, each conjugate prepared using mRNA A to H containing a random region was dissolved in 5× loading buffer (final concentration: 7 M urea, 10 mM EDTA, 1 mM Tris-HCl (pH 7.6)) and heated at 95°C for 1 minute. Then, 8% denaturing urea polyacrylamide electrophoresis and SYBR Green II staining were performed, followed by fluorescence imaging using PharosFX (Bio-Rad).

For mRNAs prepared from the template DNAs for mRNA I to L containing a random region, each mRNA (final concentration: 1 µM) was mixed with linker 8 (final concentration: 1.5 µM), 1× Ligation buffer (Takara), DMSO (final concentration: 10% (v/v)), and T4 RNA ligase (Takara, final concentration: 5U/µL)), and a ligation reaction was performed at 37°C for 1 hour. Some of the mRNA-linker conjugates prepared were dissolved in 5× loading buffer (final concentration: 7 M urea, 10 mM EDTA, 1 mM Tris-HCl (pH 7.6)) and heated at 95°C for 1 minute. Then, 8% denaturing urea polyacrylamide electrophoresis and SYBR Green II staining were performed, followed by fluorescence imaging using PharosFX (Bio-Rad).

Fig. 3 shows the results of analyzing mRNA-linker conjugates. For any mRNA-linker conjugate, a fluorescent band(s) originating from each mRNA-linker conjugate was observed at the high molecular weight position relative to the mRNA. This indicates that various linkers are applicable to a wide variety of genetic information materials.

### Example 3-2 To couple puromycin-like substances to mRNA-linker 4 precursor 2 conjugate

mRNA (final concentration: 5 µM) prepared from the template DNA for a model sequence in Example 3-1 and precursor 2 of linker 4 (final concentration: 10 µM) were heated (at 95°C for 3 min) in HEPES-KOH (final concentration: 62.5 mM, pH 7.6) and KOAc (final concentration: 375 mM, pH 76). The resulting mixture was allowed to stand at room temperature for hybridization. In this way, 5 µM mRNA-linker 4 precursor 2 conjugate was prepared. Next, to the above conjugate (final concentration: 3.3 µM) were added alkyne puromycin (final concentration: 53.4 µM), DMSO (final concentration: 10% (v/v)), tris(3-hydroxypropyltriazolylmethyl)amine (THPTA)/copper(II) sulfate solution (final concentrations: THPTA = 10 mM, copper(II) sulfate = 5 mM), and sodium ascorbate (final concentration: 10 mM). The resulting mixture was subjected to a CuAAC (AAC: Azide-Alkyne-cycloaddition) reaction for 2 hours at room temperature. After the CuAAC reaction, purification was performed by ethanol precipitation. The purified material was dissolved in 5× loading buffer (final concentration: 7 M urea, 10 mM EDTA, 1 mM Tris-HCl (pH 7.6)) and heated at 95°C for 1 min. Then, 8% denaturing urea polyacrylamide electrophoresis and SYBR Green I staining were performed, followed by fluorescence imaging using PharosFX (Bio-Rad).

Fig. 4 shows the results of fluorescence imaging. The fluorescent band derived from the mRNA-linker 4 precursor 2 conjugate shifted completely to the high molecular weight side after the CuAAC reaction. This shift should be due to the reaction of alkyne puromycin with each of the two azido groups in the linker. This has demonstrated that an mRNA-linker conjugate with two indirect puromycin parts can be produced by introducing one of a pair of functional groups, such as azide-alkyne, to mRNA and reacting it with puromycin containing the other paired functional group, which can be coupled to each other.

### [Example 4: Preparation of aminoacyl-tRNA]

Aminoacyl-tRNAs for a translation reaction in a cell-free translation system were prepared as follows.

As amino acid-activating esters used for aminoacylation of tRNA by acylation-catalyzing RNA (ARS ribozyme), prepared were: (3',6'-dihydroxy-3-oxo-3-H-spiro[isobenzofuran-1,9'-xanthene]-5-carboxyl)-L-phenylalanine cyanomethyl ester (Fph-CME, "An orthogonal ribosome-tRNA pair via engineering of the peptidyl transferase center.", N. Terasaka et al., Nat. Chem. Biol., 2014, 10, 7, 555-557); L-tryptophan cyanomethyl ester (W-CME, "A highly flexible tRNA acylation method for non-natural polypeptide synthesis ", H. Murakami et al., Nat. Methods, 2006, 3, 5, 357-359 (NPL 12)); N-methyl-L-alanine dinitrobenzyl ester (MeA-DBE, "Messenger RNA-programmed incorporation of multiple N-methyl-amino acids into linear and cyclic peptides.", T. Kawakami et al., Chem. Biol. 2008, 15, 1, 32-42 (NPL 13)); and N-2-chloroacetyl-L-tyrosine cyanomethyl ester (C1AcY-CME, "Macrocyclic peptides exhibit antiviral effects against influenza virus HA and prevent pneumonia in animal models.", M. Saito et al., Nat. Commun., 2021, 12, 1, 2654 (NPL 14)) (the method disclosed in JP 2008-125396 A was used for preparation (PTL 8)).

To attach Fph-CME, W-CME, or C1AcY-CME to tRNA, an enhanced flexizyme (Table 4, eFx, WO2007/066627 (PTL 9)) as ARS ribozyme was used. For MeA-DBE, dinitrobenzyl flexizyme (Table 4, dFx, WO2007/066627 (PTL 9)) was used.

In order to assign Fph, W, and C1AcY to the AUG codon and MeA to the UGC codon, Ini-tRNAs with CAU in the anticodon portion (Table 4, WO2012/026566 (PTL 7)) and tRNA_{GCA} with GCA (Table 4, WO 2019/077887 (PTL 10)), respectively, were used.

For Fph-CME, W-CME and C1AcY-CME (final concentration: 5 mM), eFx (final concentration: 25 µM), Ini-tRNA (final concentration: 25 µM), HEPES-KOH (final concentration: 100 mM, pH 7.5), MgCl₂ (final concentration: 50 mM), and DMSO (final concentration: 20%) were added, and the aminoacylation reaction was carried out at 0°C overnight. For MeA-DBE (final concentration: 5 mM), dFx (final concentration: 25 µM), tRNA_{GCA} (final concentration: 25 µM), HEPES-KOH (final concentration: 100 mM, pH 7.5), MgCl₂ (final concentration: 50 mM), and DMSO (final concentration: 20%) were added, and the aminoacylation reaction was carried out at 0°C overnight. An equal volume of 3M NaOAc (pH 5.2) was added to the above aminoacylated samples, followed by ethanol precipitation.

The pellets were then each redissolved using 0.3 M NaOAc (pH 5.2), and ethanol precipitation was performed again. Finally, the pellets were washed once each with 70% ethanol containing 0.1 M NaOAc (pH 5.2) and 70% ethanol. The resulting aminoacyl-tRNA pellet was dissolved in 0.2% acetic acid.

**[Table 4]**

| Sequence list of ARS ribozymes and tRNAs used in Examples. | | |
|---|---|---|
| Capital letters each refer to the commonly used abbreviation of each RNA. | | |
| Name | Sequence | SEQ ID NO: |
| dFx | GGAUCGAAAGAUUUCCGCAUCCCCGAAAGGGUACAUGGCGUUAGGU | 28 |
| eFx | GGAUCGAAAGAUUUCCGCGGCCCCGAAAGGGGAUUAGCGUUAGGU | 29 |
| Ini-tRNA | GGCGGGGUGGAGCAGCCUGGUAGCUCGUCGGGCUCAUAACCCGAAGAUCGUCGGUUCAAAUCCGGCCCCCGCAACCA | 30 |
| tRNA_{GCA} | GGGUGAUUGGCGCAGCCUGGUAGCGCACUUCGCUGCAAACGAAGGGGUCAGGGGUUCGAAUCCCCUAUCACCCGCCA | 31 |
| tRNA_{GGC} | GGGUGAUUGGCGCAGCCUGGUAGCGCACUUCGCUGGCAGCGAAGGGGUCAGGGGUUCGAAUCCCCUAUCACCCGCCA | 53 |
| tRNA_{GCA} | GGGUGAUUGGCGCAGCCUGGUAGCGCACUUCGCUGCAAACGAAGGGGUCAGGGGUUCGAAUCCCCUAUCACCCGCCA | 54 |
| tRNA_{CCA} | GGGUGAUUGGCGCAGCCUGGUAGCGCACUUCGCUCCAAACGAAGGGGUCAGGGGUUCGAAUCCCCUAUCACCCGCCA | 55 |
| tRNA_{GCG} | GGGUGAUUGGCGCAGCCUGGUAGCGCACUUCGCUGCGGACGAAGGGGUCAGGGGUUCGAAUCCCCUAUCACCCGCCA | 56 |
| tRNA_{GAA} | GGGUGAUUGGCGCAGCCUGGUAGCGCACUUCGCUGAAAACGAAGGGGUCAGGGGUUCGAAUCCCCUAUCACCCGCCA | 57 |
| tRNA_{GAU} | GGGUGAUUGGCGCAGCCUGGUAGCGCACUUCGCUGAUAACGAAGGGGUCAGGGGUUCGAAUCCCCUAUCACCCGCCA | 58 |
| tRNA_{GGU} | GGGUGAUUGGCGCAGCCUGGUAGCGCACUUCGUUGGUAACGAAGGGGUCAGGGGUUCGAAUCCCCUAUCACCCGCCA | 59 |

Table 4 lists the ARS ribozymes and tRNA sequences used in the Examples (SEQ ID NOs: 28-31). Capital letters each refer to the commonly used abbreviation of each RNA.

### [Example 5: Translation reaction (1) using mRNA-linker conjugate]

Translation reactions were performed using each mRNA-linker conjugate prepared in Example 3.

The cell-free translation system used for translation was configured as follows.

50 mM HEPES-KOH [pH7.6], 100 mM KOAc, 20 mM creatine phosphate, 12.5 mM Mg(OAc)₂, 2 mM guanosine triphosphate (GTP), 2 mM adenosine triphosphate (ATP), 1 mM cytidine triphosphate (CTP), 1 mM uridine triphosphate (UTP), 2 mM spermidine, 1 mM dithiothreitol (DTT), 1.5 mg/mL *E. coli* total tRNA (Roche), 1.2 µM ribosome, 2.7 µM initiation factor 1 (IF1), 0.4 µM initiation factor 2 (IF2), 1.5 µM initiation factor 3 (IF3), 0. 25 µM release factor 2 (RF2), 0.17 µM release factor 3 (RF3), 0.5 µM ribosomal recycling factor (RRF), 10 µM elongation factor thermo-unstable (EF-Tu), 10 µM elongation factor thermo-stable (EF-Ts), 0.26 µM elongation factor G (EF-G), 5 µM elongation factor P (EF-P), 0.6 µM methionine transformylase, 4 µg/mL creatine kinase (Roche), 3 µg/mL myokinase (Sigma), 0.1 µM pyrophosphatase, and 0.1 µM nucleoside diphosphate kinase.

To this mixture, a conjugate (final concentration: 1 µM) containing linker 2 or 3 and a model sequence mRNA encoding a fluorescent model peptide containing a fluorescein structure as prepared in Example 3, two types of aminoacyl-tRNAs (final concentrations: 10 µM Fph-Ini tRNA and 10 µM MeA-tRNA_{GCA}), 15 amino acids (final concentrations: 2 mM each of Ala, Arg, Asn, Gly, His, Ile, Leu, Phe, Pro, Ser, Thr, Trp, Val, Asp, and Tyr), and 15 corresponding aminoacyl-tRNA synthetases (final concentrations: 0.73 µM AlaRS, 0.03 µM ArgRS, 0.38 µM AsnRS, 0.09 µM GlyRS, 0.02 µM HisRS, 0.4 µM IleRS, 0.04 µM LeuRS, 0.68 µM PheRS, 0.16 µM ProRS, 0.04 µM SerRS, 0.09 µM ThrRS, 0.03 µM TrpRS, 0.02 µM ValRS, 0.13 µM AspRS, and 0.02 µM TyrRS) were added, and a translation reaction was performed at 37°C for 30 minutes (first translation reaction). After the reaction, ethylenediaminetetraacetic acid (EDTA, final concentration: 12.5 mM) was added, and the mixture was left to stand on ice for 10 minutes to denature/modify the ribosomes.

Next, a second translation reaction was carried out to perform artificial recycling translation. Mg(OAc)₂ (final concentration: 12.5 mM), translation-related solution (final concentration: 23.5 mM HEPES-KOH (pH 7.6), 0.94 mM ATP, 0.94 mM GTP, 0.47 mM CTP, 0.47 mM UTP, 9.4 mM creatine phosphate, 47.0 mM acetic acid potassium, 0.94 mM spermidine, 0.7 mg/mL *E. coli* total tRNA (Roche), 0.47 mM DTT, aminoacyl-tRNA (final concentration: 10 µM Fph-Ini tRNA, 10 µM MeA-tRNA_{GCA})), and ribosomes (final concentration: 1.2 µM) were added, and a translation reaction was carried out at 37°C for 30 min (second translation reaction). After the reaction, EDTA (final concentration: 16.7 mM) was added to stop the translation reaction.

An equal volume of Novex^{™} Tricin SDS Sample Buffer (2×, Thermo Fisher Scientific Inc.) was added to the reaction mixture after translation, and the samples were separated by electrophoresis using 16% tricine-denaturing polyacrylamide electrophoresis (with 1× Novex^{™} Tricin SDS Running Buffer). After electrophoresis, the gel was subjected to fluorescence imaging using a PharosFX system (Bio-Rad) (excitation: the fluorescein or Cy5 mode; filter: fluorescein or Cy5).

Fig. 5 shows the results. Figs. 5a and 5b show the images obtained when fluorescence derived from Cy5 and fluorescein, respectively, was detected. Linker 2 is a linker with the 5' end labeled with Cy5, and when it is conjugated to a fluorescent model peptide containing a fluorescein structure, the fluorescent bands derived from Cy5 and fluorescein are superimposed. When the mRNA-linker 2 conjugate was translated under the conditions including the amino acids required for translation, a fluorescent band overlapping Cy5 and fluorescein was positioned on the high-molecular-weight side relative to the conjugate. The above results indicate that the peptide-linker conjugation can be checked by molecular weight shift while using a fluorescein-labeled peptide.

In the case where the mRNA-linker 3 conjugate was translated, a fluorescent band was observed only under conditions containing the amino acids required for translation. Furthermore, the fluorescein-derived fluorescent band obtained appeared at a higher molecular weight position compared to that of linker 2, suggesting that the observed fluorescent band is derived from the mRNA-linker 3-peptide conjugate. In addition, two fluorescent bands derived from the peptide were obtained when translation was performed using the mRNA-linker 3 conjugate. This may be due to the difference in molecular weight as caused by the difference in the number of peptides conjugated to the end of linker 3.

Further, even without artificial recycling translation, the use of a divalent linker (linker 3) resulted in the appearance of two fluorescent bands. When artificial recycling translation was applied, the intensity of the higher molecular weight fluorescent band of the two fluorescent bands increased. This suggests that the increase in fluorescence intensity of this fluorescent band of the two fluorescent bands is due to the successive addition of peptide to the linker that had already conjugated with a single peptide during the first round of translation.

These results indicate that an mRNA-linker-peptide conjugate with multiple peptides was obtained by translating an mRNA-linker conjugate in a cell-free translation system, and that the mRNA-linker-peptide conjugate can be efficiently provided by artificial recycling translation.

### [Example 6: Translation reaction (2) using mRNA-linker conjugate]

An mRNA-linker conjugate was prepared by hybridizing a model sequence mRNA encoding a fluorescein structure-containing fluorescent model peptide with either linker 2 or 3. The mRNA-linker conjugate was used to perform artificial recycling translation under the same conditions as in Example 5. Finally, EDTA (final concentration: 5 mM) was added to stop the translation reaction.

In this Example, nuclease treatment was performed in addition to the operation in Example 5 to improve the resolution in electrophoresis. Specifically, RNase H (NEB, 2.5 units) and RNase T1 (Thermo Fisher Scientific Inc., 200 units) were added to the translation solution, and nuclease treatment was performed at 37°C for 2 hours. After the nuclease treatment, tricine-denaturing polyacrylamide electrophoresis and fluorescence imaging were performed under the same conditions as in Example 5.

Fig. 6 shows the results obtained. Figs. 6a and 6c show the images obtained when fluorescence derived from fluorescein was detected. In the mRNA-linker conjugate with a divalent linker (linker 3) after artificial recycling translation, nuclease treatment resulted in two distinct fluorescent bands compared to those before treatment (Fig. 6a). On the other hand, in the mRNA-linker conjugate with a monovalent linker (linker 2) after artificial recycling translation, there was only one fluorescent band originating from the peptide, even when the fractionation ability was improved by nuclease treatment (Fig. 6c). This indicates that multiple peptides are conjugated to the mRNA-linker conjugate after artificial recycling translation in the case of using a divalent linker (linker 3).

Furthermore, artificial recycling translation enhanced the intensity of the peptide-derived fluorescent band (Figs. 6b and d), indicating that repeated translation reactions can increase the proportion of mRNA-linker conjugate covalently bonded to the peptide.

### [Example 7: Translation reaction (3) using mRNA-linker conjugate]

An mRNA-linker conjugate was prepared by hybridizing a model sequence mRNA encoding a fluorescein structure-containing fluorescent model peptide with either linker 2 or 3. The mRNA-linker conjugate was used to perform artificial recycling translation under the same conditions as in Example 5. In this Example, in addition to the operation in Example 5, reverse transcription was performed in the reaction solution containing the translation product.

Specifically, the reaction solution containing the translation product obtained above was admixed with a reverse transcription reaction solution (final concentration: 49.5 mM Tris-HCl, 74.2 mM KCl, 18.4 mM MgCl₂, 1 mM DTT, 0.3 mM deoxynucleotide triphosphate (dNTP)s, 20 units/µL MLV reverse transcriptase, 12 µM TGG-ssG4S2.R23RT (SEQ ID NO: 9)), and a reverse transcription reaction was performed at 42°C for 30 minutes. After the reverse transcription, tricine-denaturing polyacrylamide electrophoresis and fluorescence imaging were performed under the same conditions as in Example 5.

Fig. 7 shows the results obtained. Fig. 7 shows the image obtained when fluorescence derived from fluorescein was detected. TGG-ssG4S2.R23RT was hybridized to the mRNA-linker2-peptide conjugate, so that the fluorescent band derived from the conjugate shifted to the high-molecular-weight side. The mRNA-linker-peptide conjugate hybridized to TGG-ssG4S2.R23RT shifted further to the high-molecular-weight side after the reverse transcription reaction.

In the mRNA-linker3-peptide conjugate, multiple fluorescent bands were observed, unlike the case of linker 2. This indicates that multiple peptides can be conjugated to the divalent linker and that a reverse transcription reaction can be performed.

### [Example 8: Translation reaction (4) using mRNA-linker conjugate]

An mRNA-linker conjugate was prepared by hybridizing a model sequence mRNA encoding a fluorescein structure-containing fluorescent model peptide with either linker 2, 3, 4, 5, or 6. The mRNA-linker conjugate was used to perform artificial recycling translation under the same conditions as in Example 6.

Fig. 8 shows the results obtained. Fig. 8 shows an image where the images obtained when fluorescence derived from Cy5 or fluorescein was detected were merged. For the mRNA-linker conjugate with a monovalent linker (linker 2), there was only one fluorescent band originating from the peptide. In contrast, each mRNA-linker conjugate with linker 3, 4, 5, or 6 having two puromycin parts yielded two fluorescent bands derived from the peptide.
The above results indicate that divalent linkers can each yield an mRNA-linker-peptide conjugate in which multiple peptides are conjugated even if the length of each puromycin spacing in the linker varies.

### [Example 9: Translation reaction (5) using mRNA-linker conjugate]

### Example 9-1: Specific cleavage of linker 12 by RNase T1

Linker 4 or linker 12 was enzymatically treated in RNase T1 solution (200U RNase T1 (Thermo Fisher Scientific Inc.), 50 mM Tris-HCl (pH 7.5), 2 mM EDTA) at 37°C overnight. The enzyme-treated samples were subjected to denaturing urea polyacrylamide electrophoresis and SYBR Green II staining, followed by fluorescence imaging using PharosFX (Bio-Rad).

Fig. 9a shows the results obtained. Linker 4, which does not have a guanine-based deoxynucleotide, did not cause a change in the position of the fluorescent band after enzyme treatment. On the other hand, the fluorescence band of linker 12 modified with a guanine-based deoxynucleotide just before the branching part was shifted to the low-molecular-weight side upon enzyme treatment. The results show that such a modification allows for specific cleavage.

### Example 9-2: Analysis of displayed peptides by tricin PAGE

An mRNA-linker conjugate was prepared by hybridizing a model sequence mRNA encoding a fluorescein structure-containing fluorescent model peptide with linker 12. The mRNA-linker conjugate was used to perform artificial recycling translation under the same conditions as in Example 5. Finally, EDTA (final concentration: 5 mM) was added to stop the translation reaction.

In this Example, nuclease treatment was performed in addition to the operation in Example 5 to improve the resolution in electrophoresis. Specifically, RNase T1 (Thermo Fisher Scientific Inc., 200 units) was added to the translation solution, and nuclease treatment was performed at 37°C for 2 hours. After the nuclease treatment, tricine-denaturing polyacrylamide electrophoresis and fluorescence imaging were performed under the same conditions as in Example 5.

Fig. 9b shows the results obtained. Fig. 9b shows the image obtained when each fluorescence derived from Cy5 and fluorescein was detected and superimposed. In the mRNA-linker conjugate with a divalent linker (linker 12) after artificial recycling translation, nuclease treatment resulted in two distinct fluorescent bands compared to those before treatment (Fig. 9b). On the other hand, in the mRNA-linker conjugate with a monovalent linker (linker 2) after artificial recycling translation, there was only one fluorescent band originating from the peptide, even when the fractionation ability was improved by nuclease treatment (Fig. 9b). This indicates that multiple peptides are conjugated to the mRNA-linker conjugate after artificial recycling translation in the case of using a divalent linker (linker 12).

### [Example 10: Display assay with mRNA-linker-peptide conjugate using Strep-tagII as model peptide]

A display assay was performed using an mRNA-linker-peptide conjugate with Strep-tagII as a model peptide to evaluate the recovery rate upon binding to streptavidin.

The following experiments were performed using mRNA-linker conjugates, in which the mRNA encoding Strep-tagII was hybridized with linker 1 or linker 3 to 6, as prepared in Example 3.

To the cell-free translation system constructed in Example 5, the following components were added: the mRNA-linker conjugate (final concentration: 1 µM), one type of aminoacyl-tRNA (final concentration: 10 µM W Ini-tRNA), 9 amino acids (final concentration: 2 mM each; Gly, His, Phe, Pro, Ser, Gln, Glu, Lys, Trp), and 9 aminoacyl-tRNA synthetases (final concentrations: 0.09 µM GlyRS, 0.02 µM HisRS, 0.68 µM PheRS, 0.16 µM ProRS, 0.04 µM SerRS, 0.06 µM GlnRS, 0.23 µM GluRS, 0.11 µM LysRS, 0.03 µM TrpRS). A translation reaction (first translation reaction) was then carried out at 37°C for 30 minutes. After the reaction, EDTA was added to the samples without artificial recycling translation to a final concentration of 16.7 mM, and to the samples with artificial recycling translation to a final concentration of 12.5 mM. The mixtures were then left to stand on ice for 10 minutes to modify the ribosomes.

Further, a second translation reaction was carried out for the samples with artificial recycling translation.

To the reaction solution after the first translation reaction described above, Mg(OAc)₂ (final concentration: 12.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT, aminoacyl-tRNA [final concentration: 10 µM W Ini-tRNA]), and ribosomes (final concentration: 1.2 µM) were added, and a translation reaction (second translation reaction) was carried out at 37°C for 30 minutes. After the translation reaction, EDTA (final concentration: 16.7 mM) was added to stop the translation reaction.

Next, the reaction solution containing the translation product obtained above was admixed with a reverse transcription reaction solution (final concentration: 49.5 mM Tris-HCl, 74.2 mM KCl, 18.4 mM MgCl₂, 1 mM DTT, 0.30 mM dNTPs, 20 units/µL MLV reverse transcriptase, 12 µM RT_Strep-tag II (SEQ ID NO: 14)), and a reverse transcription reaction was performed at 42°C for 30 minutes.

After the reverse transcription reaction, EDTA (final concentration: 16.7 mM) and HEPES (final concentration: 53.4 mM) were added, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

After desalting, magnetic beads (final concentration: 1 mg/mL, Dynabeads^{™} M-280 Streptavidin, Thermo Fisher Scientific Inc.) with immobilized streptavidin as a target protein were added, and a binding reaction was performed at 4°C for 1 hour. At this time, as a negative control, the desalted sample was added to magnetic beads with immobilized Protein G (final concentration: 1 mg/mL, Dynabeads^{™} Protein G for Immunoprecipitation, Thermo Fisher Scientific Inc.), and a binding reaction was performed while mixing at 4°C for 1 hour. After the binding reaction, the magnetic beads were magnetically separated and the supernatant was removed. The magnetic beads were then resuspended (0.5 mg/mL) using HBS-T, transferred to a new sample tube, and mixed at 4°C for 5 minutes. Furthermore, the sequence of magnetic separation, removal of the supernatant, resuspension in HBS-T, and mixing at 4°C for 5 minutes was repeated twice in total. Finally, after magnetic separation and removal of the supernatant, the samples were resuspended (2 mg/mL) in PCR solution (10 mM Tris-HCl (pH 8.5), 50 mM KCl, 0.1% Triton X-100) and heated at 95°C for 5 minutes. After heating, magnetic separation was performed and the supernatant was collected.

The amount of DNA in the solution before the addition of the target protein after desalting and the amount of DNA after recovery from the magnetic beads were quantified by real-time PCR. A LightCycler 96 (Roche Applied Science) was used as the real-time PCR machine. The reaction solution was prepared by adding Taq polymerase, SYBR Green I (100,000-fold dilution, Invitrogen), dNTPs (final concentration: 0.25 mM), MgCl₂ (final concentration: 2 mM), T7g10M.F52 (0.25 µM), RV_Strep-tag II (SEQ ID NO: 10) (0.25 µM), and the measurement sample solution to the above-described PCR solution, and was used for measurement.

Fig. 10 shows the results of three trials of the above display assay. Here, the recovery rate (%) is the ratio of the amount of DNA recovered from the magnetic beads to the amount of DNA in the solution before the addition of the target protein. In the evaluation of the ability of Strep-tag II as a model peptide to bind to streptavidin as a model target protein, respectively, the use of a divalent linker (linker 3) significantly improved the recovery rate of DNA due to binding to the target protein when compared to the case of using a monovalent linker (linker 1). Further, the above-mentioned enhanced recovery rate was also observed in artificial recycling translation (Fig. 10a). When compared with divalent linkers (linkers 3 to 6) with different branched chain lengths, the above recovery rates showed different enhancement rates depending on the kind of linker branched chain length (Fig. 10b). These results suggest that the ability to bind to the target protein was enhanced by the avidity effect due to the multiple peptides conjugated to the above linker.

### [Example 11: Display assay with mRNA-linker-peptide conjugate using Fc binder as model peptide]

### Example 11-1: Biotinylation of Fc

Recombinant human IgG1 Fc protein (final concentration: 29 µM, 110-HG, R&D Systems) and EZ-Link NHS-PEG4-Biotin (final concentration: 290 µM, Thermo Fisher Scientific Inc.) were reacted in 1× phosphate buffer saline (PBS) at 4°C overnight. After the reaction, purification was performed using a Bio-Gel P30 Gel (Bio-Rad) equilibrated with 1× PBS.

### Example 11-2 Immobilization of biotinylated Fc on magnetic beads

Biotinylated Fc (2.8 µM) prepared as described above was mixed with magnetic beads (final concentration: 31 mg/mL, Dynabeads^{™} M-280 streptavidin, Thermo Fisher Scientific Inc.) at 4°C for 20 minutes. The mixed magnetic beads were magnetically separated and the supernatant was removed, followed by resuspension in HBS-T. Further, the sequence of magnetic separation, removal of the supernatant, and resuspension in HBS-T was repeated twice in total. Finally, after magnetic separation and removal of the supernatant, the samples were resuspended in HBS-T (final concentration: 10 mg/mL).

### Example 11-3: Display assay with mRNA-linker-peptide conjugate using Fc binder as model peptide

The following experiments were performed using mRNA-linker conjugates, in which the mRNA encoding Fc binder was hybridized with linker 1 or linker 3, as prepared in Example 3.

To the cell-free translation system constructed in Example 5, the following components were added: the mRNA-linker conjugate (final concentration: 1 µM), 15 amino acids (2 mM each: Leu, Met, Val, Ser, Pro, Thr, Ala, Tyr, His, Lys, Asp, Glu, Cys, Trp, Gly), and 15 aminoacyl-tRNA synthetases (0.04 µM LeuRS, 0.03 µM MetRS, 0.02 µM ValRS, 0.04 µM SerRS, 0.16 µM ProRS, 0.09 µM ThrRS, 0.73 µM AlaRS, 0.02 µM TyrRS, 0.02 µM HisRS, 0.11 µM LysRS, 0.13 µM AspRS, 0.23 µM GluRS, 0.02 µM CysRS, 0.03 µM TrpRS, 0.09 µM GlyRS). A translation reaction was then carried out at 37°C for 30 minutes. After the reaction, EDTA was added to the samples without artificial recycling translation to a final concentration of 16.7 mM, and to the samples with artificial recycling translation to a final concentration of 12.5 mM. The mixtures were then left to stand on ice for 10 minutes to modify the ribosomes.

Further, a second translation reaction was carried out for the samples with artificial recycling translation.

To the reaction solution after the first translation reaction described above, Mg(OAc)₂ (final concentration: 12.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT), and ribosomes (final concentration: 1.2 µM) were added, and a translation reaction was carried out at 37°C for 30 minutes. After the reaction, EDTA (final concentration: 16.7 mM) was added to stop the translation reaction.

Next, the reaction solution containing the translation product obtained above was admixed with a reverse transcription reaction solution (final concentration: 49.5 mM Tris-HCl, 74.2 mM KCl, 18.4 mM MgCl₂, 1 mM DTT, 0.30 mM dNTPs, 20 units/µL MLV reverse transcriptase, 12 µM RT_Fc binder (SEQ ID NO: 17)), and a reverse transcription reaction was performed at 42°C for 30 minutes.

After the reverse transcription reaction, EDTA (final concentration: 16.7 mM) and oxidized glutathione (final concentration: 1 mM, Nacalai) were added, and a reaction was carried out at 37°C for 1 hour.

HEPES (final concentration: 53.4 mM) was added to the above samples, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

The desalted sample was mixed with the above Fc-immobilized magnetic beads (final concentration: 6.2 mg/mL) or Fc-non-immobilized magnetic beads (final concentration: 6.2 mg/mL, Dynabeads^{™} M-280 streptavidin, Thermo Fisher Scientific Inc.), and a binding reaction was then performed while mixing at 4°C for 1 hour. After the binding reaction, the magnetic beads were magnetically separated and the supernatant was removed. The magnetic beads were then resuspended (1.5 mg/mL) using HBS-T, transferred to a new sample tube, and mixed at 4°C for 5 minutes. Furthermore, the sequence of magnetic separation, removal of the supernatant, resuspension in HBS-T, and mixing at 4°C for 5 minutes was repeated twice in total. Finally, after magnetic separation and removal of the supernatant, the samples were resuspended (12.4 mg/mL) in PCR solution (10 mM Tris-HCl (pH 8.5), 50 mM KCl, 0.1% Triton X-100) and heated at 95°C for 5 minutes. After heating, magnetic separation was performed and the supernatant was collected.

The amount of DNA in the solution before the addition of the target protein after desalting and the amount of DNA after recovery from the magnetic beads were quantified by real-time PCR. A LightCycler 96 (Roche Applied Science) was used as the real-time PCR machine. The reaction solution was prepared by adding Taq polymerase, SYBR Green I (100,000-fold dilution, Invitrogen), dNTPs (final concentration: 0.25 mM), MgCl₂ (final concentration: 2 mM), T7g10M.F52 (0.25 µM), RV_Fc binder (SEQ ID NO: 13) (0.25 µM), and the measurement sample solution to the above-described PCR solution, and was used for measurement.

Fig. 11 shows the results of three trials of the above display assay. Here, the recovery rate (%) is the ratio of the amount of DNA recovered from the magnetic beads to the amount of DNA in the solution before the addition of the target protein. In the evaluation of recovery rates using Fc binder and Fc as a model peptide and a model target protein, DNA was recovered in high yield only when a divalent linker (linker 3) was used. Thus, a high recovery rate was obtained by using the divalent linker of interest. In addition, a higher recovery rate was obtained in the case of artificial recycling translation than in the case of no artificial recycling translation. Artificial recycling translation was effective in recovering target-bound conjugates in high yield. These results suggest that the ability to bind to the target protein was enhanced by the avidity effect due to the multiple peptides conjugated to the above linker.

### [Example 12: Display assay with mRNA-linker-peptide conjugate using HA binder as model peptide]

### Example 12-1: Immobilization of HA on magnetic beads

Influenza A H5N1 (A/Indonesia/5/2005) hemagglutinin/HA protein-HIS-tag (final concentration: 4.5 µM, 11060-V08B, ShinoBiological, Inc.) and magnetic beads (final concentration: 4 mg/mL, Dynabeads^{™} His-tag Isolation and Pulldown, Thermo Fisher Scientific Inc.) were mixed at 4°C for 20 minutes. The mixed magnetic beads were magnetically separated and the supernatant was removed, followed by resuspension in HBS-T. Further, the sequence of magnetic separation, removal of the supernatant, and resuspension in HBS-T was repeated twice in total. Finally, after magnetic separation and removal of the supernatant, the samples were resuspended in HBS-T (final concentration: 10 mg/mL).

### Example 12-2: Display assay with mRNA-linker-peptide conjugate using HA binder as model peptide]

The following experiments were performed using mRNA-linker conjugates, in which the mRNA encoding HA binder was hybridized separately with linker 1 or linker 3, as prepared in Example 3.

To the cell-free translation system constructed in Example 5, the following components were added: the mRNA-linker conjugate (final concentration: 1 µM), one aminoacyl-tRNA (10 µM ClAcY Ini-tRNA), 13 amino acids (2 mM each: Phe, Leu, Val, Ser, Thr, Ala, Tyr, His, Asn, Lys, Cys, Trp, Gly), and 12 aminoacyl-tRNA synthetases (0.68 µM PheRS, 0.04 µM LeuRS, 0.02 µM ValRS, 0.04 µM SerRS, 0.09 µM ThrRS, 0.73 µM AlaRS, 0.02 µM TyrRS, 0.02 µM HisRS, 0.38 µM AsnRS, 0.11 µM LysRS, 0.02 µM CysRS, 0.03 µM TrpRS, 0.09 µM GlyRS). A translation reaction was then carried out at 37°C for 30 minutes. After the reaction, EDTA was added to the samples without artificial recycling translation to a final concentration of 16.7 mM, and to the samples with artificial recycling translation to a final concentration of 12.5 mM. The mixtures were then left to stand on ice for 10 minutes to modify the ribosomes.

Further, to the reaction solution after the first translation reaction described above, Mg(OAc) ₂ (final concentration: 12.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT), aminoacyl-tRNA (final concentration: 10 µM ClAcY Ini-tRNA), and ribosomes (final concentration: 1.2 µM) were added, and a translation reaction was carried out at 37°C for 30 minutes. After the reaction, EDTA (final concentration: 16.7 mM) was added to stop the translation reaction.

Next, the reaction solution containing the translation product obtained above was admixed with a reverse transcription reaction solution (final concentration: 49.5 mM Tris-HCl, 74.2 mM KCl, 18.4 mM MgCl₂, 1 mM DTT, 0.30 mM dNTPs, 20 units/µL MLV reverse transcriptase, 12 µM RT_HA binder (SEQ ID NO: 16)), and a reverse transcription reaction was performed at 42°C for 30 minutes.

After the reverse transcription reaction, EDTA (final concentration: 16.7 mM) and HEPES (final concentration: 53.4 mM) were added, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

The desalted sample was mixed with the above HA-immobilized magnetic beads (final concentration: 4 mg/mL) or HA-non-immobilized magnetic beads (final concentration: 4 mg/mL, Dynabeads^{™} His-tag Isolation and Pulldown, Thermo Fisher Scientific Inc.), and a binding reaction was then performed while mixing at 4°C for 1 hour. After the binding reaction, the magnetic beads were magnetically separated and the supernatant was removed. The magnetic beads were then resuspended (2 mg/mL) using HBS-T, transferred to a new sample tube, and mixed at 4°C for 5 minutes. Furthermore, the sequence of magnetic separation, removal of the supernatant, resuspension in HBS-T, and mixing at 4°C for 5 minutes was repeated twice in total. Finally, after magnetic separation and removal of the supernatant, the samples were resuspended (8 mg/mL) in PCR solution (10 mM Tris-HCl (pH 8.5), 50 mM KCl, 0.1% Triton X-100) and heated at 95°C for 5 minutes. After heating, magnetic separation was performed and the supernatant was collected.

The amount of DNA in the solution before the addition of the target protein after desalting and the amount of DNA after recovery from the magnetic beads were quantified by real-time PCR. A LightCycler 96 (Roche Applied Science) was used as the real-time PCR machine. The reaction solution was prepared by adding Taq polymerase, SYBR Green I (100,000-fold dilution, Invitrogen), dNTPs (final concentration: 0.25 mM), MgCl₂ (final concentration: 2 mM), T7g10M.F52 (0.25 µM), RV_HA binder (SEQ ID NO: 12) (0.25 µM), and the measurement sample solution to the above-described PCR solution, and was used for measurement.

Fig. 12 shows the results of three trials of the above display assay. Here, the recovery (%) is the ratio of the amount of DNA recovered from the magnetic beads to the amount of DNA in the solution before the addition of the target protein. In the evaluation of the ability of HA binder as a model peptide to bind to HA as a model target protein, respectively, the use of a divalent linker (linker 3) resulted in an increase in the recovery rate of DNA due to binding to the target protein when compared to the case of using a monovalent linker (linker 1). By using the linker of interest, mRNA-linker-peptide conjugates bound to the target protein were recovered in high yield. Furthermore, the recovery rate was enhanced by artificial recycling translation, indicating that the target-bound mRNA-linker-peptide conjugates can be recovered efficiently when artificial recycling translation is performed. These results suggest that the ability to bind to the target protein was enhanced by the avidity effect due to the multiple peptides conjugated to the above linker.

### [Example 13: Display assay with mRNA-linker-peptide conjugate using FcRn binder as model peptide]

### Example 13-1: Preparation of FcRn-immobilized beads

Magnetic beads (final concentration: 9.3 mg/mL, Dynabeads^{™} M-280 Streptavidin, Thermo Fisher Scientific Inc.) and biotinylated FcRn (0.7 µM, FCM-H82W7, ACRObiosystems) were mixed at 4°C for 20 minutes. The mixed magnetic beads were magnetically separated and the supernatant was removed, followed by resuspension in HBS-T. Further, the sequence of magnetic separation, removal of the supernatant, and resuspension in HBS-T was repeated twice in total. Finally, after magnetic separation and removal of the supernatant, the samples were resuspended in HBS-T (final concentration: 10 mg/mL).

### Example 13-2: Display assay with mRNA-linker-peptide conjugate using FcRn binder as model peptide

The following experiments were performed using mRNA-linker conjugates, in which the mRNA encoding FcRn binder was hybridized separately with linker 1 or linkers 3 to 6, as prepared in Example 3.

To the cell-free translation system constructed in Example 5, the following components were added: the mRNA-linker conjugate (final concentration: 1 µM), 12 amino acids (2 mM each: Phe, Leu, Met, Ser, Pro, Thr, Tyr, His, Asn, Cys, Arg, Gly), and 12 aminoacyl-tRNA synthetases (0.68 µM PheRS, 0.04 µM LeuRS, 0.03 µM MetRS, 0.04 µM SerRS, 0.16 µM ProRS, 0.09 µM ThrRS, 0.02 µM TyrRS, 0.02 µM HisRS, 0.38 µM AsnRS, 0.02 µM CysRS, 0.03 µM ArgRS, 0.09 µM GlyRS). A translation reaction was then carried out at 37°C for 30 minutes. After the reaction, EDTA was added to a final concentration of 12.5 mM, and the samples were allowed to stand on an ice bath for 10 minutes to modify the ribosomes.

Further, to the reaction solution after the first translation reaction described above, Mg(OAc)₂ (final concentration: 12.5 mM) and a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT), and ribosomes (final concentration: 1.2 µM) were added, and a translation reaction was carried out at 37°C for 30 minutes. After the reaction, EDTA (final concentration: 16.7 mM) was added to stop the translation reaction.

Next, the reaction solution containing the translation product obtained above was admixed with a reverse transcription reaction solution (final concentration: 49.5 mM Tris-HCl, 74.2 mM KCl, 18.4 mM MgCl₂, 1 mM DTT, 0.30 mM dNTPs, 20 units/µL MLV reverse transcriptase, 12 µM RT_FcRn binder (SEQ ID NO: 15)), and a reverse transcription reaction was performed at 42°C for 30 minutes.

After the reverse transcription reaction, EDTA (final concentration: 16.7 mM) and oxidized glutathione (final concentration: 1 mM, Nacalai) were added, and a reaction was carried out at 37°C for 1 hour.

HEPES (final concentration: 53.4 mM) was added to the above sample, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

The desalted sample was mixed with the FcRn-immobilized magnetic beads (final concentration: 3.2 mg/mL), and a binding reaction was then performed while mixing at 4°C for 1 hour. After the binding reaction, the magnetic beads were magnetically separated and the supernatant was removed. The magnetic beads were then resuspended (1.6 mg/mL) using HBS-T, transferred to a new sample tube, and mixed at 4°C for 5 minutes. Furthermore, the sequence of magnetic separation, removal of the supernatant, resuspension in HBS-T, and mixing at 4°C for 5 minutes was repeated twice in total. Finally, after magnetic separation and removal of the supernatant, the samples were resuspended (3.2 mg/mL) in PCR solution (10 mM Tris-HCl (pH 8.5), 50 mM KCl, 0.1% Triton X-100) and heated at 95°C for 5 minutes. After heating, magnetic separation was performed and the supernatant was collected.

The amount of DNA in the solution before the addition of the target protein after desalting and the amount of DNA after recovery from the magnetic beads were quantified by real-time PCR. A LightCycler 96 (Roche Applied Science) was used as the real-time PCR machine. The reaction solution was prepared by adding Taq polymerase, SYBR Green I (100,000-fold dilution, Invitrogen), dNTPs (final concentration: 0.25 mM), MgCl₂ (final concentration: 2 mM), T7g10M.F52 (0.25 µM), RV_FcRn binder (SEQ ID NO: 11) (0.25 µM), and the measurement sample solution to the above-described PCR solution, and was used for measurement.

Fig. 13 shows the results of three trials of the above display assay. Here, the recovery (%) is the ratio of the amount of DNA recovered from the magnetic beads to the amount of DNA in the solution before the addition of the target protein. In the evaluation of the ability of FcRn binder as a model peptide to bind to FcRn as a model target protein, respectively, the use of every divalent linker (linkers 3 to 6) resulted in an increase in the recovery rate of DNA due to binding to the target protein when compared to the case of using a monovalent linker (linker 1). Use of the linker of interest resulted in an increase in the rate of recovering mRNA-linker-peptide conjugates bound to the target protein. In addition, the recovery rate was higher as the length of linker branched chain became shorter. It has been demonstrated that the rate of recovering mRNA-linker-peptide conjugates bound to the target protein was improved by selecting an appropriate length of linker branched chain. This suggests that the ability to bind to the target protein was enhanced by the avidity effect due to the multiple peptides conjugated to the above linker.

### [Example 14: Screening for target-binding peptides by using library with randomized peptides]

### Example 14-1: Preparation of aminoacyl-tRNAs

Aminoacyl-tRNAs for a translation reaction in a cell-free translation system were prepared as follows.

As amino acid-activating esters for tRNA aminoacylation using acylation catalyst RNA (ARS ribozyme), the following compounds were prepared: (2,6-dichloropyridin-4-yl)methyl methyl-L-alaninate hydrochloride (MeA-DCPE), (2,6-dichloropyridin-4-yl)methyl-L-cysteinate hydrochloride (Cys-DCPE), 2,2,2-trifluoroethyl methyl-L-phenylalaninate hydrochloride (MeF-TEE), (2,6-dichloropyridin-4-yl)methyl N-methylglycinate hydrochloride (MeG-DCPE), 2,2,2-trifluoroethyl (2-chloroacetyl)-L-phenylalaninate (ClAc-F-TEE), (2,6-dichloropyridin-4-yl)methyl (S)-2-(methylamino)hexanoate hydrochloride (MeNle-DCPE), and 2,2,2-trifluoroethyl L-tryptophanate hydrochloride (Trp-TEE). These were prepared according to the method disclosed in WO2023/234425 (PTL 11).

To attach MeF-TEE, ClAc-F-TEE, or Trp-TEE to tRNA, an enhanced flexizyme was used as ARS ribozyme (Table 4, eFx, WO2007/ 066627 (PTL 9)). For MeA-DCPE, Cys-DCPE, MeG-DCPE, and MeNle-DCPE, dinitrobenzyl flexizyme (Table 4, dFx, WO2007/066627 (PTL 9)) was used.

At that time, ClAc-F was assigned to the AUG codon, MeA to the GCC codon, Cys to the TGG codon, MeF to the UUC codon, MeG to the AUC codon, MeNle to the ACC codon, and Trp to the TGC codon, respectively. Ini-tRNAs with CAU as the anticodon portion (Table 4, WO2012/026566 (PTL 7)), tRNA_{GGC} with GGC, tRNA_{CCA} with CCA, tRNA_{GAA} with GAA, tRNA_{GAU} with GAU, tRNA_{GGU} with GGU, and tRNA_{GCA} with GCA (Table 4, WO2019/077887 (PTL 10)) were used, respectively.

For ClAc-F-TEE (final concentration: 5 mM), the corresponding ARS ribozyme (final concentration: 25 µM), Ini-tRNA (final concentration: 25 µM), bicine (final concentration: 50 mM, pH 9.0), MgCl₂ (final concentration: 50 mM), and DMSO (final concentration: 20%) were added, and the aminoacylation reaction was carried out at 0°C overnight. For Cys-DCPE (final concentration: 5 mM), the corresponding ARS ribozyme (final concentration: 25 µM), the corresponding tRNA (final concentration: 25 µM), HEPES-KOH (final concentration: 50 mM, pH 7.5), MgCl₂ (final concentration: 20 mM), DMSO (final concentration: 20%), and DTT (final concentration: 5 mM) were added, and the aminoacylation reaction was carried out at 0°C overnight. For other amino acid-activating esters (final concentration: 5 mM), the corresponding ARS ribozyme (final concentration: 25 µM), corresponding tRNA (final concentration: 25 µM), HEPES-KOH (final concentration: 50 mM, pH 7.5), MgCl₂ (final concentration: 50 mM), and DMSO (final concentration: 20%) were added, and the aminoacylation reaction was carried out at 0°C overnight. An equal volume of 3M NaOAc (pH 5.2) was added to the above aminoacylated samples, followed by ethanol precipitation.

The pellets were then each redissolved using 0.3 M NaOAc (pH 5.2), and ethanol precipitation was performed again. Finally, the pellets were washed once each with 70% ethanol containing 0.1 M NaOAc (pH 5.2) and 70% ethanol. The resulting aminoacyl-tRNA pellet was dissolved in 0.2% acetic acid.

Table 4 lists the ARS ribozymes and tRNA sequences used in the Examples (SEQ ID NOs: 28-31 and 53 to 59). Capital letters each refer to the commonly used abbreviation of each RNA.

### Example 14-2: Construction of mRNA-linker-peptide conjugate library

The following experiments were performed using mRNA-linker conjugates, in which linker 1 or linker 4 was hybridized separately to mRNA G containing a random region, as prepared in Example 3.

To the cell-free translation system constructed in Example 5, the following components were added: the mRNA-linker conjugate (final concentration: 1 µM), 7 aminoacyl-tRNAs (final concentration: 10 µM each; ClAc-F Ini-tRNA, MeF tRNA_{GAA}, MeG tRNA_{GAU}, MeNle tRNA_{GGU}, MeA tRNA_{GGC}, W tRNA_{GCA}, C tRNA_{CCA}), 10 amino acids (final concentration: 0.2 mM each; Leu, Val, Ser, Pro, Tyr, His, Asn, Asp, Arg, Gly), and 10 aminoacyl-tRNA synthetases (final concentrations: 0.04 µM LeuRS, 0.02 µM ValRS, 0.04 µM SerRS, 0.16 µM ProRS, 0.02 µM TyrRS, 0.02 µM HisRS, 0.38 µM AsnRS, 0.13 µM AspRS, 0.03 µM ArgRS, 0.09 µM GlyRS), as well as Mg(OAc)₂ (final concentration: 1 mM). A translation reaction was then carried out at 37°C for 30 minutes. After the reaction, EDTA was added to a final concentration of 12.5 mM, and the samples were allowed to stand on an ice bath for 10 minutes to modify the ribosomes.

Further, to the reaction solution after the first translation reaction described above, Mg(OAc)₂ (final concentration: 13.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT), aminoacyl-tRNA (final concentration: 10 µM ClAc-F Ini-tRNA, MeF tRNA_{GAA}, MeG tRNA_{GAU}, MeNle tRNA_{GGU}, MeA tRNA_{GGC}, W tRNA_{GCA}, C tRNA_{CCA}), and ribosomes (final concentration: 1.2 µM) were added, and a translation reaction was carried out at 37°C for 30 minutes. After the reaction, EDTA (final concentration: 16.7 mM) was added to stop the translation reaction.

Next, the reaction solution containing the translation product obtained above was admixed with a reverse transcription reaction solution (final concentration: 50 mM Tris-HCl, 75 mM KCl, 18.4 mM MgCl₂, 1 mM DTT, 0.30 mM dNTPs, 5 units/µL MLV reverse transcriptase, 3 µM TGG-ssG4S2.R23RT (SEQ ID NO: 9)), and a reverse transcription reaction was performed at 42°C for 30 minutes.

After the reverse transcription reaction, EDTA (final concentration: 16.7 mM) was added, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

### Example 14-3: Selection of target-binding peptides by using a library of mRNA-linker-peptide conjugates

The desalted sample obtained in the above section 2 was mixed with recombinant human IgG1 Fc protein (final concentration: 250 nM, 110-HG, R&D Systems) and magnetic beads (final concentration: 3 mg/mL, Dynabeads Protein G, Thermo Fisher Scientific Inc.), and a binding reaction was then performed while mixing at 4°C for 60 minutes. After the binding reaction, magnetic separation was conducted to remove the supernatant. The magnetic beads were then resuspended (1.5 mg/mL) using HBS-T, transferred to a new sample tube, and mixed at 4°C for 1 minute. Furthermore, the sequence of magnetic separation, removal of the supernatant, resuspension in HBS-T, and mixing at 4°C for 1 minute was repeated twice in total. Finally, after magnetic separation and removal of the supernatant, a PCR mix solution (10 mM Tris-HCl (pH 8.5), 50 mM KCl, 0.1% Triton X-100, dNTPs (final concentration: 0.25 mM), MgCl₂ (final concentration: 2 mM), T7g10 M.F52 (0.25 µM), TGG-ssG4S2.R44 (SEQ ID NO: 8) (0.25 µM)) was used, resuspended (3.0 mg/mL), and heated at 95°C for 5 minutes. After heating, magnetic separation was performed and the supernatant was collected. Hereafter, the above process is referred to as positive selection.

The amount of DNA in the solution before the addition of the target protein after desalting and the amount of DNA after recovery (positive selection) from the magnetic beads were quantified by real-time PCR. A LightCycler 96 (Roche Applied Science) was used as the real-time PCR machine, and the reaction solution prepared by adding Taq polymerase, SYBR Green I (100, 000-fold dilution, Invitrogen), and the measurement sample solution to the above PCR mix solution was subjected to measurement. The DNA recovered from the above magnetic beads was subjected to PCR (T100 Thermal Cycler, Bio-Rad; 94°C for 60 seconds, {94°C for 40 seconds, 61°C for 40 seconds, and 72°C for 40 seconds}) based on the threshold cycle (Cq value) obtained from the real-time PCR method, and the DNA amplification was thus performed. The reaction products were purified using AMPure XP (Beckman Coulter). mRNA was synthesized from the purified DNA by transcription reaction with T7RNA polymerase, and the obtained reaction product was purified using RNAClean XP (Beckman Coulter). After purification, the concentration of mRNA was determined from UV absorption at 260 nm and the mRNA was diluted to 20 µM.

The mRNA obtained above was used to construct a peptide library using the same procedure as in Example 13-2, and the desalted sample was mixed with magnetic beads (final concentration: 3 mg/mL, Dynabeads Protein G, Thermo Fisher Scientific Inc.), and a binding reaction was then performed while mixing at 4°C for 10 minutes. After the binding reaction, the magnetic beads were removed by magnetic separation. The above mixing with magnetic beads and removal of the magnetic beads by magnetic separation were performed twice in total. This process is referred to as negative selection. The magnetic beads after magnetic separation were resuspended in HBS-T (3.0 mg/mL) and heated at 95°C for 5 minutes. After heating, magnetic separation was performed, and the supernatant was collected and used as a sample for negative selection in the real-time PCR method. The supernatant after magnetic separation was further subjected to the positive selection described above, and the amount of DNA in the obtained sample was quantified by the real-time PCR method described above. Based on the threshold cycle (Cq value) obtained from the real-time PCR method, DNA was amplified by PCR and mRNA was prepared in the same way as above only for the sample after positive selection. In addition, the above negative selection, positive selection, real-time PCR, amplification of DNA by PCR, and mRNA preparation were performed three times in total.

Fig. 14 shows the results of the above selection process. Here, the recovery (%) is the ratio of the amount of DNA recovered from the magnetic beads to the amount of DNA in the solution before the addition of the target protein. In the selection of target-binding peptides by using Fc as a model target protein, a monovalent linker (linker 1) was used for selection. In this case, no significant difference was obtained between the recovery rate of DNA due to binding to the target protein (obtained in the positive selection) and the recovery rate of DNA due to nonspecific binding (obtained in the negative selection). By contrast, in the case of the selection using the divalent linker (linker 4), the recovery rate of DNA due to binding to the target protein was significantly higher than that of DNA due to nonspecific binding. In the case of the monovalent linker (linker 1), the binding between the target binding peptide and the Fc protein was not maintained during the washing operation in the selection process due to its weak binding ability. However, in the case of the divalent linker (linker 4), the ability to bind to the target protein is enhanced by the avidity effect due to the multiple peptides conjugated to the linker, and the binding between the target-binding peptide and the Fc protein is considered to be maintained during the washing operation.

### Example 14-4: Gene sequence analysis by next-generation sequencing (NGS)

For each round of DNA, desired sequences were added by PCR, followed by NGS analysis. Specific test procedures are described below.

For the post-final-round PCR samples, in order to add hybridization regions for the forward and reverse primers that initiate sequencing in NGS analysis, as well as an index region, a 50 µL reaction mixture was prepared containing the post-PCR sample (final concentration: 2% [v/v]) and the 1st PCR reaction solution (final concentrations: 1× Phusion HF buffer, 200 µM dNTPs, 250 nM forward primer [F70-ID12-07-4 (SEQ ID NO: 46) for selection samples using a monovalent linker, or F70-ID12-07-5 (SEQ ID NO: 47) for selection samples using a divalent linker], 250 nM R38-3UTR-SBS-R (SEQ ID NO: 48), and 20 U/mL Phusion DNA polymerase). A reaction was carried out using a thermal cycler (T100 Thermal Cycler, Bio-Rad) at 95°C for 120 seconds and 6 cycles of {95°C for 20 seconds, 61°C for 30 seconds, and 72°C for 30 seconds}. Next, for the post-PCR sample, the hybridization regions were added to the nucleic acid sequence immobilized on the flow cell for the NGS analysis. For this purpose, a 50 µL reaction mixture was prepared containing the post-PCR sample (final concentration: 2% [v/v]) and the 2nd PCR reaction solution (final concentrations: 1× Phusion HF buffer, 200 µM dNTPs, 250 nM F63-P5-SBS-F (SEQ ID NO: 49), 250 nM R58-SBSR-P7 (SEQ ID NO: 50), and 20 U/mL Phusion DNA polymerase). A reaction was carried out using a thermal cycler (T100 Thermal Cycler, Bio-Rad) at 95°C for 120 seconds and 6 cycles of {95°C for 20 seconds, 61°C for 30 seconds, and 72°C for 30 seconds}. The reaction products were purified using AMPure XP (Beckman Coulter). The purified sample was subjected to gene sequence analysis using Miseq (registered trademark) System and Miseq Regent Micro Kit v2.

### Example 14-5: Evaluation of binding ability by display assay

In the NGS analysis of each of the selections using the above monovalent linker or the divalent linker, the peptide sequences translated from the read gene information were analyzed and only those sequences that retained the fixed sequence of the peptide designed using the gene information and the number of randomized amino acids of the peptide were extracted. Further, from the extracted peptide sequences, the top 15 sequences in terms of frequency of occurrence were selected, and mRNA was prepared according to Example 2. After preparation, mRNA-linker conjugates were prepared by hybridizing linker 1 or linker 4 separately to the top 15 most frequently occurring mRNA sequences selected from the selection using the monovalent linker or divalent linker, respectively.

To the cell-free translation system constructed in Example 5, the following components were added: the mRNA-linker conjugate (final concentration: 1 µM), 7 aminoacyl-tRNAs (final concentration: 10 µM each; ClAc-F Ini-tRNA, MeF tRNA_{GAA}, MeG tRNA_{GAU}, MeNle tRNA_{GGU}, MeA tRNA_{GGC}, W tRNA_{GCA}, C tRNA_{CCA}), 10 amino acids (final concentration: 0.2 mM each; Leu, Val, Ser, Pro, Tyr, His, Asn, Asp, Arg, Gly), and 10 aminoacyl-tRNA synthetases (final concentrations: 0.04 µM LeuRS, 0.02 µM ValRS, 0.04 µM SerRS, 0.16 µM ProRS, 0.02 µM TyrRS, 0.02 µM HisRS, 0.38 µM AsnRS, 0.13 µM AspRS, 0.03 µM ArgRS, 0.09 µM GlyRS), as well as Mg(OAc)₂ (final concentration: 1 mM). A translation reaction was then carried out at 37°C for 30 minutes. After the reaction, EDTA was added to a final concentration of 12.5 mM, and the samples were allowed to stand on an ice bath for 10 minutes to modify the ribosomes.

Further, to the reaction solution after the first translation reaction described above, Mg(OAc)₂ (final concentration: 13.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT), aminoacyl-tRNA (final concentration: 10 µM ClAc-F Ini-tRNA, MeF tRNA_{GAA}, MeG tRNA_{GAU}, MeNle tRNA_{GGU}, MeA tRNA_{GGC}, W tRNA_{GCA}, C tRNA_{CCA}), and ribosomes (final concentration: 1.2 µM) were added, and a translation reaction was carried out at 37°C for 30 minutes. After the reaction, EDTA (final concentration: 16.7 mM) was added to stop the translation reaction.

Next, the reaction solution containing the translation product obtained above was admixed with a reverse transcription reaction solution (final concentration: 50 mM Tris-HCl, 75 mM KCl, 18.4 mM MgCl₂, 1 mM DTT, 0.30 mM dNTPs, 5 units/µL MLV reverse transcriptase, 3 µM TGG-ssG4S2.R23RT (SEQ ID NO: 9)), and a reverse transcription reaction was performed at 42°C for 30 minutes.

After the reverse transcription reaction, EDTA (final concentration: 16.7 mM) was added, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

The desalted sample was mixed with recombinant human IgG1 Fc protein (final concentration: 250 nM, 110-HG, R&D Systems) and magnetic beads (final concentration: 3 mg/mL, Dynabeads Protein G, Thermo Fisher Scientific Inc.) or magnetic beads alone (final concentration: 3 mg/mL, Dynabeads Protein G, Thermo Fisher Scientific Inc.), and a binding reaction was then performed while mixing at 4°C for 30 minutes. After the binding reaction, magnetic separation was conducted to remove the supernatant. The magnetic beads were then resuspended (1.5 mg/mL) using HBS-T, and transferred to a new sample tube. Further, the sequence of magnetic separation, removal of the supernatant, and resuspension in HBS-T was repeated three times in total. Finally, after magnetic separation and removal of the supernatant, the sample was resuspended (3.0 mg/mL) in HBS-T and heated at 95°C for 5 minutes. After heating, magnetic separation was performed and the supernatant was collected.

The amount of DNA in the solution before the addition of the target protein after desalting and the amount of DNA after recovery from the magnetic beads were quantified by real-time PCR. A LightCycler 96 (Roche Applied Science) was used as the real-time PCR machine. The reaction solution prepared by adding Taq polymerase, SYBR Green I (100, 000-fold dilution, Invitrogen), and the measurement sample solution to the above PCR mix solution was subjected to measurement.

Fig. 15 shows the results of the above display assay. Here, the recovery (%) is the ratio of the amount of DNA recovered from the magnetic beads to the amount of DNA in the solution before the addition of the target protein. In the selection of target-binding peptides by using Fc as a model target protein, for the top 15 most frequently occurring sequences selected from the selection using the monovalent linker (linker 1), no significant difference was found between the recovery rate of DNA due to binding to the target protein and the recovery rate of DNA due to nonspecific binding, regardless of the use of the monovalent or divalent linker in the display assay. By contrast, for the top 15 frequently occurring sequences selected from the selection using the divalent linker (linker 4), the recovery rate of DNA due to binding to the target protein was significantly higher than the recovery rate of DNA due to nonspecific binding in the display assay using the divalent linker. This may be due to the fact that in the selection using the randomized peptide library, when the divalent linker (linker 4) was used, binding to Fc was maintained by the avidity effect due to multiple peptides conjugated to the linker, and the sequences with target-binding ability were selected as the top frequently occurring sequences.

### [Example 15: Display assay with mRNA-linker conjugate (covalent conjugate)]

### Example 15-1: Preparation of mRNA-linker conjugate (covalent conjugate) by ligation

Each mRNA (final concentration: 1.0 µM) prepared from the template DNA of mRNA display format Strep-tag II as prepared in Example 2 and linker 7 or linker 8 (final concentration: 1.5 µM) prepared in Example 1 were reacted in a ligation solution (10% DMSO [v/v], 1× ligation buffer [TAKARA], 5 U/µL T4 RNA ligase) at 37°C for 1 hour. After the reaction, NaCl (final concentration: 0.3 M) and EDTA (5 mM) were added to stop the reaction, followed by phenol/chloroform extraction, ethanol precipitation, drying, and dissolution in ultrapure water. In this way, 5 µM mRNA-linker conjugate (covalent conjugate) was prepared.

### Example 15-2: Display assay with mRNA-linker-peptide conjugate using Strep-tagII as model peptide

The below-described display assay was performed using an mRNA-linker-peptide conjugate (covalently bonded conjugate) obtained by the ligation reaction in above section 1 to evaluate the recovery rate upon binding to streptavidin.

To the cell-free translation system constructed in Example 5, the following components were added: the mRNA-linker conjugate (final concentration: 1 µM), one type of aminoacyl-tRNA (final concentration: 10 µM W Ini-tRNA), 9 amino acids (final concentration: 0.2 mM each; Gly, His, Phe, Pro, Ser, Gln, Glu, Lys, Trp), and 9 aminoacyl-tRNA synthetases (final concentrations: 0.09 µM GlyRS, 0.02 µM HisRS, 0.68 µM PheRS, 0.16 µM ProRS, 0.04 µM SerRS, 0.06 µM GlnRS, 0.23 µM GluRS, 0.11 µM LysRS, 0.03 µM TrpRS). A translation reaction (first translation reaction) was then carried out at 37°C for 30 minutes. After the reaction, EDTA was added to the samples with artificial recycling translation to a final concentration of 12.5 mM. The mixture was then left to stand on ice for 10 minutes to modify the ribosomes.

To the reaction solution after the first translation reaction described above, Mg(OAc)₂ (final concentration: 12.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT, aminoacyl-tRNA [final concentration: 10 µM W Ini-tRNA]), and ribosomes (final concentration: 1.2 µM) were added, and a translation reaction (second translation reaction) was carried out at 37°C for 30 minutes. After the translation reaction, EDTA (final concentration: 16.7 mM) was added to stop the translation reaction.

Next, the reaction solution containing the translation product obtained above was admixed with a reverse transcription reaction solution (final concentration: 50 mM Tris-HCl, 75 mM KCl, 18.4 mM MgCl₂, 1 mM DTT, 0.30 mM dNTPs, 5 units/µL MLV reverse transcriptase, 3 µM RT_Strep-tag II (SEQ ID NO: 14)), and a reverse transcription reaction was performed at 42°C for 30 minutes.

After the reverse transcription reaction, EDTA (final concentration: 16.7 mM) was added, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

After desalting, magnetic beads (final concentration: 1 mg/mL, Dynabeads^{™} M-280 Streptavidin, Thermo Fisher Scientific Inc.) with immobilized streptavidin as a target protein were added, and a binding reaction was performed at 4°C for 1 hour. At this time, as a negative control, the desalted sample was added to magnetic beads with immobilized Protein G (final concentration: 1 mg/mL, Dynabead^{™} Protein G for Immunoprecipitation, Thermo Fisher Scientific Inc.), and a binding reaction was performed while mixing at 4°C for 1 hour. After the binding reaction, the magnetic beads were magnetically separated and the supernatant was removed. The magnetic beads were then resuspended (0.5 mg/mL) using HBS-T, transferred to a new sample tube, and mixed at 4°C for 5 minutes. Furthermore, the sequence of magnetic separation, removal of the supernatant, resuspension in HBS-T, and mixing at 4°C for 5 minutes was repeated twice in total. Finally, after magnetic separation and removal of the supernatant, the samples were resuspended (2 mg/mL) in PCR solution (10 mM Tris-HCl (pH 8.5), 50 mM KCl, 0.1% Triton X-100) and heated at 95°C for 5 minutes. After heating, magnetic separation was performed and the supernatant was collected.

The amount of DNA in the solution before the addition of the target protein after desalting and the amount of DNA after recovery from the magnetic beads were quantified by real-time PCR. A LightCycler 96 (Roche Applied Science) was used as the real-time PCR machine. The reaction solution was prepared by adding Taq polymerase, SYBR Green I (100,000-fold dilution, Invitrogen), dNTPs (final concentration: 0.25 mM), MgCl₂ (final concentration: 2 mM), T7g10M.F52 (0.25 µM), RV_mRNA display_Strep-tag II (SEQ ID NO: 45) (0.25 µM), and the measurement sample solution to the above-described PCR solution, and was used for measurement.

Fig. 16 shows the results of three trials of the above display assay. Here, the recovery rate (%) is the ratio of the amount of DNA recovered from the magnetic beads to the amount of DNA in the solution before the addition of the target protein. In the evaluation of the ability of Strep-tag II as a model peptide to bind to streptavidin as a model target protein, respectively, the use of a divalent linker (linker 8) significantly improved the recovery rate of DNA due to binding to the target protein when compared to the case of using a monovalent linker (linker 7). Further, the above-mentioned enhanced recovery rate was also observed in artificial recycling translation. These results suggest that like Example 10, the ability to bind to the target protein was enhanced by the avidity effect due to the multiple peptides conjugated to the above linker.

The results of Examples 10 and 15-2 above indicate that the use of a divalent linker enables multiple peptides to be displayed and improves the ability to bind to the target protein by the avidity effect, regardless of the method of attaching mRNA to the linker.

### [Example 16: Construction of linker in which amino acid is covalently bonded to 3'-end ribose via ester bond (RAPID linker), and display assay using this linker]

### Example 16-1 Acylation of the 3' end of linker 9 or 10 by ARS ribozyme

As an amino acid-activating ester used for aminoacylation of the 3' end of linker 9 or 10 by ARS ribozyme, 2,2,2-trifluoroethyl methyl-L phenylalaninate hydrochloride (NMe-Phe-TEE) was prepared (prepared by the method disclosed in WO2023/234425 (PTL 11)). To link the 3' end of linker 9 or 10 to each amino acid-activating ester separately, eFx was used as the ARS ribozyme. For NMe-Phe-TEE (final concentration: 5 mM), eFx (final concentration: 25 µM), linker 9 or linker 10 (final concentration: 25 µM), HEPES-KOH (final concentration: 50 mM, pH 7.5), MgCl₂ (final concentration: 50 mM), and DMSO (final concentration: 20%) were added, and the aminoacylation reaction was carried out at 0°C overnight. An equal volume of 3M NaOAc (pH 5.2) was added to the above aminoacylated samples, followed by ethanol precipitation.

The pellets were then each redissolved using 0.3 M NaOAc (pH 5.2), and ethanol precipitation was performed again. Finally, the pellets were washed once each with 70% ethanol containing 0.1 M NaOAc (pH 5.2) and 70% ethanol. The resulting pellet of aminoacylated sample was dissolved in 0.2% acetic acid. After the aminoacylated sample was dissolved in 2.64× loading buffer (final concentrations: 41 mM sodium acetate, 23% formamide, 2.7 mM EDTA), it was separated under acidic conditions by electrophoresis on a 20% denaturing polyacrylamide gel (50 mM sodium acetate [pH 5.2], 6 M urea) (using the running buffer: 50 mM sodium acetate [pH 5.2]). After electrophoresis, the gel was analyzed by fluorescent staining using SYBR Green II (Invitrogen, SYBRha Molecular Probes Inc.).

Fig. 17a shows the results obtained. Fig. 17a is images obtained when fluorescence derived from SYBR Green I (Invitrogen, SYBRha Molecular Probes Inc.) was detected. In the case of acylation using the monovalent linker (linker 9), one band shifted to the high-molecular-weight side when compared to the band position before acylation, suggesting that acylation of one amino acid to the linker increased the molecular weight, resulting in such a band shift. In the case of the divalent linker (linker 10), two bands shifted to the high-molecular-weight side when compared to the band position before acylation. This may be because the molecular weight is increased by acylation of one or two amino acids to the divalent linker (linker 10), which has two CCAs at the ends, namely the nucleic acid recognition sites of ARS ribozyme, resulting in such a band shift, respectively.

### Example 16-2: Display assay with mRNA-linker-peptide conjugate using Strep-tagII as model peptide

A display assay was performed using an mRNA-linker-peptide conjugate with Strep-tagII as a model peptide to evaluate the recovery rate upon binding to streptavidin.

For the mRNA encoding Strep-tag II as prepared in Example 3, linker 9 or linker 10 acylated with MePhe in Example 16-1 was hybridized in 1 mM sodium acetate solution, and the following experiments were performed.

To the cell-free translation system constructed in Example 5, the following components were added: the mRNA-linker conjugate (final concentration: 1 µM), one type of aminoacyl-tRNA (final concentration: 10 µM W Ini-tRNA), 9 amino acids (final concentration: 0.2 mM each; Gly, His, Phe, Pro, Ser, Gln, Glu, Lys, Trp), and 9 aminoacyl-tRNA synthetases (final concentrations: 0.09 µM GlyRS, 0.02 µM HisRS, 0.68 µM PheRS, 0.16 µM ProRS, 0.04 µM SerRS, 0.06 µM GlnRS, 0.23 µM GluRS, 0.11 µM LysRS, 0.03 µM TrpRS). A translation reaction (first translation reaction) was then carried out at 37°C for 30 minutes. After the reaction, EDTA was added to the samples with artificial recycling translation to a final concentration of 12.5 mM. The mixture was then left to stand on ice for 10 minutes to modify the ribosomes.

To the reaction solution after the first translation reaction described above, Mg(OAc)₂ (final concentration: 12.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT, aminoacyl-tRNA [final concentration: 10 µM W Ini-tRNA]), and ribosomes (final concentration: 1.2 µM) were added, and a translation reaction (second translation reaction) was carried out at 37°C for 30 minutes. After the translation reaction, EDTA (final concentration: 16.7 mM) was added to stop the translation reaction.

Next, the reaction solution containing the translation product obtained above was admixed with a reverse transcription reaction solution (final concentration: 50 mM Tris-HCl, 75 mM KCl, 18.4 mM MgCl₂, 1 mM DTT, 0.30 mM dNTPs, 5 units/µL MLV reverse transcriptase, 3 µM RT_Strep-tag II (SEQ ID NO: 14)), and a reverse transcription reaction was performed at 42°C for 15 minutes.

After the reverse transcription reaction, EDTA (final concentration: 16.7 mM) was added, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

After desalting, magnetic beads (final concentration: 1 mg/mL, Dynabeads^{™} M-280 Streptavidin, Thermo Fisher Scientific Inc.) with immobilized streptavidin as a target protein were added, and a binding reaction was performed at 4°C for 1 hour. At this time, as a negative control, the desalted sample was added to magnetic beads with immobilized Protein G (final concentration: 1 mg/mL, Dynabead^{™} Protein G for Immunoprecipitation, Thermo Fisher Scientific Inc.), and a binding reaction was performed while mixing at 4°C for 1 hour. After the binding reaction, the magnetic beads were magnetically separated and the supernatant was removed. The magnetic beads were then resuspended (0.5 mg/mL) using HBS-T, transferred to a new sample tube, and mixed at 4°C for 5 minutes. Furthermore, the sequence of magnetic separation, removal of the supernatant, resuspension in HBS-T, and mixing at 4°C for 5 minutes was repeated twice in total. Finally, after magnetic separation and removal of the supernatant, the samples were resuspended (2 mg/mL) in PCR solution (10 mM Tris-HCl (pH 8.5), 50 mM KCl, 0.1% Triton X-100) and heated at 95°C for 5 minutes. After heating, magnetic separation was performed and the supernatant was collected.

The amount of DNA in the solution before the addition of the target protein after desalting and the amount of DNA after recovery from the magnetic beads were quantified by real-time PCR. A LightCycler 96 (Roche Applied Science) was used as the real-time PCR machine. The reaction solution was prepared by adding Taq polymerase, SYBR Green I (100,000-fold dilution, Invitrogen), dNTPs (final concentration: 0.25 mM), MgCl₂ (final concentration: 2 mM), T7g10M.F52 (0.25 µM), RV_Strep-tag II (SEQ ID NO: 10) (0.25 µM), and the measurement sample solution to the above-described PCR solution, and was used for measurement.

Fig. 18 shows the results of three trials of the above display assay. Here, the recovery rate (%) is the ratio of the amount of DNA recovered from the magnetic beads to the amount of DNA in the solution before the addition of the target protein. In the evaluation of the ability of Strep-tag II as a model peptide to bind to streptavidin as a model target protein, respectively, the use of a divalent linker (linker 10) significantly improved the recovery rate of DNA due to binding to the target protein when compared to the case of using a monovalent linker (linker 9). Further, the above-mentioned enhanced recovery rate was also observed in artificial recycling translation. These results suggest that like Example 10, the ability to bind to the target protein was enhanced by the avidity effect due to the multiple peptides conjugated to the above linker.

From the results of Examples 10 and 16-1 and 16-2, the amino acid was covalently bonded via an ester bond to the ribose at the 3' end of the linker, thereby conferring the characteristics of a puromycin-like substance; in this case, the use of a divalent linker still allows for the display of multiple peptides, which is considered to enhance the ability to bind to the target protein through the avidity effect.

### [Example 17: Display assay with trivalent linker]

### Example 17-1: Display assay 1 with mRNA-linker-peptide conjugate using Strep-tagII as model peptide

A display assay was performed using an mRNA-linker-peptide conjugate with Strep-tagII as a model peptide to evaluate the recovery rate upon binding to streptavidin.

The following experiments were performed using mRNA-linker conjugates, in which the mRNA encoding Strep-tagII was hybridized with linker 1 or linker 11, as prepared in Example 3.

To the cell-free translation system constructed in Example 5, the following components were added: the mRNA-linker conjugate (final concentration: 1 µM), one type of aminoacyl-tRNA (final concentration: 10 µM W Ini-tRNA), 9 amino acids (final concentration: 0.2 mM each; Gly, His, Phe, Pro, Ser, Gln, Glu, Lys, Trp), and 9 aminoacyl-tRNA synthetases (final concentrations: 0.09 µM GlyRS, 0.02 µM HisRS, 0.68 µM PheRS, 0.16 µM ProRS, 0.04 µM SerRS, 0.06 µM GlnRS, 0.23 µM GluRS, 0.11 µM LysRS, 0.03 µM TrpRS). A translation reaction (first translation reaction) was then carried out at 37°C for 30 minutes. After the reaction, EDTA was added to the samples without artificial recycling translation to a final concentration of 16.7 mM, and to the samples with the second translation by artificial recycling translation to a final concentration of 12.5 mM. The mixtures were then left to stand on ice for 10 minutes to modify the ribosomes.

Further, a second translation reaction was carried out for the samples with artificial recycling translation.

To the reaction solution after the first translation reaction described above, Mg(OAc)₂ (final concentration: 12.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT, aminoacyl-tRNA [final concentration: 10 µM W Ini-tRNA]), and ribosomes (final concentration: 1.2 µM) were added, and a translation reaction (second translation reaction) was carried out at 37°C for 30 minutes. After the reaction, EDTA was added to the samples without the third translation by artificial recycling translation to a final concentration of 16.7 mM, and to the samples with the third translation by artificial recycling translation to a final concentration of 12.5 mM. The mixtures were then left to stand on ice for 10 minutes to modify the ribosomes.

Further, a third translation reaction was carried out for the samples with artificial recycling translation.

To the reaction solution after the second translation reaction described above, Mg(OAc)₂ (final concentration: 12.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT, aminoacyl-tRNA [final concentration: 10 µM W Ini-tRNA]), and ribosomes (final concentration: 1.2 µM) were added, and a translation reaction (third translation reaction) was carried out at 37°C for 30 minutes. After the translation reaction, EDTA (final concentration: 16.7 mM) was added to stop the translation reaction.

Next, the reaction solution containing the translation product obtained above was admixed with a reverse transcription reaction solution (final concentration: 50 mM Tris-HCl, 75 mM KCl, 18.4 mM MgCl₂, 1 mM DTT, 0.30 mM dNTPs, 5 units/µL MLV reverse transcriptase, 3 µM RT_Strep-tag II (SEQ ID NO: 14)), and a reverse transcription reaction was performed at 42°C for 30 minutes.

After the reverse transcription reaction, EDTA (final concentration: 16.7 mM) was added, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

After desalting, magnetic beads (final concentration: 1 mg/mL, Dynabeads^{™} M-280 Streptavidin, Thermo Fisher Scientific Inc.) with immobilized streptavidin as a target protein were added, and a binding reaction was performed at 4°C for 1 hour. At this time, as a negative control, the desalted sample was added to magnetic beads with immobilized Protein G (final concentration: 1 mg/mL, Dynabead^{™} Protein G for Immunoprecipitation, Thermo Fisher Scientific Inc.), and a binding reaction was performed while mixing at 4°C for 1 hour. After the binding reaction, the magnetic beads were magnetically separated and the supernatant was removed. The magnetic beads were then resuspended (0.5 mg/mL) using HBS-T, transferred to a new sample tube, and mixed at 4°C for 30 minutes. Furthermore, the sequence of magnetic separation, removal of the supernatant, resuspension in HBS-T, and mixing at 4°C for 30 minutes was repeated twice in total. Finally, after magnetic separation and removal of the supernatant, the samples were resuspended (2 mg/mL) in PCR solution (10 mM Tris-HCl (pH 8.5), 50 mM KCl, 0.1% Triton X-100) and heated at 95°C for 5 minutes. After heating, magnetic separation was performed and the supernatant was collected.

The amount of DNA in the solution before the addition of the target protein after desalting and the amount of DNA after recovery from the magnetic beads were quantified by real-time PCR. A LightCycler 96 (Roche Applied Science) was used as the real-time PCR machine. The reaction solution was prepared by adding Taq polymerase, SYBR Green I (100,000-fold dilution, Invitrogen), dNTPs (final concentration: 0.25 mM), MgCl₂ (final concentration: 2 mM), T7g10M.F52 (0.25 µM), RV_Strep-tag II (SEQ ID NO: 10) (0.25 µM), and the measurement sample solution to the above-described PCR solution, and was used for measurement.

Fig. 19 shows the results of three trials of the above display assay. Here, the recovery rate (%) is the ratio of the amount of DNA recovered from the magnetic beads to the amount of DNA in the solution before the addition of the target protein. In the evaluation of the ability of Strep-tag II as a model peptide to bind to streptavidin as a model target protein, respectively, the use of a trivalent linker (linker 11) significantly improved, depending on the number of times for artificial recycling translation, the recovery rate of DNA due to binding to the target protein when compared to the case of using a monovalent linker (linker 1). This suggests that the ability to bind to the target protein was enhanced by the avidity effect due to the multiple peptides conjugated to the above linker.

### Example 17-2: Display assay 2 with mRNA-linker-peptide conjugate using Strep-tagII as model peptide

A display assay was performed using an mRNA-linker-peptide conjugate with Strep-tagII as a model peptide to evaluate the recovery rate upon binding to streptavidin.

The following experiments were performed using mRNA-linker conjugates, in which the mRNA encoding Strep-tagII was hybridized with linker 1 or linker 15, as prepared in Example 3.

To the cell-free translation system constructed in Example 5, the following components were added: the mRNA-linker conjugate (final concentration: 1 µM), one type of aminoacyl-tRNA (final concentration: 10 µM W Ini-tRNA), 9 amino acids (final concentration: 0.2 mM each; Gly, His, Phe, Pro, Ser, Gln, Glu, Lys, Trp), and 9 aminoacyl-tRNA synthetases (final concentrations: 0.09 µM GlyRS, 0.02 µM HisRS, 0.68 µM PheRS, 0.16 µM ProRS, 0.04 µM SerRS, 0.06 µM GlnRS, 0.23 µM GluRS, 0.11 µM LysRS, 0.03 µM TrpRS). A translation reaction (first translation reaction) was then carried out at 37°C for 30 minutes. After the reaction, EDTA was added to the samples with artificial recycling translation to a final concentration of 12.5 mM. The mixture was then left to stand on ice for 10 minutes to modify the ribosomes.

To the reaction solution after the first translation reaction described above, Mg(OAc)₂ (final concentration: 12.5 mM), a translation-related solution (final concentrations: 23.5 mM HEPES-KOH [pH 7.6], 0.94 mM ATP, 0.94 mM GTP, 0.47 mM CTP, 0.47 mM UTP, 9.4 mM creatine phosphate, 47.0 mM potassium acetate, 0.94 mM spermidine, 0.7 mg/mL *E. coli* total tRNA [Roche], 0.47 mM DTT, aminoacyl-tRNA [final concentration: 10 µM W Ini-tRNA]), and ribosomes (final concentration: 1.2 µM) were added, and a translation reaction (second translation reaction) was carried out at 37°C for 30 minutes. After the translation reaction, EDTA (final concentration: 16.7 mM) was added to stop the translation reaction.

Next, the reaction solution containing the translation product obtained above was admixed with a reverse transcription reaction solution (final concentration: 50 mM Tris-HCl, 75 mM KCl, 18.4 mM MgCl₂, 1 mM DTT, 0.30 mM dNTPs, 5 units/µL MLV reverse transcriptase, 3 µM RT_Strep-tag II (SEQ ID NO: 14)), and a reverse transcription reaction was performed at 42°C for 30 minutes.

After the reverse transcription reaction, EDTA (final concentration: 16.7 mM) was added, followed by desalting using a Bio-Gel P30 Gel (Bio-Rad) desalting column equilibrated with HBS-T (0.05% [v/v] Tween 20, 25 mM HEPES-NaOH [pH 7.4], 150 mM NaCl).

After desalting, magnetic beads (final concentration: 1 mg/mL, Dynabeads^{™} M-280 Streptavidin, Thermo Fisher Scientific Inc.) with immobilized streptavidin as a target protein were added, and a binding reaction was performed at 4°C for 1 hour. At this time, as a negative control, the desalted sample was added to magnetic beads with immobilized Protein G (final concentration: 1 mg/mL, Dynabead^{™} Protein G for Immunoprecipitation, Thermo Fisher Scientific Inc.), and a binding reaction was performed while mixing at 4°C for 1 hour. After the binding reaction, the magnetic beads were magnetically separated and the supernatant was removed. The magnetic beads were then resuspended (0.5 mg/mL) using HBS-T, transferred to a new sample tube, and mixed at 4°C for 30 minutes. Furthermore, the sequence of magnetic separation, removal of the supernatant, resuspension in HBS-T, and mixing at 4°C for 30 minutes was repeated twice in total. Finally, after magnetic separation and removal of the supernatant, the samples were resuspended (2 mg/mL) in PCR solution (10 mM Tris-HCl (pH 8.5), 50 mM KCl, 0.1% Triton X-100) and heated at 95°C for 5 minutes. After heating, magnetic separation was performed and the supernatant was collected.

The amount of DNA in the solution before the addition of the target protein after desalting and the amount of DNA after recovery from the magnetic beads were quantified by real-time PCR. A LightCycler 96 (Roche Applied Science) was used as the real-time PCR machine. The reaction solution was prepared by adding Taq polymerase, SYBR Green I (100,000-fold dilution, Invitrogen), dNTPs (final concentration: 0.25 mM), MgCl₂ (final concentration: 2 mM), T7g10M.F52 (0.25 µM), RV_Strep-tag II (SEQ ID NO: 10) (0.25 µM), and the measurement sample solution to the above-described PCR solution, and was used for measurement.

Fig. 20 shows the results of two trials of the above display assay. Here, the recovery rate (%) is the ratio of the amount of DNA recovered from the magnetic beads to the amount of DNA in the solution before the addition of the target protein. In the evaluation of the ability of Strep-tag II as a model peptide to bind to streptavidin as a model target protein, respectively, the use of a trivalent linker (linker 15) significantly improved the recovery rate of DNA due to binding to the target protein when compared to the case of using a monovalent linker (linker 1). This suggests that the ability to bind to the target protein was enhanced by the avidity effect due to the multiple peptides conjugated to the above linker.

### INDUSTRIAL APPLICABILITY

In the present invention, the "conjugate in which multiple translation products are conjugated via puromycin-like substances to one genetic information material" can recognize the target protein through the multiple translation products. Therefore, for example, the use for displays (e.g., mRNA display) using a cell-free translation system makes it possible to significantly improve the recovery rate of peptide-nucleic acid conjugates that bind to the target protein, and to obtain peptide-binding substances that have a weaker ability to bind to the peptide. The provision of such a novel display should contribute, for example, to the development of superior peptide-based therapeutics.

## Claims

1. A linker comprising:
an attachment part having a structure that can be attached to a desired genetic information material; and
at least two or more puromycin-like substances, wherein
each of the puromycin-like substances can be covalently bonded to a C-terminus of a desired peptide.

2. The linker according to claim 1, wherein the attachment part having a structure that can be attached to a desired genetic information material comprises a nucleic acid that can be attached to the desired genetic information material.

3. The linker according to claim 1 or 2, for conjugating the genetic information material and a peptide encoded by the genetic information material.

4. The linker according to any one of claims 1 to 3, wherein the genetic information material is a nucleic acid.

5. The linker according to claims 1 to 3, wherein the puromycin-like substance is puromycin.

6. Use of a linker comprising:
an attachment part having a structure that can be attached to a desired genetic information material; and
at least two or more puromycin-like substances, wherein
each of the puromycin-like substances can be covalently bonded to a C-terminus of a desired peptide, wherein
the use is for conjugating the genetic information material and the peptide encoded by the genetic information material.

7. A genetic information material-linker conjugate comprising:
(a) the linker according to any one of claims 1 to 3; and
(b) the genetic information material attached to the attachment part of the linker (a).

8. A genetic information material-linker-peptide conjugate comprising:
(a) the linker according to any one of claims 1 to 3;
(b) the genetic information material attached to the attachment part of the linker (a); and
(c) a peptide encoded by the genetic information material and bonded to each of the at least two or more puromycin-like substances of the linker (a).

9. A method for producing a genetic information material-linker-peptide conjugate, comprising:
step (1) of subjecting the genetic information material-linker conjugate according to claim 7 to a cell-free translation system to translate the genetic information material, wherein each of the puromycin-like substances in the linker and a translated peptide are bonded to each other to produce the genetic information material-linker-peptide conjugate.

10. The production method according to claim 9, comprising, before step (1), step (0) of attaching the linker according to any one of claims 1 to 3 to the desired genetic information material to obtain the genetic information material-linker conjugate.

11. The production method according to claim 9 or 10, comprising step (2) of repeating step (1) two or more times.

12. A library comprising at least two of the genetic information material-linker-peptide conjugate according to claim 8.

13. A method of screening for a peptide capable of binding to a desired target substance, comprising the step of bringing a library containing at least two of the genetic information material-linker-peptide conjugate according to claim 8 into contact with the target substance.

14. A method of evaluating an ability of a peptide to bind to a desired target substance, comprising the step of bringing the genetic information material-linker-peptide conjugate according to claim 8 into contact with the target substance.

15. A method for producing a genetic information material-linker-peptide conjugate, comprising:
step (1-i) of subjecting a genetic information material-linker conjugate, in which a linker containing at least one puromycin-like substance and a desired genetic information material are attached to each other, to a cell-free translation system to translate the genetic information material, wherein the puromycin-like substance in the linker and a translated peptide are bonded to each other to produce the genetic information material-linker-peptide conjugate; and
step (2-i) of repeating step (1-i) two or more times.

16. A method for producing a genetic information material-linker-peptide conjugate, comprising:
step (1-ii) of subjecting a genetic information material-linker conjugate, in which a linker containing at least two or more puromycin-like substances and a desired genetic information material are attached to each other, to a cell-free translation system to translate the genetic information material, wherein each of the puromycin-like substances in the linker and a translated peptide are bonded to each other to produce the genetic information material-linker-peptide conjugate.

17. A method of displaying, from a desired genetic information material, two or more peptides encoded by the genetic information material, wherein the peptides are each conjugated to the genetic information material via a functional group that can be covalently bonded to a C-terminus of each of the peptides.

18. The method of displaying according to claim 17, comprising the step of subjecting the genetic information material-linker conjugate according to claim 7 to a cell-free translation system to translate the genetic information material into the peptides, wherein the puromycin-like substances and the translated peptides are bonded to each other.
